# EUROPEAN PATENT APPLICATION

(11) **EP 1 964 548 A1**
(43) Date of publication of application: **03.09.2008**
(21) Application number: 07103365.8
(22) Date of filing: 02.03.2007
(51) Int. Cl.: A61K 9/107, A61K 9/14, A61K 47/44, A61K 31/517, A61K 31/4184

(54) **Pharmaceutical compositions comprising a calcilytic agent**

(71) Applicant: Novartis AG, 4056 Basel (CH)
(72) Inventor: The designation of the inventor has not yet been filed
(74) Representative: Leon, Susanna Iris

(57) **Abstract**

Provided is a pharmaceutical composition comprising a calcilytic agent which, when administered orally to a subject induces a rapid and short-lasting absorption of the calcilytic agent and/or a rapid and short-lasting release of the parathyroid hormone.

## Description

The present invention relates to pharmaceutical compositions, in particular to compositions for oral administration of a calcilytic as an active agent.

Osteoporosis is characterized by low bone mass and micro-architectural deterioration of bone tissue that leads to fragility and increased risk of fractures. Current therapies for treatment of osteoporosis are based on the inhibition of bone resorption to prevent further bone loss. Approved therapies for treatment of osteoporosis targeting the osteoclast by decelerating bone loss are bisphosphonates (e.g. alendronate, risedronate, ibandronate), calcitonin, estrogen and selective estrogen receptor modulators. Calcium and vitamin D are baseline therapies. Because many osteoporosis patients have already lost a substantial amount of bone at the time of diagnosis, there is a need for developing agents that increase bone mass by stimulating new bone formation. Currently the only anabolic treatment for osteoporosis is teriparatide, the 1-34 fragment of parathyroid hormone (PTH), or parathyroid hormone itself. They cause significant increase in bone mass and reduce vertebral fracture risk substantially. These peptides must presently be administered by subcutaneous injection, which is inconvenient for patients.

Another way to stimulate the release of parathyroid hormone into the plasma is the mobilization of endogenous stores of the hormone. Parathyroid hormone is stored in relatively large amounts in parathyroid cells and its secretion is controlled by a calcium sensing receptor (PCaR) located on the cell surface. Antagonists or allosteric modulators of parathyroid calcium sensing receptors mimic a state of hypocalcemia and stimulate parathyroid hormone release. They are referred to as calcilytic agents. It has been found that a short period of elevation of parathyroid hormone is crucial for an effective therapeutic result, since constantly elevated plasma levels of parathyroid hormone increase not only bone formation but also resorption and result in a net loss of predominantly cortical bone.

Surprisingly the inventors have now found a pharmaceutical composition comprising a calcilytic agent which, when administered orally to a subject induces a rapid and short-lasting release of parathyroid hormone into the plasma. The rapid and short-lasting release may be followed by a rapid decrease of the parathyroid hormone to baseline levels.

In another aspect the present invention provides a pharmaceutical composition comprising a calcilytic agent which, when administered orally to a subject results in a rapid release and rapid gastro-intestinal absorption of the calcilytic agent with a subsequent, rapid and short-lasting increase of its plasma levels.

In a further aspect of the invention the parathyroid hormone and the calcilytic agent may show super-imposed plasma concentration profiles.

In another aspect the present invention provides a pharmaceutical composition comprising a calcilytic agent which, when administered orally to a subject results in a rapid and short-lasting gastro-intestinal absorption of the calcilytic agent and rapid release of the parathyroid hormone. The calcilytic agent may be rapidly absorbed gastro-intestinally, triggering the rapid and short-lasting release of parathyroid hormone into the plasma. In turn, this may be followed by a rapid decrease of the parathyroid hormone to baseline levels. After absorption the calcilytic agent may rapidly be eliminated.

As used herein "rapid and short-lasting release" means that the parathyroid hormone may be present in the plasma at pharmacodynamically relevant concentrations of five- to seven-fold of the baseline concentration for a maximum duration of 4 h, e.g. for 15 to 90 min, or for 30 to 60 minutes.

The rapid and short-lasting release may be characterized by the following pharmacokinetic parameters.

As used herein the term "Tstart" means the time after oral administration when the plasma concentration of the calcilytic agent is equal to or above 20 % of the maximum plasma concentration (Cmax, cf. below) or the parathyroid hormone is 100 % more than the baseline level.

As used herein the term "Tmax" means the time to peak plasma concentration of the active agent or the parathyroid hormone after oral administration.

As used herein the term maximum concentration or "Cmax" means the maximum concentration of the calcilytic agent or the parathyroid hormone in plasma after oral administration.

The pharmaceutical composition according to the present invention may induce a rapid and short-lasting release of the parathyroid hormone wherein the parathyroid hormone may show a release profile having a Tmax not later than 90 minutes after Tstart and a Cmax which is a five to seven fold increase compared to the baseline levels.

The pharmaceutical composition according to the present invention may induce a rapid and short-lasting absorption of the calcilytic agent wherein the calcilytic agent may show a release profile having a Tmax not later than 90 min after Tstart.

Advantageously the compositions of the present invention enable that a calcilytic agent, after oral administration, induces the release of endogenous parathyroid hormone. There is no need to administer exogenous parathyroid hormone via injection or any other mode, e.g. oral, nasal or inhalation.

According to the present invention the calcilytic agent may easily penetrate gastrointestinal epithelia, but may show limited solubility and slow dissolution rate. The term "limited solubility", as used herein, is understood to mean a solubility in water at 20 °C of less than 1 e.g. 0.05% weight/volume.

In a preferred aspect of the invention, the calcilytic agent is a compound of formula IV 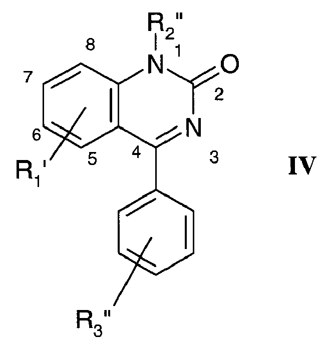 wherein R₁' represents 1 or 2 substituents independently selected from H, OH, halo, NO₂, optionally substituted (C1-C7 alkyl, C1-C7 alkoxy, C2-C7 alkenyl, C2-C7 alkenyloxy, C2-C7 alkynyl, C2-C7 alkynyloxy, C1-C7 alkanoyl or amino) wherein the optional substituents are 1 or 2 substituents independently selected from halo, C1-C7 alkyl, C2-C7 alkenyl, C2-C7 alkynyl, cycloalkyl, or cyano;
R₂" is optionally substituted aryl-methyl, the optional substituents being up to 5, usually 1, 2 or 3 substituents, independently selected from halo, nitro, cyano, amino, OH, SH, C1-C7 alkyl, C1-C7 alkoxy, C1-C7 thioalkoxy, C1-C7 alkoxycarbonyl, C1-C7 alkylsulphonyl, C1-C7 alkoxysulphonyl, C1-C7 alkylcarbonyloxy, trifluoromethyl, optionally halo-substituted aryl, optionally oxo-substitued pyrrolidinyl or -X-A-Z, wherein -X- is -CO-O-, -O-, -CH₂-O-, -CO-NR5-, -NR5-, -CH₂-NR5-, -CO-CH₂-, -S-, -SO-NR5-,-SO₂-NR5-, -NR5-CO- or -O-CO-, where R5 is H or optionally substituted (C1-C7 alkyl, C2-C7 alkenyl, C1-C7 alkoxy- C1-C7 alkyl, aryl C1-C7 alkyl or optionally mono-or di- C1-C7 alkylsubstituted amino C1-C7 alkyl),
-A- is C₁-C₁₀ alkyl, preferably C₃-C₈ alkyl optionally interrupted by 1, 2 or 3, of -O-, -S- or-NR5-, and
Z is H, halo, C1-C7 alkoxy, C1-C7 alkoxy- C1-C7 alkoxy, -NR5R5', -N⁺R5R5'R5", -COOH, imidazolyl, optionally R5 substituted -piperazinyl, -CH(COOH)₂, -SO₃⁻, -NR5-(CH₂)ₙ-CH₂-NR5R5', -NR5-(CH₂)ₙ-CH₂-OR5, morpholino or tetrahydropyranyl,
where R5, R5' and R5" are independently H or optionally substituted (C1-C7 alkyl, C1-C7 alkoxy- C1-C7 alkyl or aryl C1-C7 alkyl), or
R5, R5' or R5" may be linked together in an optionally substituted N-heterocyclic ring containing from 3 to 8 ring atoms one or more of which may comprise a further heteroatom selected from O, S or -NR5-, wherein R5 is as defined above;
R3" is C1-C7 alkyl;
or a pharmaceutically-acceptable ester, or acid addition salt thereof.

For example, the calcilytic maybe a compound selected from the following:
6-Amino-1-benzyl-4-(4-tert.butyl-phenyl)-1.H.-quinazolin-2-one
6-Propargylamino-1-benzyl-4-(4-isopropyl-phenyl)-1.H.-quinazolin-2-one
6-Allylamino-1-benzyl-4-(4-isopropyl-phenyl)-1.H.-quinazolin-2-one
1-Benzyl-4-(4-isopropyl-phenyl)-6-methoxy-1.H.-quinazolin-2-one
[1-Benzyl-4-(4-isopropyl-phenyl)-2-oxo-1,2-dihydro-quinazolin-6-yloxy]-acetonitrile
1-(3-Chloro-benzyl)-4-(4-isopropyl-phenyl)-6-methoxy-1.H.-quinazolin-2-one
1-(3-Chloro-benzyl)-4-(4-isopropyl-phenyl)-6-methoxy-1.H.-quinazolin-2-one
1-(3-Fluoro-benzyl)-4-(4-isopropyl-phenyl)-6-methoxy-1.H.-quinazolin-2-one
4-(4-Isopropyl-phenyl)-6-methoxy-1-naphthalen-2-ylmethyl-1.H.-quinazolin-2-one
4-[4-(4-Isopropyl-phenyl)-6-methoxy-2-oxo-2.H.-quinazolin-1-ylmethyl]-benzonitrile
4-[4-(4-Isopropyl-phenyl)-6-methoxy-2-oxo-2.H.-quinazolin-1-ylmethyl]-3-methoxy-benzoic acid methyl ester
3-[4-(4-Isopropyl-phenyl)-6-methoxy-2-oxo-2.H.-quinazolin-1-ylmethyl]-benzoic acid methyl ester
4-(4-Isopropyl-phenyl)-6-methoxy-1-(3-nitro-benzyl)-1.H.-quinazolin-2-one
3-[4-(4-isopropyl-phenyl)-6-methoxy-2-oxo-2.H.-quinazolin-1-ylmethyl]-benzoic acid
3-[4-(4-isopropyl-phenyl)-6-methoxy-2-oxo-2.H.-quinazolin-1-ylmethyl]-benzamide
3-[4-(4-Isopropyl-phenyl)-6-methoxy-2-oxo-2.H.-quinazolin-1-ylmethyl]-N,N-dimethylbenzamide
3-[4-(4-Isopropyl-phenyl)-6-methoxy-2-oxo-2.H.-quinazolin-1-ylmethyl]-benzoic acid 2-dimethylamino-ethyl ester
3-[4-(4-Isopropyl-phenyl)-6-methoxy-2-oxo-2.H.-quinazolin-1-ylmethyl]-N-methyl-benzamide
3-[4-(4-Isopropyl-phenyl)-6-methoxy-2-oxo-2.H.-quinazolin-1-ylmethyl]-benzoic acid isopropyl ester
3-[4-(4-Isopropyl-phenyl)-6-methoxy-2-oxo-2.H.-quinazolin-1-ylmethyl]-benzoic acid 2-(2-dimethylamino-ethoxy)-ethyl ester
3-[4-(4-Isopropyl-phenyl)-2-oxo-6-prop-2-ynyloxy-2H-quinazolin-1-ylmethyl]-benzoic acid 2-(2-dimethylamino-ethoxy)-ethyl ester (trifluoroacetic acid salt)
3-[4-(4-Isopropyl-phenyl)-6-methoxy-2-oxo-2.H.-quinazolin-1-ylmethyl]-benzoic acid 4-dimethylamino-butyl ester
3-[4-(4-Isopropyl-phenyl)-6-methoxy-2-oxo-2.H.-quinazolin-1-ylmethyl]-benzoic acid 3-dimethylamino-propyl ester
3-[4-(4-Isopropyl-phenyl)-6-methoxy-2-oxo-2.H.-quinazolin-1-ylmethyl]-benzoic acid 3-(4-methyl-piperazin-1-yl)-propyl ester
1-(3-Amino-benzyl)-4-(4-isopropyl-phenyl)-6-methoxy-1.H.-quinazolin-2-one
1-(3-Formylamino-benzyl)-4-(4-isopropyl-phenyl)-6-methoxy-1.H.-quinazolin-2-one
1-Benzyl-4-(4-isopropyl-phenyl)-6,7-dimethoxy-1.H.-quinazolin-2-one
1-(2-Fluoro-benzyl)-4-(4-isopropyl-phenyl)-6,7-dimethoxy-1H-quinazolin-2-one
1-(4-Fluoro-benzyl)-4-(4-isopropyl-phenyl)-6,7-dimethoxy-1H-quinazolin-2-one
(2-Benzylamino-5-propargyloxy-phenyl)-(4-isopropyl-phenyl)-methanone
1-Benzyl-4-(4-isopropyl-phenyl)-6-propargyloxy-1.H.-quinazolin-2-one
Acetic acid 4-{[2-(4-isopropyl-benzoyl)-4-propargyloxy-phenylamino]-methyl}-phenyl ester
6-Allyloxy-1-benzyl-4-(4-isopropyl-phenyl)-1.H.-quinazolin-2-one
Acetic acid 4-[6-allyloxy-4-(4-isopropyl-phenyl)-2-oxo-2.H.-quinazolin-1-ylmethyl]-phenyl ester
Acetic acid 4-[4-(4-isopropyl-phenyl)-2-oxo-6-propargyloxy-2.H.-quinazolin-1-ylmethyl]-phenyl ester
1-Benzo[1,2,5]thiadiazol-5-ylmethyl-4-(4-isopropyl-phenyl)-6-propargyloxy-1H-quinazolin-2-one
[2-(2-Hydroxy-benzylamino)-4,5-dimethoxy-phenyl]-(4-isopropyl-phenyl)-methanone
1-(2-Hydroxy-benzyl)-4-(4-isopropyl-phenyl)-6-prop-2-ynyloxy-1H-quinazolin-2-one
1-(2-Hydroxy-benzyl)-4-(4-isopropyl-phenyl)-6,7-dimethoxy-1H-quinazolin-2-one
1-(3-Hydroxy-benzyl)-4-(4-isopropyl-phenyl)-6-prop-2-ynyloxy-1H-quinazolin-2-one
1-(4-Hydroxy-benzyl)-4-(4-isopropyl-phenyl)-6-prop-2-ynyloxy-1H-quinazolin-2-one
1-[2-(6-Chloro-hexyloxy)-benzyl]-4-(4-isopropyl-phenyl)-6-prop-2-ynyloxy-1H-quinazolin-2-one
1-[2-(6-dimethylamino-hexyloxy)-benzyl]-4-(4-isopropyl-phenyl)-6-prop-2-ynyloxy-1H-quinazolin-2-one
1-[2-(6-Imidazol-1-yl-hexyloxy)-benzyl]-4-(4-isopropyl-phenyl)-6-prop-2-ynyloxy-1H-quinazolin-2-one
4-(4-Isopropyl-phenyl)-1-[3-(7-piperidin-1-yl-heptyloxy)-benzyl]-6-prop-2-ynyloxy-1H-quinazolin-2-one (trifluoroacetic acid salt)
(3-Dimethylamino-propyl)-methyl-carbamic acid 4-[4-(4-isopropyl-phenyl)-2-oxo-6-prop-2-ynyloxy-2H-quinazolin-1-ylmethyl]-phenyl ester (trifluoroacetic acid salt)
4-(4-Isopropyl-phenyl)-1-{3-[2-(2-methoxy-ethoxy)-ethoxy]-benzyl}-6-prop-2-ynyloxy-1H-quinazolin-2-one
4-(4-Isopropyl-phenyl)-1-[3-(2-(2-[2-(2-methoxy-ethoxy)-ethoxy]-ethoxy)-ethoxy)-benzyl]-6-prop-2-ynyloxy-1H-quinazolin-2-one
4-(4-Isopropyl-phenyl)-1-[4-(2-{2-[2-(2-methoxy-ethoxy)-ethoxy]-ethoxy}-ethoxy)-benzyl]-6-prop-2-ynyloxy-1H-quinazolin-2-one
4-(4-Isopropyl-phenyl)-1-[3-(2-methoxy-ethoxy)-benzyl]-6-prop-2-ynyloxy-1H-quinazolin-2-one
4-(4-Isopropyl-phenyl)-1-(3-{2-[2-(2-methoxy-ethoxy)-ethoxy]-ethoxy}-benzyl)-6-prop-2-ynyloxy-1H-quinazolin-2-one
4-(4-Isopropyl-phenyl)-1-{2-[2-(2-methoxy-ethoxy)-ethoxy]-benzyl}-6-prop-2-ynyloxy-1H-quinazolin-2-one
1-[3-(2-Hydroxy-ethoxy)-benzyl]-4-(4-isopropyl-phenyl)-6-prop-2-ynyloxy-1H-quinazolin-2-one
4-(4-Isopropyl-phenyl)-6-prop-2-ynyloxy-1-{3-[2-(tetrahydro-pyran-2-yloxy)-ethoxy]-benzyl}-1H-quinazolin-2-one
4-(4-Isopropyl-phenyl)-1-(2-{2-[2-(2-methoxy-ethoxy)-ethoxy]-ethoxy}-benzyl)-6-prop-2-ynyloxy-1H-quinazolin-2-one
Methanesulfonic acid 2-[4-(4-isopropyl-phenyl)-2-oxo-6-prop-2-ynyloxy-2H-quinazolin-1-ylmethyl]-phenyl ester
2-[(3-Dimethylamino-propyl)-methyl-amino]-N-{3-[4-(4-isopropyl-phenyl)-2-oxo-6-propargyloxy-2H-quinazolin-1-ylmethyl]-phenyl}-acetamide
(4-Isopropyl-phenyl)-[2-(3-nitro-benzylamino)-5-propargyloxy-phenyl]-methanone
4-(4-Isopropyl-phenyl)-1-(3-nitro-benzyl)-6-propargyloxy-1H-quinazolin-2-one
1-(3-Amino-benzyl)-4-(4-isopropyl-phenyl)- 6-propargyloxy-1H-quinazolin-2-one
4-bromo-N-{3-[4-(4-isopropyl-phenyl)-2-oxo-6-propargyloxy-2H-quinazolin-1-ylmethyl]-phenyl}-butyramide
N- {3-[4-(4-Isopropyl-phenyl)-2-oxo-6-propargyloxy-2H-quinazolin-1-ylmethyl]-phenyl}-4-[4-(2-methoxy-ethyl)-piperazin-1-yl]-butyramide
4-(4-Isopropyl-phenyl)-1-[3-(2-oxo-pyrrolidin-1-yl)-benzyl]-6-propargyloxy-1H-quinazolin-2-one
2-[(3-Dimethyimino-propyl)-methyl-amino]-N-{3-[4-(4-isopropyl-phenyl)-2-oxo-6-propargyloxy-2H-quinazolin-1-ylmethyl]-phenyl}-acetamide
2-Chloro-N-{3-[4-(4-isopropyl-phenyl)-2-oxo-6-propargyloxy-2H-quinazolin-1-ylmethyl]-phenyl}-acetamide
2-[(3-Dimethylmino-propyl)-methyl-amino]-N-{3-[4-(4-isopropyl-phenyl)-2-oxo-6-propargyloxy-2H-quinazolin-1-ylmethyl]-phenyl}-acetamide
2-(4-Allyl-piperazin-1-yl)-N-{3-[4-(4-isopropyl-phenyl)-2-oxo-6-propargyloxy-2H-quinazolin-1-ylmethyl]-phenyl}-acetamide
N-{3-[4-(4-Isopropyl-phenyl)-2-oxo-6-propargyloxy-2H-quinazolin-1-ylmethyl]-phenyl}-2-(4-methyl-piperazin-1-yl)-acetamide
N-{3-[4-(4-Isopropyl-phenyl)-2-oxo-6-propargyloxy-2H-quinazolin-1-ylmethyl]-phenyl}-2-[4-(2-methoxy-ethyl)-piperazin-1-yl]-acetamide
N-{3-[4-(4-Isopropyl-phenyl)-2-oxo-6-propargyloxy-2H-quinazolin-1-ylmethyl]-phenyl}-2-[4-(2-methoxy-ethyl)-piperazin-1-yl]-N-methyl-acetamide
N-[4-(3-Dimethylamino-propyl)-piperazin-1-yl]-N-{3-[4-(4-isopropyl-phenyl)-2-oxo-6-propargyloxy-2H-quinazolin-1-ylmethyl]-phenyl}-acetamide
N-{3-[4-(4-Cyclopropyl-phenyl)-2-oxo-6-propargyloxy-2H-quinazolin-1-ylmethyl]-phenyl}-2-(4-methyl-piperazin-1-yl)-acetamide
4-[4-(3-Dimethylamino-propyl)-piperazin-1-yl]-N-{3-[4-(4-isopropyl-phenyl)-2-oxo-6-propargyloxy-2H-quinazolin-1-ylmethyl]-phenyl}-butyramide
N-{3-[4-(4-Isopropyl-phenyl)-2-oxo-6-propargyloxy-2H-quinazolin-1-ylmethyl]-phenyl}-4-[(2-methoxy-ethyl)-methyl-amino]-butyramide
N-{3-[4-(4-Isopropyl-phenyl)-2-oxo-6-propargyloxy-2H-quinazolin-1-ylmethyl]-phenyl}-4-morpholin-4-yl-butyramide
N-{3-[6-Allyloxy-4-(4-isopropyl-phenyl)-2-oxo-2H-quinazolin-1-ylmethyl]-phenyl}-4-(4-methylpiperazin-1-yl)-butyramide
4-(4-Isopropyl-phenyl)-1-(4-nitro-benzyl)-6-propargyloxy-1H-quinazolin-2-one
1-(4-Amino-benzyl)-4-(4-isopropyl-phenyl)-6-propargyloxy-1H-quinazolin-2-one
1-(2-Nitro-benzyl)-4-(4-isopropyl-phenyl)-6-propargyloxy-1H-quinazolin-2-one
1-(2-Amino-benzyl)-4-(4-isopropyl-phenyl)-6-propargyloxy-1H-quinazolin-2-one
1-Benzyl-4-(3-chloro-4-isopropyl-phenyl)-6-prop-2-ynyloxy-1H-quinazolin-2-one
(2-Benzylamino-5-prop-2-ynyloxy-phenyl)-(3-chloro-4-isopropylphenyl)-methanone (hydrochloric acid salt)
1-Benzyl-4-(3-chloro-4-isopropyl-phenyl)-6-prop-2-ynyloxy-1H-quinazolin-2-one
1-(3-Fluoro-benzyl)-4-(4-isopropyl-phenyl)-6-methoxy-1.H.-quinazoline-2-thione
1-(4-Chloro-benzyl)-4-(4-isopropyl-phenyl)-6-methoxy-1.H.-quinazolin-2-one
1-(4-Bromo-benzyl)-4-(4-isopropyl-phenyl)-6-methoxy-1.H.-quinazolin-2-one
1-(4-Fluoro-benzyl)-4-(4-isopropyl-phenyl)-6-methoxy-1.H.-quinazolin-2-one
Acetic acid 4-[4-(4-isopropyl-phenyl)-6-methoxy-2-oxo-2.H.-quinazolin-1-ylmethyl]-phenyl ester
4-(4-Isopropyl-phenyl)-6-methoxy-1-(4-methoxy-benzyl)-1.H.-quinazolin-2-one
1-(4-Hydroxy-benzyl)-4-(4-isopropyl-phenyl)-6-methoxy-1.H.-quinazolin-2-one
4-(4-Isopropyl-phenyl)-6-methoxy-1-(4-trifluoromethyl-benzyl)-1.H.-quinazolin-2-one
4-(4-Isopropyl-phenyl)-6-methoxy-1-(4-nitro-benzyl)-1.H.-quinazolin-2-one
4-(4-Isopropyl-phenyl)-6-methoxy-1-(4-methylsulfanyl-benzyl)-1. H.-qu inazolin-2-one
1-(4-Amino-benzyl)-4-(4-isopropyl-phenyl)-6-methoxy-1.H.-quinazolin-2-one
4-[4-(4-Isopropyl-phenyl)-6-methoxy-2-oxo-2.H.-quinazolin-1-ylmethyl]-benzoic acid methyl ester
4-(4-Isopropyl-phenyl)-1-(4-methanesulfonyl-benzyl)-6-methoxy-1.H.-quinazolin-2-one
1-[4-(2-Chloro-ethoxy)-benzyl]-4-(4-isopropyl-phenyl)-6-methoxy-1. H.-quinazolin-2-one
N-(2-Dimethylamino-ethyl)-4-[4-(4-isopropyl-phenyl)-6-methoxy-2-oxo-2.H.-quinazolin-1-ylmethyl]-benzamide
4-[4-(4-Isopropyl-phenyl)-6-methoxy-2-oxo-2.H.-quinazolin-1-ylmethyl]-.N.-(2-pyrrolidin-1-yl-ethyl)-benzamide
N-(2-Ethylamino-ethyl)-4-[4-(4-isopropyl-phenyl)-6-methoxy-2-oxo-2.H.-quinazolin-1-ylmethyl]-benzamide
1-(2-Hydroxy-benzyl)-4-(4-isopropyl-phenyl)-6-methoxy-1.H.-quinazolin-2-one
1-(2-Chloro-benzyl)-4-(4-isopropyl-phenyl)-6-methoxy-1.H.-quinazolin-2-one
4-(4-Isopropyl-phenyl)-6-methoxy-1-(2-methyl-benzyl)-1.H.-quinazolin-2-one
4-(4-Isopropyl-phenyl)-6-methoxy-1-(2-nitro-benzyl)-1.H.-quinazolin-2-one
2-[4-(4-Isopropyl-phenyl)-6-methoxy-2-oxo-2.H.-quinazolin-1-ylmethyl]-benzonitrile
4-(4-Isopropyl-phenyl)-6-methoxy-1-(3-methoxy-benzyl)-1.H.-quinazolin-2-one
3-[4-(4-Isopropyl-phenyl)-6-methoxy-2-oxo-2.H.-quinazolin-1-ylmethyl]-benzonitrile
1-(2,6-Difluoro-benzyl)-4-(4-isopropyl-phenyl)-6-methoxy-1.H.-quinazolin-2-one
1-(2,4-Difluoro-benzyl)-4-(4-isopropyl-phenyl)-6-methoxy-1.H.-quinazolin-2-one
1-(3,4-Difluoro-benzyl)-4-(4-isopropyl-phenyl)-6-methoxy-1.H.-quinazolin-2-one
1-(3,4-Dichloro-benzyl)-4-(4-isopropyl-phenyl)-6-methoxy-1.H.-quinazolin-2-one
4-(4-Isopropyl-phenyl)-6-methoxy-1-(2,4,6-trifluoro-benzyl)-1.H.-quinazolin-2-one
4-(4-Isopropyl-phenyl)-6-methoxy-1-pentafluorophenylmethyl-1.H.-quinazolin-2-one
4-(4-Isopropyl-phenyl)-6-methoxy-1-pyridin-3-ylmethyl-1.H.-quinazolin-2-one
1-(6-Chloro-pyridin-3-ylmethyl)-4-(4-isopropyl-phenyl)-6-methoxy-1.H.-quinazolin-2-one
4-(4-Isopropyl-phenyl)-6-methoxy-1-(5-nitro-furan-2-ylmethyl)-1.H.-quinazolin-2-one
or a pharmaceutically-cleavable ester, or acid addition salt thereof.

In another preferred aspect of the invention, the calcilytic is a compound of formula I: 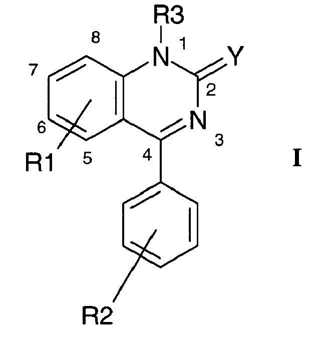 wherein Y is O or S;
R1 represents from 1 to 3 substituents independently selected from OH, SH, halo, NO₂, optionally substituted (lower alkyl, lower alkoxy, lower alkenyl, lower alkenyloxy, lower alkynyl, lower alkynyloxy, lower alkanoyl, cycloalkyl, lower alkylsulphone, lower alkylsulphoxide or amino);
R2 represents from 1 to 3 substituents selected from halo, optionally substituted (lower alkyl, lower alkenyl, cycloalkyl or lower alkoxy);
R3 is
A) lower alkyl optionally substituted by 1 to 3 substituents selected from cycloalkyl, lower alkylene, lower alkyl, Br, F, CF₃, CN, COOH, lower alkyl-carboxylate, OH, lower alkoxy or -Oₓ-(CH₂)_{y}-SO_{z}-lower alkyl, wherein x is 0 or 1, y is 0, 1 or 2 and z is 0, 1 or 2; or
B) Benzyl which is
   a. mono-or di- (preferably mono-) substituted by -Oₓ-(CH₂)_{y}-SO_{z}-lower alkyl or - Oₓ-(CR, R')_{y}-COO-R, wherein x, y and z are as defined above and R or R' is H or lower alkyl,
   b. substituted by 1 or 2 substituents selected from morpholino-lower alkoxy, aryl-lower alkoxy, optionally N-lower alkyl substituted arylamino-lower alkoxy,
   c. substituted at the 2-position by lower alkoxy-, hydroxy-lower alkoxy- or lower alkoxy-lower alkoxy,
   d. substituted on the -CH₂- group thereof; or
C) optionally substituted (aryl-C₂-C₈-alkyl, aryl- C₂-C₈-alkenyl, heteroarylmethyl or 4-heteroarylbenzyl); or
when R1 is 2 substituents one of which is OH, preferably at the 6-position, and the other of which is optionally substituted (lower alkyl, cycloalkyl-lower-alkyl or lower alkenyl), preferably at the 5-position, R3 is H or optionally substituted (lower alkyl, aryl, aryl-lower alkyl, arylcycloalkyl, cycloalkyl-lower alkyl, cycloalkenyl-lower alkyl, hetereoaryl-lower alkyl, hetereoaryl, or carbonyl lower alkyl); or
when R1 is 2-propynyloxy and R2 is isopropyl, R3 is also benzyl which is substituted by 1 to 3 substituents selected from lower alkyl, lower alkoxy, halo, halo-lower alkyl, e.g. CF₃; or when R1 is 2-propynyloxy and R2 is isopropyl, R3 is also benzyl which is substituted by OH and a second and optionally third substituent selected from lower alkyl, lower alkoxy, halo, - O-CH(H or lower alkyl)-COO(H or lower alkyl); or
when R1 is 2-propynyloxy and R2 is cyclopropyl, R3 is also optionally substituted lower alkyl or benzyl (preferably R3 is also benzyl which is substituted by 1 to 3 substituents selected from lower alkyl, lower alkoxy, halo, -O-CH(H or lower alkyl)-COO(H or lower alkyl)); or
when Y is S and R1 is as defined above but not methoxy, R3 is also optionally substituted benzyl; or
a compound selected from 4-(4-isopropyl-phenyl)-1-(3,4-diamino-benzyl)-6-prop-2-ynyloxy-1H-quinazolin-2-one, 1-(2,6-dichloro-benzyl)-4-(4-isopropyl-phenyl)-6-prop-2-ynyloxy-1H-quinazolin-2-one, 1-benzyl-4-(4-isopropyl-phenyl)-6-prop-2-ynyloxy-1H-quinazotine-2-thione; 1-(3,5di-tert-butyl-4-hydroxy-benzyl)-4-(4-isopropyl-phenyl)-6-prop-2-ynyloxy-1H-quinazolin-2-one, or 1-[3-(2-hydroxy-ethoxy)-benzyl]-4-(4-isopropyl-phenyl)-6-prop-2-ynyloxy-1H-quinazoline-2-thione;
or a pharmaceutically-acceptable and -cleavable ester, or acid addition salt thereof; and
provided that when Y is O and R3 is lower alkyl or cycloalkyl, R3 is not isopropyl or cyclopentyl.

In a more preferred aspect of the invention, the calcilytic is a compound of formula I' 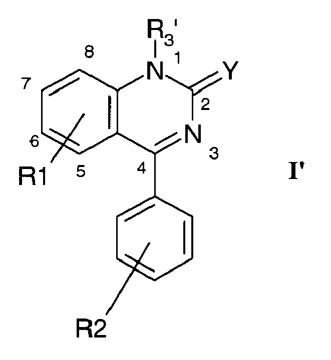 wherein Y is O or S;
R1 and R2 are as defined in claim 1;
R3' is
A) lower alkyl substituted by 1 to 3 substituents independently selected from -S-lower alkyl, lower alkylene, cycloalkyl, Br, F or CF₃; or
B) benzyl which is
   a. mono-or di- (preferably mono-) substituted by -Oₓ-(CH₂)_{y}-SO_{z}-lower alkyl, wherein x is 0 or 1, y is 0, 1 or 2 and z is 0, 1 or 2,
   b. substituted by 1 or 2 substituents selected from morpholino-lower alkoxy, aryl-lower alkoxy, optionally N-lower alkyl substituted arylamino-alkoxy,
   c. substituted at the 2-position by lower alkoxy-, hydroxy-lower alkoxy- or lower alkoxy-lower alkoxy; or
C) optionally substituted (arylvinyl, arylethyl, heteroarylmethyl or 4-heteroarylbenzyl) ; or when R1 is 2 substituents one of which is OH, preferably at the 6-position, and the other of which is optionally substituted (lower alkyl or lower alkenyl), preferably at the 5-position, R3 is H or optionally substituted (lower alkyl, aryl, aryl-lower alkyl, arylcycloalkyl, cycloalkyl-lower alkyl , cycloalkenyl-lower alkyl, hetereoaryl-lower alkyl, hetereoaryl, or carbonyl lower alkyl); or
when R1 is 2-propynyl and R2 is isopropyl, R3 is also benzyl which is substituted by 1 to 3 substituents selected from lower alkyl, lower alkoxy, halo, halo-lower alkyl, e.g. CF₃, -O-CH(H or lower alkyl)-COO(H or lower alkyl); or
when R1 is 2-propynyl and R2 is isopropyl, R3 is also benzyl which is substituted by OH and a second and optionally third subtituent selected from lower alkyl, lower alkoxy, halo, -O-CH(H or lower alkyl)-COO(H or lower alkyl); or
when R1 is 2-propynyl and R2 is cyclopropyl, R3 is also optionally substituted benzyl (preferably R3 is also benzyl which is substituted by 1 to 3 substituents selected from lower alkyl, lower alkoxy, halo, -O-CH(H or lower alkyl)-COO(H or lower alkyl)); or
when X is S and R1 is as defined above but not methoxy, R3 is also optionally substituted benzyl; or
a compound selected from:
4-(4-isopropyl-phenyl)-1-(3,4-diamino-benzyl)-6-prop-2-ynyloxy-1H-quinazolin-2-one,
1-(2,6-dichloro-benzyl)-4-(4-isopropyl-phenyl)-6-prop-2-ynyloxy-1H-quinazolin-2-one,
1-benzyl-4-(4-isopropyl-phenyl)-6-prop-2-ynyloxy-1H-quinazoline-2-thione;
1-(3di-tert-butyl-4-hydroxy-benzyl)-4-(4-isopropyl-phenyl)-6-prop-2-ynyloxy-1H-quinazolin-2-one, or
1-[3-(2-hydroxy-ethoxy)-benzyl]-4-(4-isopropyl-phenyl)-6-prop-2-ynyloxy-1H-quinazoline-2-thione;
or a pharmaceutically-acceptable and -cleavable ester, or acid addition salt thereof; and
provided that when X is O and R3 is lower alkyl or cycloalkyl, R3 is not isopropyl or cyclopentyl.

The calcilytic may for example be a compound selected from the following:
1-(2,3-Dimethoxy-quinoxalin-6-ylmethyl)-4-(4-isopropyl-phenyl)-6-prop-2-ynyloxy-1H-quinazolin-2-one
4-(4-isopropyl-phenyl)-1-(3-methane-sulphonyl-benzyl)-5-propargyloxy-phenyl-methanone
4-(4-Isopropyl-phenyl)-1-(3-methane-sulphonyl-benzyl)-6-propargyloxy-1H-quinazoline-2-one
4-(4-Isopropyl-phenyl)-1-[3-(2-methanesulphinyl-ethoxy)-benzyl]-6-prop-2-ynyloxy-1H-quinazolin-2-one
4-(4-Isopropyl-phenyl)-1-[3-(2-methanesulphonyl-ethoxy)-benzyl]-6-prop-2-ynyloxy-1H-quinazoline-2-one
4-(4-Isopropyl-phenyl)-1-[2-(2-methoxy-ethyl)-2H-tetrazol-5-ylmethyl]-6-prop-2-ynyloxy-1H-quinazolin-2-thione.
4-(4-Isopropyl-phenyl)-1-(3-methane-sulphonyl-benzyl)-5-propargyloxy-phenyl-methanone
4-(4-Isopropyl-phenyl)-1-(3-methane-sulphinyl-benzyl)-6-propargyloxy-1H-quinazoline-2-one
4-(4-Isopropyl-phenyl)-6-prop-2-ynyloxy-1-pyridin-2-ylmethyl-1H-quinazolin-2-one
4-(4-Isopropyl-phenyl)-6-prop-2-ynyloxy-1-(4-[1,2,3]triazol-2-yl-benzyl)-1H-quinazolin-2-one
1-(3-Bromo-propyl)-4-(4-isopropyl-phenyl)-6-prop-2-ynyloxy-1H-quinazolin-2-one
4-(4-Isopropyl-phenyl)-6-prop-2-ynyloxy-1-pyridin-3-ylmethyl-1H-quinazolin-2-one
1-[2-(2-Hydroxy-ethoxy)-benzyl]-4-(4-isopropyl-phenyl)-6-prop-2-ynyloxy-1H-quinazolin-2-one
1-[3-(2-Hydroxy-ethoxy)-thiophen-2-ylmethyl]-4-(4-isopropyl-phenyl)-6-prop-2-ynyloxy-1H-quinazolin-2-one.
1-(3-Chloro-4-hydroxy-5-methoxy-benzyl)-4-(4-isopropyl-phenyl)-6-prop-2-ynyloxy-1H-quinazolin-2-one
1-(2-Ethoxy-benzyl)-4-(4-isopropyl-phenyl)-6-prop-2-ynyloxy-1H-quinazolin-2-one
1-(3-Ethoxy-benzyl)-4-(4-isopropyl-phenyl)-6-prop-2-ynyloxy-1H-quinazolin-2-one
1-(2-Hydroxy-6-methoxy-benzyl)-4-(4-isopropyl-phenyl)-6-prop-2-ynyloxy-1H-quinazolin-2-one
1-(3-Ethoxy-4-methoxy-benzyl)-4-(4-isopropyl-phenyl)-6-prop-2-ynyloxy-1H-quinazolin-2-one
1-(1H-Indol-4-ylmethyl)-4-(4-isopropyl-phenyl)-6-prop-2-ynyloxy-1H-quinazolin-2-one
1-(4-Hydroxy-3-methoxy-benzyl)-4-(4-isopropyl-phenyl)-6-prop-2-ynyloxy-1H-quinazolin-2-one
1-(2-Hydroxy-4-methoxy-benzyl)-4-(4-isopropyl-phenyl)-6-prop-2-ynyloxy-1H-quinazolin-2-one
1-(3-Chloro-4-hydroxy-benzyl)-4-(4-isopropyl-phenyl)-6-prop-2-ynyloxy-1H-quinazoiin-2-one
1-(2-Chloro-benzyl)-4-(4-isopropyl-phenyl)-6-prop-2-ynyloxy-1H-quinazolin-2-one
1-(4-Hydroxy-3,5-dimethoxy-benzyl)-4-(4-isopropyl-phenyl)-6-prop-2-ynyloxy-1H-quinazolin-2-one
1-(2,5-Dimethoxy-benzyl)-4-(4-isopropyl-phenyl)-6-prop-2-ynyloxy-1H-quinazolin-2-one
4-[4-(4-Isopropyl-phenyl)-2-oxo-6-prop-2-ynyloxy-2H-quinazolin-1-ylmethyl]-1H-indole-2-carboxylic acid amide
1-(2-Ethyl-butyl)-4-(4-isopropyl-phenyl)-6-prop-2-ynyloxy-1H-quinazolin-2-one
{3-[4-(4-Isopropyl-phenyl)-2-oxo-6-prop-2-ynyloxy-2H-quinazolin-1-ylmethyl]-phenoxy}-acetic acid
1-(2,3-Dimethoxy-benzyl)-4-(4-isopropyl-phenyl)-6-prop-2-ynyloxy-1H-quinazolin-2-one
1-(2,3-Dihydro-benzo[1,4]dioxin-6-ylmethyl)-4-(4-isopropyl-phenyl)-6-prop-2-ynyloxy-1H-quinazolin-2-one
4-(4-Isopropyl-phenyl)-1-(4-oxo-4H-chromen-3-ylmethyl)-6-prop-2-ynyloxy-1H-quinazolin-2-one
4-(4-Isopropyl-phenyl)-1-(2-methyl-butyl)-6-prop-2-ynyloxy-1H-quinazolin-2-one
1-(2,6-Dichloro-benzyl)-4-(4-isopropyl-phenyl)-6-prop-2-ynyloxy-1H-quinazolin-2-one
1-(2,3-Dichloro-benzyl)-4-(4-isopropyl-phenyl)-6-prop-2-ynyloxy-1H-quinazolin-2-one
4-(4-Isopropyl-phenyl)-6-prop-2-ynyloxy-1-(3-trifluoromethyl-benzyl)-1H-quinazolin-2-one
4-(4-Isopropyl-phenyl)-6-prop-2-ynyloxy-1-(4-trifluoromethyl-benzyl)-1H-quinazolin-2-one
1-(3-Ethoxy-4-hydroxy-benzyl)-4-(4-isopropyl-phenyl)-6-prop-2-ynyloxy-1H-quinazolin-2-one
4-(4-Isopropyl-phenyl)-1-(3-phenyl-butyl)-6-prop-2-ynyloxy-1H-quinazolin-2-one
1-(3,4-Diethoxy-benzyl)-4-(4-isopropyl-phenyl)-6-prop-2-ynyloxy-1H-quinazolin-2-one
1-(3-Fluoro-4-methoxy-benzyl)-4-(4-isopropyl-phenyl)-6-prop-2-ynyloxy-1H-quinazolin-2-one
{4-[4-(4-Isopropyl-phenyl)-2-oxo-6-prop-2-ynyloxy-2H-quinazolin-1-ylmethyl]-phenoxy}-acetic acid
4-(4-Isopropyl-phenyl)-1-(4-methoxy-2,3-dimethyl-benzyl)-6-prop-2-ynyloxy-1H-quinazolin-2-one
1-(4-Benzyloxy-benzyl)-4-(4-isopropyl-phenyl)-6-prop-2-ynyloxy-1H-quinazolin-2-one
1-(3-Hydroxy-6-methyl-pyridin-2-ylmethyl)-4-(4-isopropyl-phenyl)-6-prop-2-ynyloxy-1H-quinazolin-2-one
{2-[4-(4-Isopropyl-phenyl)-2-oxo-6-prop-2-ynyloxy-2H-quinazolin-1-ylmethyl]-6-methoxyphenoxy}-acetic acid
4-(4-Isopropyl-phenyl)-1-(3-methoxy-benzyl)-6-propargyloxy-1H-quinazolin-2-one
4-(4-Isopropyl-phenyl)-1-(3,4-dimethoxy-benzyl)-6-propargyloxy-1H-quinazolin-2-one
4-(4-Isopropyl-phenyl)-1-(4-methoxy-benzyl)-6-propargyloxy-1H-quinazolin-2-one
4-(4-Isopropyl-phenyl)-1-(3,5-dimethoxy-benzyl)-6-propargyloxy-1H-quinazolin-2-one
4-(4-Isopropy)-phenyl)-1-(3,5-dimethoxy-benzyl)-8-propargyloxy-1H-quinazolin-2-one
4-(4-Isopropyl-phenyl)-1-(4-ethoxy-2-hydroxy-benzyl)-6-propargyloxy-1H-quinazolin-2-one
4-(4-Isopropyl-phenyl)-1-(2,4-diethoxy-benzyl)-6-propargyloxy-1H-quinazolin-2-one
4-(4-Isopropyl-phenyl)-1-(2,4-diethoxy-benzyl)-6-propargyloxy-1H-quinazolin-2-one
4-(4-Isopropyl-phenyl)-1-(2-methoxy-benzyl)-6-prop-2-ynyloxy-1H-quinazolin-2-one
4-(4-Isopropyl-phenyl)-1-(4-ethoxy-benzyl)-6-propargyloxy-1H-quinazolin-2-one
4-(4-Isopropyl-phenyl)-1-(3-isopropoxy-benzyl)-6-propargyloxy-1H-quinazolin-2-one
4-(4-Isopropyl-phenyl)-1-(2,4-diethoxy-benzyl)-6-propargyloxy-1H-quinazolin-2-one
1-(4-Bromo-3-methoxy-benzyl)-4-(4-isopropyl-phenyl)-6-propargyloxy-1H-quinazolin-2-one
4-(4-Isopropyl-phenyl)-1-(3-hydroxy-4-methoxy-benzyl)-6-propargyloxy-1H-quinazolin-2-one
4-(4-Isopropyl-phenyl)-1-(2-methoxymethoxy-benzyl)-6-prop-2-ynyloxy-1H-quinazolin-2-one
1-(4-Bromo-3-ethoxy-benzyl)-4-(4-isopropyl-phenyl)-6-propargyloxy-1H-quinazolin-2-one
1-(4-Chloro-4-methoxy-benzyl)-4-(4-isopropyl-phenyl)-6-propargyloxy-1H-quinazolin-2-one
1-(3-Chloro-4-methoxy-benzyl)-4-(4-isopropyl-phenyl)-6-propargyloxy-1H-quinazolin-2-one
1-(3-Chloro-4,5-dimethoxy-benzyl)- 4-(4-isopropyl-phenyl)-6-propargyloxy-1H-quinazolin-2-one
1-(4-Chloro-3-methoxy-benzyl)-4-(4-isopropyl-phenyl)-6-propargyloxy-1H-quinazolin-2-one
1-(3-Fluoro-benzyl)-4-(4-isopropyl-phenyl)-6-propargyloxy-1H-quinazolin-2-one
1-(3,4-Difluoro-benzyl)-4-(4-isopropyl-phenyl)-6-propargyloxy-1H-quinazolin-2-one
1-(4-Chloro-benzyl)-4-(4-isopropyl-phenyl)-6-propargyloxy-1H-quinazolin-2-one
1-(4-Fluoro-benzyl)-4-(4-isopropyl-phenyl)-6-propargyloxy-1H-quinazolin-2-one
1-(3-Chloro-benzyl)-4-(4-isopropyl-phenyl)-6-propargyloxy-1H-quinazolin-2-one
1-(3-Bromo-4-hydroxy-5-methoxy-benzyl)-4-(4-isopropyl-phenyl)-6-propargyloxy-1H-quinazolin-2-one
1-(3-Bromo-4-hydroxy-5-methoxy-benzyl)-4-(4-isopropyl-phenyl)-6-propargyloxy-1H-quinazolin-2-one
1-(4-Bromo-4-hydroxy-5-methoxy-benzyl)-4-(4-isopropyl-phenyl)-6-propargyloxy-1H-quinazolin-2-one
1-(3-Bromo-benzyl)-4-(4-isopropyl-phenyl)-6-propargyloxy-1H-quinazolin-2-one
1-(3-Bromo-4,5-dimethoxy-benzyl)-4-(4-isopropyl-phenyl)-6-propargyloxy-1H-quinazolin-2-one
1-(3,4-Dibromo-benzyl)-4-(4-isopropyl-phenyl)-6-propargyloxy-1H-quinazolin-2-one
1-(3,4-Dichloro-benzyl)-4-(4-isopropyl-phenyl)-6-propargyloxy-1H-quinazolin-2-one
1-(4-Methyl-benzyl)-4-(4-isopropyl-phenyl)-6-propargyloxy-1H-quinazolin-2-one
1-(3-Methyl-benzyl)-4-(4-isopropyl-phenyl)-6-propargyloxy-1H-quinazolin-2-one
1-(4-Ethyl-benzyl)-4-(4-isopropyl-phenyl)-6-propargyloxy-1H-quinazolin-2-one
1-(3,4-Dimethyl-benzyl)-4-(4-isopropyl-phenyl)-6-propargyloxy-1H-quinazolin-2-one
1-Cyclopropylmethyl- 4-(4-isopropyl-phenyl)-6-propargyloxy-1H-quinazolin-2-one
1-(2-Bromo-thiazol-5-ylmethyl)-4-(4-isopropyl-phenyl)-6-prop-2-ynyloxy-1H-quinazolin-2-one
1-(4,5-Dichloro-thiophen-2-ylmethyl)-4-(4-isopropyl-phenyl)-6-prop-2-ynyloxy-1H-quinazolin-2-one
4-(4-Isopropyl-phenyl)-1-(5-methyl-thiophen-2-ylmethyl)-6-prop-2-ynyloxy-1H-quinazolin-2-one
4-(4-Isopropyl-phenyl)-6-prop-2-ynyloxy-1-quinolin-2-ylmethyl-1H-quinazolin-2-one
4-(4-Isopropyl-phenyl)-6-prop-2-ynyloxy-1-[2-(2,6,6-trimethyl-cyclohex-1-enyl)-ethyl]-1H-quinazolin-2-one
4-Ethyl-4-{[2-(4-isopropyl-benzoyl)-4-prop-2-ynyloxy-phenylamino]-methyl}-hexanoic acid
4-(4-Isopropyl-phenyl)-6-propargyloxy-1-(3,3,3-trifluoro-propyl)-1H-quinazolin-2-one
1-(3,3-Dimethyl-butyl)-4-(4-Isopropyl-phenyl)-6-propargyloxy-1H-quinazolin-2-one
1-(2,2-Dimethyl-pent-4-enyl)-4-(4-isopropyl-phenyl)-6-prop-2-ynyloxy-1H- quinazolin-2-one
1-(3,5-Dimethyl-1-phenyl-1H-pyrazol-4-ylmethyl)-4-(4-isopropyl-phenyl)-6-prop-2-ynyloxy-1H-quinazolin-2-one
1-(5-Bromo-thiophen-2-ylmethyl)-4-(4-isopropyl-phenyl)-6-prop-2-ynyloxy-1H-quinazolin-2-one
1-(5-Hydroxymethyl-furan-2-ylmethyl)-4-(4-isopropyl-phenyl)-6-prop-2-ynyloxy-1H-quinazolin-2-one
1-(2-Butyl-5-chloro-1H-imidazol-4-ylmethyl)-4-(4-isopropyl-phenyl)-6-prop-2-ynyloxy-1H-quinazolin-2-one
4-(4-Isopropyl-phenyl)-1-(6-methoxy-pyridin-3-ylmethyl)-6-prop-2-ynyloxy-1H-quinazolin-2-one
7-[4-(4-Isopropyl-phenyl)-2-oxo-6-prop-2-ynyloxy-2H-quinazolin-1-ylmethyl]-1H-indole-2-carbonitrile
1-(2,4-Diamino-pyrimidin-5-ylmethyl)-4-(4-isopropyl-phenyl)-6-prop-2-ynyloxy-1H-quinazolin-2-one
1-(6-Hydroxymethyl-pyridin-2-ylmethyl)-4-(4-isopropyl-phenyl)-6-prop-2-ynyloxy-1H-quinazolin-2-one
1-(3,5-Di-tert-butyl-4-hydroxy-benzyl)-4-(4-isopropyl-phenyl)-6-prop-2-ynyloxy-1H-quinazolin-2-one
4-[4-(4-Isopropyl-phenyl)-2-oxo-6-prop-2-ynyloxy-2H-quinazolin-1-ylmethyl]-2,2-dimethyloxazolidine-3-carboxylic acid tert-butyl ester
4-(4-Isopropyl-phenyl)-1-(4-methylamino-2-methylsulphanyl-pyrimidin-5-ylmethyl)-6-prop-2-ynyloxy-1H-quinazolin-2-one
4-(4-Isopropyl-phenyl)-1-{4-[2-(methyl-pyridin-2-yl-amino)-ethoxy]-benzyl}-6-prop-2-ynyloxy-1H-quinazolin-2-one
4-(4-Isopropyl-phenyl)-1-(2-methyl-hex-4-enyl)-6-prop-2-ynyloxy-1H-quinazolin-2-one
4-(4-Isopropyl-phenyl)-6-prop-2-ynyloxy-1-(4-pyrazin-2-yl-benzyl)-1H-quinazolin-2-one
4-(4-Isopropyl-phenyl)-1-(3-methylsulphanyl-propyl)-6-prop-2-ynyloxy-1H-quinazolin-2-one
4-(4-Isopropyl-phenyl)-6-prop-2-ynyloxy-1-thiophen-2-ylmethyl-1H-quinazolin-2-one
1-Benzyl-4-(4-isopropyl-phenyl)-6-prop-2-ynyloxy-1H-quinazoline-2-thione
4-(4-Isopropyl-phenyl)-1-(3-methane-sulphonyl-benzyl)-6-propargyloxy-1H-quinazoline-2-thione.
4-(4-Isopropyl-phenyl)-6-prop-2-ynyloxy-1-thiophen-2-ylmethyl-1H quinazoline-2-thione
1-[3-(2-Hydroxy-ethoxy)-benzyl]-4-(4-isopropyl-phenyl)-6-prop-2-ynyloxy-1H-quinazoline-2-thione
1-Benzyl-4-(4-isopropyl-phenyl)-6-methoxy-1H-quinazoline-2-thione
4-(4-Isopropyl-phenyl)-1-[2-(2-methoxy-ethoxy)-pyridin-3-ylmethyl]-6-propargyloxy-1H-quinazolin-2-thione
4-(4-Isopropyl-phenyl)-1-[2-(2-methoxy-ethoxy)-pyridin-3-ylmethyl]-6-propargyloxy-1H-quinazolin-2-thione
1-Benzo[12,5]thiadiazol-5-ylmethyl-4-(4-isopropyl-phenyl)-6-propargyloxy-1H-quinazolin-2-thione
Acetic acid 2-{3-[4-(4-isopropyl-phenyl)-6-prop-2-ynyloxy-2-thioxo-2H-quinazolin-1-ylmethyl]-phenoxy}-ethyl ester
1-(2,3-Dimethoxy-quinoxalin-6-ylmethyl)-4-(4-isopropyl-phenyl)-6-prop-2-ynyloxy-1H-quinazoline
1-[3-(2-Hydroxy-ethoxy)-thiophen-2-ylmethyl]-4-(4-isopropyl-phenyl)-6-prop-2-ynyloxy-1H-quinazoline-2-thione
1-Isopropyl-4-(4-isopropyl-phenyl)-6-prop-2-ynyloxy-1H-quinazoline-2-thione.
1-Benzyl-4-(4-cyclopropyl-phenyl)-6-propargyloxy-1H-quinazolin-2-one
4-(4-Cyclopropyl-phenyl)-1-(3,3-dimethyl-butyl)-6-propargyloxy-1H-quinazolin-2-one
4-(4-Cyclopropyl-phenyl)-1-(3-ethoxy-4-methoxy-benzyl)-6-propargyloxy-1H-quinazolin-2-one
4-(4-Cyclopropyl-phenyl)-1-isopropyl-6-propargyloxy-1H-quinazolin-2-one
1-Benzyl-4-(4-cyclopropyl-phenyl)-6-propargyloxy-1H-quinazolin-2-thione
2-{3-[4-(4-Isopropyl-phenyl)-2-oxo-6-propargyloxy-2H-quinazolin-1-ylmethyl]-peony}-butyric acid
2-{3-[4-(4-Isopropyl-phenyl)-2-oxo-6-propargyloxy-2H-quinazolin-1-ylmethyl]-phenoxy}-2-methyl-propionic acid
4-(4-Isopropyl-phenyl)-1-[2-(2-methoxy-ethoxy)-pyridin-3-ylmethyl]-6-prop-2-ynyloxy-1H-quinazolin-2-one
1-[6-Chloro-pyridin-3-ylmethyl]-4-(4-isopropyl-phenyl)-6-propargyloxy-1H-quinazolin-2-one (4-Isopropy-phenyl)-(2-{[6-(2-methoxy-ethoxy)-pyridin-2-yimethyl]-amino}-5-propargyloxyphenyl)-methanone
1- (2-Hydroxy-pyridin-3-ylmethyl)-4-(4-isopropy-phenyl)-6-propargyloxy-1H-quinazolin-2-one
4-(4-Isopropyl-phenyl)-1-(5-methoxy-pyridin-2-ylmethyl)-6-propargyloxy-1H-quinazolin-2-one
4-(4-Isopropyl-phenyl)-1-(6-methyl-pyridin-2-ylmethyl)-6-propargyloxy-1H-quinazolin-2-one
1-(2-Chloro-pyridin-4-ylmethyl)-4-(4-isopropyl-phenyl)-6-propargyloxy-1H-quinazolin-2-one
1-(2-Chloro-pyridin-3-ylmethyl)-4-(4-isopropyl-phenyl)-6-propargyloxy-1H-quinazolin-2-one
4-(4-Isopropyl-phenyl)-1-{6-[2-(2-methoxy-ethoxy)-ethoxy]-pyridin-2-ylmethyl)-6-propargyloxy-1H-quinazolin-2-one
4-(4-Isopropyl-phenyl)-1-[6-(2-methoxy-ethoxy)-ethoxy)-pyridin-2-ylmethyl)-6-propargyloxy-1H-quinazolin-2-one
5-Allyl-1-benzyl-6-hydroxy-4-(4-isopropyl-phenyl)-1H-quinazolin-2-one
5-Allyl-1-[3-(2-chloro-ethoxy)-4-methoxy-benzyl]-6-hydroxy-4-(4-isopropyl-phenyl)-1H-quinazolin-2-one
5-Allyl-6-hydroxy-4-(4-isopropyl-phenyl)-1-thiophen-2-ylmethyl-1H-quinazolin-2-one
5-Allyl-6-hydroxy-4-(4-isopropyl-phenyl)-1-(3-methylsulphanyl-butyl)-1H-quinazolin-2-one
5-Allyl-6-hydroxy-4-(4-isopropyl-phenyl)-1-(1-methyl-2-phenyl-ethyl)-1H-quinazolin-2-one
5-Allyl-6-hydroxy-4-(4-isopropyl-phenyl)-1-pyridin-3-ylmethyl-1H-quinazolin-2-one
5-Allyl-6-hydroxy-1-(4-hydroxy-3-methoxy-benzyl)-4-(4-isopropyl-phenyl)-1H-quinazolin-2-one
5-Allyl-6-hydroxy-1-[2-(2-hydroxy-ethoxy)-benzyl]-4-(4-isopropyl-phenyl)-1H-quinazolin-2-one
5-Allyl-6-hydroxy-1-[3-(2-hydroxy-ethoxy)-benzyl]-4-(4-isopropyl-phenyl)-1H-quinazolin-2-one
5-Allyl-1-(3,5-dimethoxy-benzyl)-6-hydroxy-4-(4-isopropyl-phenyl)-1H-quinazolin-2-one
1-Benzyl-6-hydroxy-4-(4-isopropyl-phenyl)-5-propyl-1H-quinazolin-2-one
6-Hydroxy-1-isobutyl-4-(4-isopropyl-phenyl)-5-propyl-1H-quinazolin-2-one
5-Cyclopropylmethyl-1-(3,3-dimethyl-butyl)-6-hydroxy-4-(4-isopropyl-phenyl)-1H-quinazolin-2-one
1-Benzyl-5-cyclopropylmethyl-6-hydroxy-4-(4-isopropyl-phenyl)-1H-quinazolin-2-one
5-Cyclopropylmethyl-1-(3,4-dimethoxy-benzyl)-6-hydroxy-4-(4-isopropyl-phenyl)-1H-quinazolin-2-one
5-Cyclopropylmethyl-6-hydroxy-4-(4-isopropyl-phenyl)-1-(3-methoxy-benzyl)-1H-quinazolin-2-one
5-Cyclopropylmethyl-1-(3,5-dimethoxy-benzyl)-6-hydroxy-4-(4-isopropyl-phenyl)-1H-quinazolin-2-one
5-Cyclopropylmethyl-6-hydroxy-1-(4-hydroxy-3-methoxy-benzyl)-4-(4-isopropyl-phenyl)-1H-quinazolin-2-one
1-[(3-(3,4-Dimethoxy-phenyl)-propyl)]-4-(4-isopropyl-phenyl)-6-propargyloxy-1H-quinazolin-2-one
4-(4-Isopropyl-phenyl)-1-(3-phenyl-propyl)-6-propargyloxy-1H-quinazolin-2-one
2-[2-(3,4-dimethoxy-phenyl)-2-methyl-propylamino]-4,5-dimethoxy-phenyl}-(4-isopropylphenyl)-methanone
2-[2-(3,5-Dimethoxy-phenyl)-ethylamino]-4,5-dimethoxy-phenyl}-(4-isopropyl-phenyl)-methanone
{4,5-Dimethoxy-2-[2-(3-methoxy-phenyl)-2-methyl-propylamino]-phenyl}-(4-isopropyl-phenyl)-methanone
{2-[2-(3,5-Dimethoxy-phenyl)-2-methyl-propylamino]-5-prop-2-ynyloxy-phenyl}-(4-isopropylphenyl)-methanone
{2-[2-(3,5-Dimethoxy-phenyl)-ethylamino]-5-prop-2-ynyloxy-phenyl}-(4-isopropyl-phenyl)-methanone
{2-[2-(3,4-Dimethoxy-phenyl)-ethylamino]-4,5-dimethoxy-phenyl}-(4-isopropyl-phenyl)-methanone
4-Ethyl-4-{[2-(4-isopropyl-benzoyl)-4,5-dimethoxy-phenylamino]-methyl}-hexanenitrile
1-[2-(3,5-Dimethoxy-phenyl)-ethyl]-4-(4-isopropyl-phenyl)-6-prop-2-ynyloxy-1H-quinazolin-2-one
{2-[2-(3,5-Dimethoxy-phenyl)-2-methyl-propylamino]-4-hydroxy-5-methoxy-phenyl}-(4-isopropyl-phenyl)-methanone
(2-Benzo[1,3]dioxol-5-yl-ethyl)-[5-hydroxy-2-(4-isopropyl-benzoyl)-4-methoxy-phenyl]-ammonium; chloride
[2-(Cyclopropylmethyl-amino)-4-hydroxy-5-methoxy-phenyl]-(4-isopropyl-phenyl)-methanone
1-[2-Hydroxy-2-(2,4,6-trimethyl-phenyl)-ethyl]-4-(4-isopropyl-phenyl)-6-prop-2-ynyloxy-1H-quinazolin-2-one
1-[2-(3,5-Difluoro-phenyl)-2-hydroxy-ethyl]-4-(4-isopropyl-phenyl)-6-prop-2-ynyloxy-1H-quinazolin-2-one
4-(4-Isopropyl-phenyl)-6-prop-2-ynyloxy-1-[(E)-2-(2,4,6-trimethyl-phenyl)-vinyl]-1H-quinazolin-2-one
4-(4-Isopropyl-phenyl)-6-prop-2-ynyloxy-1-((E)-styryl)-1H-quinazolin-2-one
1-[(E)-2-(3-Chloro-4-methoxy-phenyl)-vinyl]-4-(4-isopropyl-phenyl)-6-prop-2-ynyloxy-1H-quinazolin-2-one
1-[(E)-2-(3,5-Dimethyl-phenyl)-vinyl]-4-(4-isopropyl-phenyl)-6-prop-2-ynyloxy-1H-quinazolin-2-one
2-Benzylsulphanyl-4-(4-isopropyl-phenyl)-6-prop-2-ynyloxy-quinazoline
4-(4-Isopropyl-phenyl)-2-isopropylsulphanyl-6-prop-2-ynyloxy-quinazoline 2-Isobutylsulphanyl-4-(4-isopropyl-phenyl)-6-prop-2-ynyloxy-quinazoline.

In an alternative aspect of the invention the calcilytic may be a compound of formula (I) or a pharmaceutically acceptable salt or prodrug ester thereof: 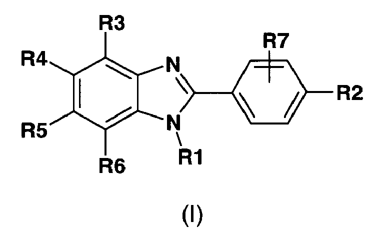 wherein
R1 is selected from the group consisting of optionally substituted (C₁-C₆alkyl, lower alkoxy, lower alkoxy-lower alkyl, cycloalkyloxy-lower alkyl, lower thioalkyl, lower alkylthio-lower alkyl, cycloalkyl, cycloalkyl-lower alkyl, lower alkenyl and lower alkynyl);
R2 is selected from the group consisting of optionally substituted (lower alkyl, cycloalkyl, cycloalkyl-lower alkyl, aryl, heteroaryl, aryl-lower alkyl, heteroaryl-lower alkyl);
R3 is selected from the group consisting of halo, cyano, optionally substituted (lower alkyl, lower alkoxy, lower thioalkyl, lower thioalkenyl, aryl, aryl-lower alkyl, heteroaryl, lower alkenyl, lower alkynyl, heteroaryl, aryl-lower alkyl and heteroaryl-lower alkyl and amino);
R4 is selected from the group consisting of H, halo, cyano, hydroxy, optionally substituted (lower alkyl, lower alkoxy, lower thioalkyl, lower thioalkenyl, aryl, heteroaryl, aryl-lower alkyl, heteroaryl-lower alkyl, alkenyl, alkynyl and amino) and the group having the formula R8-Z-(CH₂)ₙ-;
wherein Z represents a direct bond or is selected from the group consisting of O, NH, CH₂, CO, SO, SO₂ or S;
wherein R8 is selected from the group consisting of optionally substituted (aryl, heteroaryl, carbocyclic aryl, cycloalkyl, heterocycloalkyl);
and wherein n is 0, 1, 2 or 3;
R5 is selected from the group consisting of H, halo, cyano, hydroxyl, optionally substituted (lower alkyl, lower alkoxy, lower alkoxy-lower alkyl, aryl, heteroaryl, aryl-lower alkyl, heteroaryl-lower alkyl, alkenyl, alkynyl and amino);
R6 is selected from the group consisting of halo, cyano, optionally substituted (lower alkyl, lower alkoxy, lower thioalkyl, lower alkenyl, lower alkynyl, lower alkoxy-lower alkyl, aryl, heteroaryl, aryl-lower alkyl, heteroaryl-lower alkyl and amino);
R7 represents one or more substituents independently selected from the group consisting of H, halo, hydroxyl, optionally substituted (lower alkyl, lower alkoxy, amino, cyano, and carbonyl);
the optional substituent or substituents on R1-R8 being independently selected from the group consisting of halogen, hydroxy, lower alkyl, mono or di-lower alkylamino, aminocarbonyl, sulfinyl, sulfonyl, sulfanyl, mono or di-lower alkylaminocarbonyl, amino, carboxy, lower alkoxy, C₃-C₁₂ cycloalkyl, C₃-C₁₈ heterocycloalkyl, lower alkylcarbonyl, lower alkoxycarbonyl, nitryl, aryl; all of which, except halogen, are independently optionally substituted by one or more substituents, selected from the group consisting of halogen, hydroxy, lower alkyl, mono or di-lower alkylamino, aminocarbonyl, sulfinyl, sulfonyl, sulfanyl, mono or di-lower alkylaminocarbonyl, amino, carboxy, lower alkoxy, C₃-C₁₂ cycloalkyl, C₃-C₁₈ heterocycloalkyl, lower alkylcarbonyl, lower alkoxycarbonyl, nitryl, aryl.

Yet more preferably the calcilytic may be a compound of formula (I') or a pharmaceutically acceptable salt, or prodrug ester thereof: 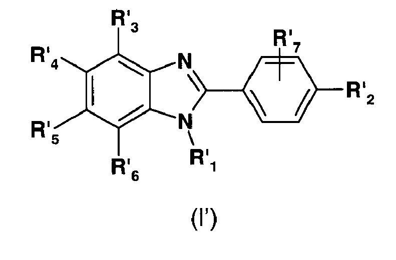 wherein
R'₁ is selected from the group consisting of optionally substituted (C₁-C₆ alkyl, lower alkoxy-lower alkyl, lower alkynyl, lower thioalkyl-lower alkyl, cycloalkyl-lower alkyl);
R'₂ is lower alkyl;
R'₃ is selected from the group consisting of halo, cyano, optionally substituted (lower alkyl, lower alkoxy, lower thioalkyl, lower thioalkenyl, lower alkynyl, aryl and aryl-lower alkyl);
R'₄ is selected from the group consisting of H, halo, cyano, optionally substituted (lower alkyl, aryl, aryl-lower alkyl, heteroaryl, heteroaryl-lower alkyl) and the group having the formula R'₈-Z(CH₂)ₙ-;
wherein Z represents a direct bond or is selected from the group consisting of O, NH, CH₂, CO, SO, SO₂ or S;
wherein R'₈ is selected from the group consisting of optionally substituted (aryl, pyrazolyl, thiazolyl, cyclobutyl, tetrazolyl, pyridyl, indazolyl, pyrazinyl, furanyl, isoxazolyl, pyrrolidinyl, benzimidazolyl, imidazolyl, oxazolyl);
and wherein n is 0, 1, 2 or 3;
R'₅ is H, halo, or lower alkyl;
R'₆ is selected from the group consisting of halo, optionally substituted (lower alkyl, lower alkoxy, lower alkenyl, lower alkynyl);
R'₇ represents one or more substituents independently selected from the group consisting of H, halo, hydroxyl, optionally substituted (lower alkyl, lower alkoxy, amino, cyano, and carbonyl);
the optional substituent or substituents on R'₁-R'₈ being independently selected from the group consisting of halogen, hydroxy, lower alkyl, mono or di-lower alkylamino, aminocarbonyl, sulfinyl, sulfonyl, sulfanyl, mono or di-lower alkylaminocarbonyl, amino, carboxy, lower alkoxy, C₃-C₁₂ cycloalkyl, C₃-C₁₈ heterocycloalkyl, lower alkylcarbonyl, lower alkoxycarbonyl, nitryl, aryl; all of which, except halogen, are independently optionally substituted by one or more substituents, selected from the group consisting of halogen, hydroxy, lower alkyl, mono or di-lower alkylamino, aminocarbonyl, sulfinyl, sulfonyl, mono or di-lower alkylaminocarbonyl, amino, carboxy, lower alkoxy, C₃-C₁₂ cycloalkyl, C₃-C₁₈ heterocycloalkyl, lower alkylcarbonyl, lower alkoxycarbonyl, nitryl, aryl.

For example, the calcilytic may be a compound selected from:
4-Bromo-2-(4-isopropyl-phenyl)-7-methoxy-1-(2-methoxy-ethyl)-1H-benzoimidazole
4-lodo-2-(4-isopropyl-phenyl)-7-methoxy-1-(2-methoxy-ethyl)-1H-benzoimidazole
4-lodo-2-(4-isopropyl-phenyl)-7-methoxy-1-(2-methylsulfanyl-ethyl)-1H-benzoimidazole
4-Bromo-2-(4-isopropyl-phenyl)-7-methoxy-1-(2-methylsulfanyl-ethyl)-1H-benzoimidazole
4-Bromo-1-cyclopropylmethyl-2-(4-isopropyl-phenyl)-7-methoxy-1H-benzoimidazole
4-Bromo-1-propyl-2-(4-isopropyl-phenyl)-7-methoxy-1H-benzoimidazole
4-Bromo-1-butyl-2-(4-isopropyl-phenyl)-7-methoxy-1H-benzoimidazole
4-Bromo-1-ethyl-2-(4-isopropyl-phenyl)-7-methoxy-1H-benzoimidazole
{2-[4-Bromo-2-(4-isopropyl-phenyl)-7-methoxy-benzoimidazol-1-yl]-ethyl}-dimethyl-amine
4-Chloro-2-(4-isopropyl-phenyl)-7-methoxy-1-(2-methoxy-ethyl)-1H-benzoimidazole
4-Ethynyl-2-(4-isopropyl-phenyl)-7-methoxy-1-(2-methoxy-ethyl)-1H-benzoimidazole
2-(4-Isopropyl-phenyl)-7-methoxy-1-(2-methoxy-ethyl)-4-phenyl-1H-benzoimidazole
3-[2-(4-Isopropyl-phenyl)-7-methoxy-1-(2-methoxy-ethyl)-1H-benzoimidazol-4-yl]-phenol
2-(4-Isopropyl-phenyl)-7-methoxy-4-[3-(2-methoxy-ethoxy)-phenyl]-1-(2-methoxy-ethyl)-1H-benzoimidazole
4-(3,5-Dimethoxy-phenyl)-2-(4-isopropyl-phenyl)-7-methoxy-1-(2-methoxy-ethyl)-1H-benzoimidazole
4-Methyl-2-(4-isopropyl-phenyl)-7-methoxy-1-(2-methoxy-ethyl)-1H-benzoimidazole
4-Ethyl-2-(4-isopropyl-phenyl)-7-methoxy-1-(2-methoxy-ethyl)-1H-benzoimidazole
4-Ethylsulfanyl-2-(4-isopropyl-phenyl)-7-methoxy-1-(2-methoxy-ethyl)-1H-benzoimidazole
4-Bromo-2-(4-cyclopropyl-phenyl)-7-methoxy-1-(2-methoxy-ethyl)-1H-benzoimidazole
4-Bromo-2-(4-cyclopropyl-phenyl)-7-methoxy-1-(2-methylsulfanyl-ethyl)-1H-benzoimidazole
4-Bromo-1-cyclopropylmethyl-2-(4-cyclopropyl-phenyl)-7-methoxy-1H-benzoimidazole
5-Bromo-2-(4-isopropyl-phenyl)-7-methoxy-1-(2-methoxy-ethyl)-1H-benzoimidazole
4,5-Dibromo-2-(4-isopropyl-phenyl)-7-methoxy-1-(2-methoxy-ethyl)-1H-benzoimidazole
4,5-Dibromo-2-(4-cyclopropyl-phenyl)-7-methoxy-1-(2-methoxy-ethyl)-1H-benzoimidazole
4,5-Dibromo-2-(4-isopropyl-2-methoxy-phenyl)-7-methoxy-1-(2-methoxy-ethyl)-1H-benzoimidazole
4-Iodo-5-bromo-2-(4-isopropyl-phenyl)-7-methoxy-1-(2-methoxy-ethyl)-1H-benzoimidazole
5-Bromo-4-iodo-2-(4-isopropyl-2-methoxy-phenyl)-7-methoxy-1-(2-methoxy-ethyl)-1H-benzoimidazole
4-Bromo-2-(4-isopropyl-phenyl)-7-methoxy-1-(2-methoxy-ethyl)-5-trifluoromethyl-1H-benzoimidazole
4-Bromo-1-cyclopropylmethyl-2-(4-isopropyl-phenyl)-7-methoxy-5-trifluoromethyl-1H-benzoimidazole
4-Bromo-5-iodo-2-(4-isopropyl-phenyl)-7-methoxy-1-(2-methoxy-ethyl)-1H-benzoimidazole
5-Bromo-4-ethynyl-2-(4-isopropyl-phenyl)-7-methoxy-1-(2-methoxy-ethyl)-1H-benzoimidazole
2-(4-Isopropyl-phenyl)-7-methoxy-1-(2-methoxy-ethyl)-1H-benzoimidazole-5-carbonitrile
4-Bromo-2-(4-isopropyl-phenyl)-7-methoxy-1-(2-methoxy-ethyl)-1H-benzoimidazole-5-carbonitrile
4-Bromo-5-fluoro-2-(4-isopropyl-phenyl)-7-methoxy-1-(2-methoxy-ethyl)-1H-benzoimidazole
5-Benzyl-4-bromo-2-(4-isopropyl-phenyl)-7-methoxy-1-(2-methoxy-ethyl)-1H-benzoimidazole
5-Benzyl-4-iodo-2-(4-isopropyl-phenyl)-7-methoxy-1-(2-methoxy-ethyl)-1H-benzoimidazole
5-Benzyl-4-ethynyl-2-(4-isopropyl-phenyl)-7-methoxy-1-(2-methoxy-ethyl)-1H-benzoimidazole
4-Ethynyl-2-(4-isopropyl-phenyl)-7-methoxy-5-(2-methoxy-benzyl)-1-(2-methoxy-ethyl)-1H-benzoimidazole
4-Bromo-5-ethyl-2-(4-isopropyl-phenyl)-7-methoxy-1-(2-methoxy-ethyl)-1H-benzoimidazole
4-Bromo-5-cyclobutylmethyl-2-(4-isopropyl-phenyl)-7-methoxy-1-(2-methoxy-ethyl)-1H-benzoimidazole
4-Bromo-5-(3-fluoro-benzyl)-2-(4-isopropyl-phenyl)-7-methoxy-1-(2-methoxy-ethyl)-1H-benzoimidazole
4-Bromo-5-(3-chloro-benzyl)-2-(4-isopropyl-phenyl)-7-methoxy-1-(2-methoxy-ethyl)-1H-benzoimidazole
4-Bromo-2-(4-isopropyl-phenyl)-7-methoxy-1-(2-methoxy-ethyl)-5-thiazol-2-ylmethyl-1H-benzoimidazole
4-Bromo-5-(3,5-difluoro-benzyl)-2-(4-isopropyl-phenyl)-7-methoxy-1-(2-methoxy-ethyl)-1H-benzoimidazole
4-Bromo-2-(4-isopropyl-phenyl)-7-methoxy-1-(2-methoxy-ethyl)-5-pyridin-3-ylmethyl-1H-benzoimidazole
4-Bromo-2-(4-isopropyl-phenyl)-7-methoxy-1-(2-methoxy-ethyl)-5-(2-methylsulfanyl-benzyl)-1H-benzoimidazole
4-Bromo-2-(4-isopropyl-phenyl)-5-(2-methanesulfinyl-benzyl)-7-methoxy-1-(2-methoxyethyl)-1H-benzoimidazole
4-Bromo-2-(4-isopropyl-phenyl)-5-(2-methanesulfonyl-benzyl)-7-methoxy-1-(2-methoxyethyl)-1H-benzoimidazole
4-Bromo-2-(4-isopropyl-phenyl)-7-methoxy-1-(2-methoxy-ethyl)-5-pyridin-2-ylmethyl-1H-benzoimidazole
4-lodo-2-(4-isopropyl-phenyl)-7-methoxy-5-(2-methoxy-benzyl)-1-(2-methoxy-ethyl)-1H-benzoimidazole
4-Bromo-2-(4-isopropyl-phenyl)-7-methoxy-5-(2-methoxy-benzyl)-1-(2-methoxy-ethyl)-1H-benzoimidazole
4-Bromo-5-(3,4-dimethoxy-benzyl)-2-(4-isopropyl-phenyl)-7-methoxy-1-(2-methoxy-ethyl)-1H-benzoimidazole
4-Bromo-2-(4-isopropyl-phenyl)-7-methoxy-1-(2-methoxy-ethyl)-5-(3-methoxy-pyridin-2-ylmethyl)-1H-benzoimidazole
5-Benzyl-4-ethyl-2-(4-isopropyl-phenyl)-7-methoxy-1-(2-methoxy-ethyl)-1H-benzoimidazole
4-Bromo-2-(4-isopropyl-phenyl)-7-methoxy-5-(3-methoxy-benzyl)-1-(2-methoxy-ethyl)-1H-benzoimidazole
[4-Bromo-2-(4-isopropyl-phenyl)-7-methoxy-1-(2-methoxy-ethyl)-1H-benzoimidazol-5-yl]-(3-methoxy-phenyl)-methanone
[4-Bromo-2-(4-isopropyl-phenyl)-7-methoxy-1-(2-methoxy-ethyl)-1H-benzoimidazol-5-yl]-(2-methoxy-phenyl)-methanone
4-Bromo-2-(4-isopropyl-phenyl)-7-methoxy-1-(2-methoxy-ethyl)-5-(1-phenyl-ethyl)-1H-benzoimidazole
4-lodo-2-(4-isopropyl-phenyl)-7-methoxy-1-(2-methoxy-ethyl)-1H-benzoimidazole-5-carbonitrile
2-(4-Isopropyl-phenyl)-7-methoxy-1-(2-methoxy-ethyl)-1H-benzoimidazole-4-carbonitrile
4-Isobutyl-2-(4-isopropyl-phenyl)-7-methoxy-1-(2-methoxy-ethyl)-1H-benzoimidazole
4-Benzyl-2-(4-isopropyl-phenyl)-7-methoxy-1-(2-methoxy-ethyl)-1H-benzoimidazole
4,7-Dibromo-2-(4-isopropyl-phenyl)-1-(2-methoxy-ethyl)-1H-benzoimidazole
4,7-Dibromo-2-(4-isopropyl-phenyl)-1-(2-methoxy-ethyl)-1H-benzoimidazole
4-Bromo-2-(4-isopropyl-phenyl)-7-methoxy-1-(2-methoxy-ethyl)-5-phenyl-1H-benzoimidazole
4-Bromo-5-(3,4-dimethoxy-phenyl)-2-(4-isopropyl-phenyl)-7-methoxy-1-(2-methoxy-ethyl)-1H-benzoimidazole
3-[4-Bromo-2-(4-isopropyl-phenyl)-7-methoxy-1-(2-methoxy-ethyl)-1H-benzoimidazol-5-yl]-phenol
4-Bromo-2-(4-isopropyl-phenyl)-7-methoxy-1-(2-methoxy-ethyl)-5-(3-methoxy-phenyl)-1H-benzoimidazole
3-[4-Bromo-2-(4-isopropyl-phenyl)-7-methoxy-1-(2-methoxy-ethyl)-1H-benzoimidazol-5-yl]-benzoic acid ethyl ester
4-[4-Bromo-2-(4-isopropyl-phenyl)-7-methoxy-1-(2-methoxy-ethyl)-1H-benzoimidazol-5-yl]-benzoic acid ethyl ester
4-Bromo-2-(4-isopropyl-phenyl)-7-methoxy-1-(2-methoxy-ethyl)-5-pyridin-3-yl-1H-benzoimidazole
3-[4-Bromo-2-(4-isopropyl-phenyl)-7-methoxy-1-(2-methoxy-ethyl)-1H-benzoimidazol-5-yl]-benzonitrile
1-{5-[4-Bromo-2-(4-isopropyl-phenyl)-7-methoxy-1-(2-methoxy-ethyl)-1H-benzoimidazol-5-yl]-2-methoxy-phenyl}-ethanone
2-[4-Bromo-2-(4-isopropyl-phenyl)-7-methoxy-1-(2-methoxy-ethyl)-1H-benzoimidazol-5-yl]-benzonitrile
2-(4-Isopropyl-phenyl)-7-methoxy-1-(2-methoxy-ethyl)-5-(3-methoxy-phenyl)-1H-benzoimidazole
4-lodo-2-(4-isopropyl-phenyl)-7-methoxy-1-(2-methoxy-ethyl)-5-pyridin-4-yl-1H-benzoimidazole
4-Bromo-2-(4-isopropyl-phenyl)-7-methoxy-1-(2-methoxy-ethyl)-5-(4-methyl-pyrazol-1-ylmethyl)-1H-benzoimidazol
4-Bromo-5-imidazol-1-ylmethyl-2-(4-isopropyl-phenyl)-7-methoxy-1-(2-methoxy-ethyl)-1H-benzoimidazole
4-Bromo-5-(4-bromo-5-methyl-pyrazol-1-ylmethyl)-2-(4-isopropyl-phenyl)-7-methoxy-1-(2-methoxy-ethyl)-1H-benzoimidazole
4-Bromo-5-(4-bromo-3-methyl-pyrazol-1-ylmethyl)-2-(4-isopropyl-phenyl)-7-methoxy-1-(2-methoxy-ethyl)-1H-benzoimidazole
4-Bromo-5-(3,5-dimethyl-pyrazol-1-ylmethyl)-2-(4-isopropyl-phenyl)-7-methoxy-1-(2-methoxy-ethyl)-1H-benzoimidazole
1-[4-Bromo-1-(2-hydroxy-ethyl)-2-(4-isopropyl-phenyl)-7-methoxy-1H-benzoimidazol-5-ylmethyl]-1H-imidazole-2-carboxylic acid ethyl ester
4-Bromo-2-(4-isopropyl-phenyl)-7-methoxy-1-(2-methoxy-ethyl)-5-(2-methoxymethylimidazol-1-ylmethyl)-1H-benzoimidazole
4-Bromo-2-(4-isopropyl-phenyl)-7-methoxy-1-(2-methoxy-ethyl)-5-(2-methylsulfanyl-imidazol-1-ylmethyl)-1H-benzoimidazole
1-[4-Bromo-2-(4-isopropyl-phenyl)-7 -methoxy-1-(2-methoxy-ethyl)-1H-benzoimidazol-5-ylmethyl]-1H-benzoimidazol-2-ol
4-Bromo-2-(4-isopropyl-phenyl)-7-methoxy-1-(2-methoxy-ethyl)-5-(2-methylsulfanylbenzoimidazol-1-ylmethyl)-1H-benzoimidazole
4-Bromo-2-(4-isopropyl-phenyl)-5-(2-methanesulfinyl-benzoimidazol-1-ylmethyl)-7-methoxy-1-(2-methoxy-ethyl)-1H-benzoimidazole
4-Bromo-2-(4-isopropyl-phenyl)-7-methoxy-5-(2-methoxy-benzoimidazol-1-ylmethyl)-1-(2-methoxy-ethyl)-1H-benzoimidazole
3-[4-Bromo-1-(2-hydroxy-ethyl)-2-(4-isopropyl-phenyl)-7-methoxy-1H-benzoimidazol-5-ylmethyl]-3H-imidazole-4-carboxylic acid methyl ester
2-[4-Bromo-5-imidazo[4,5-b]pyridin-3-ylmethyl-2-(4-isopropyl-phenyl)-7-methoxybenzoimidazol-1-yl]-ethanol
2-[4-Bromo-5-indazol-1-ylmethyl-2-(4-isopropyl-phenyl)-7-methoxy-benzoimidazol-1-yl]-ethanol
2-[4-Bromo-2-(4-isopropyl-phenyl)-7-methoxy-5-(5-methyl-tetrazol-2-ylmethyl)-benzoimidazol-1-yl]-ethanol
4-Bromo-5-(4-bromo-5-methyl-pyrazol-1-ylmethyl)-2-(4-cyclopropyl-phenyl)-7-methoxy-1-(2-methoxy-ethyl)-1H-benzoimidazole
4-Bromo-2-(4-isopropyl-phenyl)-7-methoxy-5-(4-methyl-pyrazol-1-ylmethyl)-1-(2-methylsulfanyl-ethyl)-1H-benzoimidazole
4-Bromo-5-isopropoxymethyl-2-(4-isopropyl-phenyl)-7-methoxy-1-(2-methoxy-ethyl)-1H-benzoimidazole
1-[4-Bromo-2-(4-isopropyl-phenyl)-7-methoxy-1-(2-methoxy-ethyl)-1H-benzoimidazol-5-ylmethyl]-pyrrolidin-2-one
4-Bromo-2-(4-isopropyl-phenyl)-7-methoxy-1-(2-methoxy-ethyl)-5-phenylsulfanyl-1H-benzoimidazole
5-Benzenesulfinyl-4-bromo-2-(4-isopropyl-phenyl)-7-methoxy-1-(2-methoxy-ethyl)-1H-benzoimidazole
5-Benzyl-2-(4-isopropyl-phenyl)-7-methoxy-1-(2-methoxy-ethyl)-4-trifluoromethyl-1H-benzoimidazole
2-(4-Isopropyl-phenyl)-7-methoxy-5-(2-methoxy-benzyl)-1-(2-methoxy-ethyl)-4-trifluoromethyl-1H-benzoimidazole
2-(4-Isopropyl-phenyl)-7-methoxy-1-(2-methoxy-ethyl)-5-pyridin-2-ylmethyl-4-trifluoromethyl-1H-benzoimidazole
2-(4-Isopropyl-phenyl)-7-methoxy-1-(2-methoxy-ethyl)-5-pyrazol-1-ylmethyl-4-trifluoromethyl-1H-benzoimidazole
4-Bromo-2-(4-isopropyl-phenyl)-7-methoxy-1-(2-methoxy-ethyl)-5-phenoxy methyl-1H-benzoimidazole
2-{2-[4-Bromo-2-(4-isopropyl-phenyl)-7-methoxy-1-(2-methoxy-ethyl)-1H-benzoimidazol-5-ylmethoxy]-phenyl}-ethanol
2-{2-[4-Bromo-2-(4-isopropyl-phenyl)-7-methoxy-1-(2-methoxy-ethyl)-1H-benzoimidazol-5-ylmethoxy]-phenoxy}-ethanol
{2-[4-Bromo-2-(4-isopropyl-phenyl)-7-methoxy-1-(2-methoxy-ethyl)-1H-benzoimidazol-5-ylmethoxy]-phenyl}-methanol
N-{2-[4-Bromo-2-(4-isopropyl-phenyl)-7-methoxy-1-(2-methoxy-ethyl)-1H-benzoimidazol-5-ylmethoxy]-phenyl}-acetamide
2-[4-Bromo-2-(4-isopropyl-phenyl)-7-methoxy-1-(2-methoxy-ethyl)-1H-benzoimidazol-5-ylmethoxy]-benzamide
2-[4-Bromo-2-(4-isopropyl-phenyl)-7-methoxy-1-(2-methoxy-ethyl)-1H-benzoimidazol-5-ylmethoxy]-benzenesulfonamide
2-[4-Bromo-2-(4-isopropyl-phenyl)-7-methoxy-1-(2-methoxy-ethyl)-1H-benzoimidazol-5-ylmethoxy]-phenylamine
1-{2-[4-Bromo-2-(4-isopropyl-phenyl)-7-methoxy-1-(2-methoxy-ethyl)-1H-benzoimidazol-5-ylmethoxy]-phenyl}-ethanone
2-[4-Bromo-2-(4-isopropyl-phenyl)-7-methoxy-1-(2-methoxy-ethyl)-1H-benzoimidazol-5-ylmethoxy]-phenol
2-[4-Bromo-2-(4-isopropyl-phenyl)-7-methoxy-1-(2-methoxy-ethyl)-1H-ben zoimidazol-5-ylmethoxy]-pyridin-3-ol
4-Bromo-2-(4-isopropyl-phenyl)-7-methoxy-1-(2-methoxy-ethyl)-5-(pyridin-2-yloxymethyl)-1H-benzoimidazole
4-Bromo-2-(4-isopropyl-phenyl)-7-methoxy-1-(2-methoxy-ethyl)-5-(2-methoxy-phenoxymethyl)-1H-benzoimidazole
{3-[4-Bromo-2-(4-isopropyl-phenyl)-7-methoxy-1-(2-methoxy-ethyl)-1H-benzoimidazol-5-ylmethoxy]-2-methyl-phenyl}-methanol
4-Bromo-2-(4-isopropyl-phenyl)-7-methoxy-1-(2-methoxy-ethyl)-5-(pyridin-3-yloxymethyl)-1H-benzoimidazole
4-Bromo-2-(4-isopropyl-phenyl)-5-(2-methanesulfonyl-phenoxymethyl)-7-m ethoxy-1-(2-methoxy-ethyl)-1H-benzoimidazole
2-{3-[4-Bromo-2-(4-isopropyl-phenyl)-7-methoxy-1-(2-methoxy-ethyl)-1H-benzoimidazol-5-ylmethoxy]-phenoxy}-ethanol
2-{2-[4-Bromo-2-(4-isopropyl-phenyl)-7-methoxy-1-(2-methoxy-ethyl)-1H-benzoimidazol-5-ylmethoxy]-phenyl}-acetamide
2-{2-[2-(4-Isopropyl-phenyl)-7-methoxy-1-(2-methoxy-ethyl)-4-trifluoromethyl-1H-benzoimidazol-5-ylmethoxy]-phenoxy}-ethanol
2-{2-[2-(4-Isopropyl-phenyl)-7-methoxy-1-(2-methoxy-ethyl)-4-trifluoromethyl-1H-benzoimidazol-5-ylmethoxy]-phenyl}-ethanol
[4-Bromo-2-(4-isopropyl-phenyl)-7-methoxy-1-(2-methoxy-ethyl)-1H-benzo imidazol-5-ylmethyl]-phenyl-amine
[4-Bromo-2-(4-isopropyl-phenyl)-7-methoxy-1-(2-methoxy-ethyl)-1H-benzo imidazol-5-ylmethyl]-(2-methanesulfonyl-phenyl)-amine
[4-Bromo-2-(4-isopropyl-phenyl)-7-methoxy-1-(2-methoxy-ethyl)-1H-benzo imidazol-5-ylmethyl]-[2-(2-methanesulfonyl-ethyl)-phenyl]-amine
2-(2-{[4-Bromo-2-(4-isopropyl-phenyl)-7-methoxy-1-(2-methoxy-ethyl)-1H-benzoimidazol-5-ylmethyl]-amino}-phenyl)-acetamide
2-{[4-Bromo-2-(4-isopropyl-phenyl)-7-methoxy-1-(2-methoxy-ethyl)-1H-be nzoimidazol-5-ylmethyl]-amino}-benzenesulfonic acid
[4-Bromo-2-(4-isopropyl-phenyl)-7-methoxy-1-(2-methoxy-ethyl)-1H-benzo imidazol-5-ylmethyl]-(2-fluoro-phenyl)-amine
[4-Bromo-2-(4-isopropyl-phenyl)-7-methoxy-1-(2-methoxy-ethyl)-1H-benzo imidazol-5-ylmethyl]-pyridin-2-yl-amine
2-{[4-Bromo-2-(4-isopropyl-phenyl)-7-methoxy-1-(2-methoxy-ethyl)-1H-benzoimidazol-5-ylmethyl]-amino}-benzoic acid methyl ester
[4-Bromo-2-(4-isopropyl-phenyl)-7-methoxy-1-(2-methoxy-ethyl)-1H-benzo imidazol-5-ylmethyl]-pyridin-3-yl-amine
[4-Bromo-2-(4-isopropyl-phenyl)-7-methoxy-1-(2-methoxy-ethyl)-1H-benzo imidazol-5-ylmethyl]-methyl-phenyl-amine
[4-Bromo-2-(4-isopropyl-phenyl)-7-methoxy-1-(2-methoxy-ethyl)-1H-benzo imidazol-5-ylmethyl]-(3-methanesulfonyl-phenyl)-amine
2-(2-{[2-(4-Isopropyl-phenyl)-7-methoxy-1-(2-methoxy-ethyl)-4-trifluoromethyl-1H-benzoimidazol-5-ylmethyl]-amino}-phenyl)-acetamide
[2-(4-Isopropyl-phenyl)-7-methoxy-1-(2-methoxy-ethyl)-4-trifluoromethyl-1H-benzoimidazol-5-ylmethyl]-(2-methanesulfonyl-phenyl)-amine
[2-(4-Isopropyl-phenyl)-7-methoxy-1-(2-methoxy-ethyl)-4-trifluoromethyl-1H-benzoimidazol-5-ylmethyl]-[2-(2-methanesulfonyl-ethyl)-phenyl]-amine
1-[4-Bromo-2-(4-isopropyl-phenyl)-7-methoxy-1-(2-methoxy-ethyl)-1H-benzoimidazol-5-ylmethyl]-1H-imidazole-2-carboxylic acid methyl ester
1-[4-Bromo-2-(4-isopropyl-phenyl)-7-methoxy-1-(2-methoxy-ethyl)-1H-benzoimidazol-5-ylmethyl]-1H-imidazole-2-carboxylic acid dimethylamide
1-{1-[4-Bromo-2-(4-isopropyl-phenyl)-7-methoxy-1-(2-methoxy-ethyl)-1H-benzoimidazol-5-ylmethyl]-1H-imidazol-2-yl}-ethanone
1-[4-Bromo-2-(4-isopropyl-phenyl)-7-methoxy-1-(2-methoxy-ethyl)-1H-benzoimidazol-5-ylmethyl]-1H-indole-2,3-dione
4-Bromo-2-(4-isopropyl-phenyl)-7-methoxy-1-(2-methoxy-ethyl)-5-oxazol-2-ylmethyl-1H-benzoimidazole
1-[4-Bromo-2-(4-isopropyl-phenyl)-7-methoxy-1-(2-methoxy-ethyl)-1H-benzoimidazol-5-ylmethyl]-1H-imidazole-2-carbonitrile
1-[4-Bromo-2-(4-isopropyl-phenyl)-7-methoxy-1-(2-methoxy-ethyl)-1H-benzoimidazol-5-ylmethyl]-1H-imidazole-2-carboxylic acid methylamide
2-(4-Isopropyl-phenyl)-7-methoxy-1-(2-methoxy-ethyl)-5-bromo-4-trifluoromethyl-1H-benzoimidazole
[2-(4-Isopropyl-phenyl)-7-methoxy-1-(2-methoxy-ethyl)-4-trifluoromethyl-1H-benzoimidazol-5-ylmethyl]-phenyl-amine
[2-(4-Isopropyl-phenyl)-7-methoxy-1-(2-methoxy-ethyl)-4-trifluoromethyl-1H-benzoimidazol-5-ylmethyl]-pyridin-2-yl-amine
2-{[2-(4-Isopropyl-phenyl)-7-methoxy-1-(2-methoxy-ethyl)-4-trifluoromethyl-1H-benzoimidazol-5-ylmethyl]-amino}-benzenesulfonamide
2-(4-Isopropyl-phenyl)-7-methoxy-1-(2-methoxy-ethyl)-5-phenoxymethyl-4-trifluoromethyl-1H-benzoimidazole
2-(4-Isopropyl-phenyl)-7-methoxy-1-(2-methoxy-ethyl)-5-(pyridin-2-yloxymethyl)-4-trifluoromethyl-1H-benzoimidazole
2-[2-(4-Isopropyl-phenyl)-7-methoxy-1-(2-methoxy-ethyl)-4-trifluoromethyl-1H-benzoimidazol-5-ylmethoxy]-benzenesulfonamide
4-Bromo-2-(4-isopropyl-phenyl)-7-methoxy-1-(2-methoxy-ethyl)-5-(pyridin-2-yloxy)-1H-benzoimidazole.

In an alternative embodiment of the invention, the calcilytic is compound of formula (I) or a pharmaceutically acceptable salt or prodrug ester thereof: 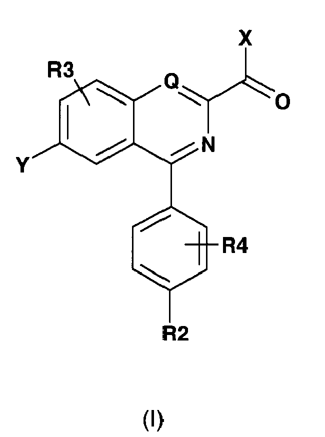 wherein:
Q is CH or N;
R2 is C₁-C₄ alkyl;
Y is selected from the group consisting of: R5-O-, C₁-C₄ alkyl, C₁-C₄ alkenyl, C₁-C₄ alkynyl, R5-NH-;
where R5 is C₁-C₄ alkyl, C₁-C₄ alkenyl, C₁-C₄ alkynyl;
X is selected from the group consisting of aryl, heteroaryl, C₁-C₁₀alkyl, C₁-C₁₀alkyloxy, cycloalkyl, heterocycloalkyl, aryl C₁-C₄ alkyl, heteroaryl C₁-C₄ alkyl, cycloalkyl C₁-C₄ alkyl, heterocycloalkyl C₁-C₄ alkyl, arylamino, heteroarylamino, aryl C₁-C₄ alkylamino, heteroaryl C₁-C₄ alkylamino, C₁-C₆ alkylamino, C₁-C₆ dialkylamino, aryloxy, heteroaryloxy, aryl C₁-C₄ alkyloxy, heteroaryl C₁-C₄ alkyloxy, cycloalkyl C₁-C₄ alkylamino, heterocycloalkyl C₁-C₄ alkylamino, cycloalkyl C₁-C₄ alkyloxy or heterocycloalkyl C₁-C₄ alkyloxy each of which is optionally substituted once or more;
the optional substituent or substituents on X being independently selected from the group consisting of halo, cyano, trifluoromethyl, nitro, hydroxy, optionally substituted (C₁-C₄ alkyl, C₁-C₄ alkyloxy, amino, sulfanyl, sulfonyl, amino, oxycarbonyl, hydroxyl, sulfinyl, aminosulfonyl, sulfonylamino, carbonyl, carbonyloxy, carbonyl amino, carboxyl, acyl, acylamino, or carbamoyl); the optional substituent or substituents being selected from C₁-C₆ alkyl, C₁-C₆ alkyloxy, carboxyl, hydroxyl, hydroxy C₁-C₄ alkyl; each of which in turn may be optionally substituted by C₁-C₆ alkyloxy, C₁-C₆alkyl, C₁-C₃ fluorinated alkyl, C₁-C₆ alkyloxy, carboxyl, hydroxyl, hydroxy C₁-C₄ alkyl, halo, cyano, nitro.
R3 and R4 each represent one or more substituents independently selected from: H, halo, C₁-C₄ alkyl, C₁-C₄alkyloxy, CF₃;
the optional substituent or substituents on R3 or R4 being independently selected from the group consisting of C₁-C₄alkyl, halo, C₁-C₄ alkyloxy, cyano, sulfanyl, sulfonyl, amino, oxycarbonyl, hydroxyl which may in turn be optionally substituted once or more by C₁-C₄ alkyl, halo, C₁-C₄ alkyloxy, cyano, sulfanyl, sulfonyl, amino, oxycarbonyl or hydroxyl.

In yet a further embodiment of the invention, the calcilytic is a compound of formula (II) or a pharmaceutically acceptable salt or prodrug ester thereof: 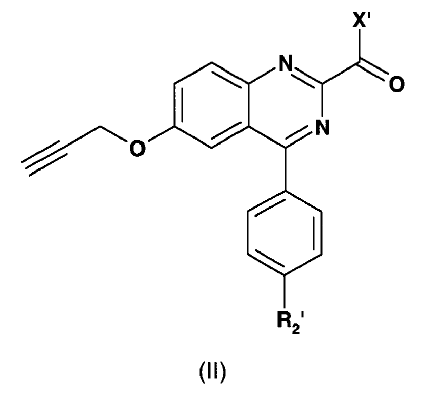 wherein:
X' is selected from the group consisting of aryl, heteroaryl, cycloalkyl, heterocycloalkyl, -C₁-C₄ alkylaryl, -C₁-C₄ alkylheteroaryl, arylamino, heteroarylamino, aryl C₁-C₄ alkylamino, heteroaryl C₁-C₄ alkylamino, aryloxy, heteroaryloxy, aryl C₁-C₄ alkyloxy, heteroaryl C₁-C₄ alkyloxy, aryl C₁-C₄ alkyl, heteroaryl C₁-C₄ alkyl, C₁-C₆alkyl, -C₁-C₄ alkylamino or amino, each of which is optionally substituted once or more;
the optional substituent or substituents on X' being independently selected from the group consisting of halo, cyano, trifluoromethyl, nitro, hydroxy, optionally substituted (C₁-C₄ alkyl, C₁-C₄ alkyloxy, amino, sulfanyl, sulfonyl, amino, oxycarbonyl, hydroxyl, sulfinyl, carbonyl, carboxyl, acyl, acylamino, carbamoyl or aminoacyl); the optional substituent or substituents being selected from C₁-C₆alkyl, C₁-C₆alkyloxy, carboxyl, hydroxyl, hydroxy C₁-C₄ alkyl; each of which in turn may be optionally substituted by C₁-C₆alkyloxy, C₁-C₆alkyl, C₁-C₆ alkyloxy, carboxyl, hydroxyl, hydroxy C₁-C₄ alkyl, halo, cyano, nitro.
R₂' is C₁-C₄ alkyl.

For example, the calcilytic may be a compound selected from the following:
(4-tert-Butyl-phenyl)-[4-(4-isopropyl-phenyl)-6-propargyloxy-quinazolin-2-yl]-methanone
[4-(4-Isopropyl-phenyl)-6-propargyloxy-quinazolin-2-yl]-phenyl-methanone
(2-Methoxy-phenyl)-[4-(4-Isopropyl-phenyl)-6-propargyloxy -quinazolin-2-yl]-methanone
(3-Methoxy-phenyl)-[4-(4-Isopropyl-phenyl)-6- propargyloxy -quinazolin-2-yl]-methanone
(4-Methoxy-phenyl)-[4-(4-Isopropyl-phenyl)-6-propargyloxy -quinazolin-2-yl]-methanone
(4-Fluoro-phenyl)-[4-(4-isopropyl-phenyl)-6-propargyloxy-quinazolin-2-yl]-methanone
(3-Fluoro-phenyl)-[4-(4-Isopropyl-phenyl)-6-propargyloxy-quinazolin-2-yl]-methanone
(3-Chloro-phenyl)-[4-(4-Isopropyl-phenyl)-6-propargyloxy-quinazolin-2-yl]-methanone
(4-Chloro-phenyl)-[4-(4-Isopropyl-phenyl)-6-propargyloxy-quinazolin-2-yl]-methanone
(4-Fluoro-phenyl)-[4-(4-tert.butyl-phenyl)-6-propargyloxy-quinazolin-2-yl]-methanone
(3-Fluoro-phenyl)-[4-(4-tert.butyl-phenyl)-6-propargyloxy-quinazolin-2-yl]-methanone
(3-Bromo-phenyl)-[4-(4-isopropyl-phenyl)-6-propargyloxy-quinazolin-2-yl]-methanone
(4-Bromo-phenyl)-[4-(4-isopropyl-phenyl)-6-propargyloxy-quinazolin-2-yl]-methanone
(4-Methyl-phenyl)-[4-(4-isopropyl-phenyl)-6-propargyloxy-quinazolin-2-yl]-methanone
(4-Isopropyl-phenyl)-[4-(4-isopropyl-phenyl)-6-propargyloxy-quinazolin-2-yl]-methanone
(4-Ethyl-phenyl)-[4-(4-isopropyl-phenyl)-6-propargyloxy-quinazolin-2-yl]-methanone
(4-Propyl-phenyl)-[4-(4-isopropyl-phenyl)-6-propargyloxy-quinazolin-2-yl]-methanone
(4-Cyano-phenyl)-[4-(4-isopropyl-phenyl)-6-propargyloxy-quinazolin-2-yl]-methanone
(4-Methylthio-phenyl)-[4-(4-isopropyl-phenyl)-6-propargyloxy-quinazolin-2-yl]-methanone
(4-Methansulfonyl-phenyl)-[4-(4-isopropyl-phenyl)-6-propargyloxy-quinazolin-2-yl]-methanone
(4-Dimethylamino-phenyl)-[4-(4-isopropyl-phenyl)-6-propargyloxy-quinazolin-2-yl]-methanone
(4-Ethoxy-phenyl)-[4-(4-isopropyl-phenyl)-6-propargyloxy-quinazolin-2-yl]-methanone
4-[4-(4-Isopropyl-phenyl)-6-propargyloxy-quinazoline-2-carbonyl]-benzoic acid methyl ester
(4-Dimethylamino-phenyl)-[4-(4-tert.butyl-phenyl)-6-propargyloxy-quinazolin-2-yl]-methanone
(4-Dimethylamino-phenyl)-[4-(4-cyclopropyl-phenyl)-6-propargyloxy-quinazolin-2-yl]-methanone
4-[4-(4-Isopropyl-phenyl)-6-propargyloxy-quinazoline-2-carbonyl]-benzoic acid ethyl ester
(4-Methoxy-phenyl)-[4-(4-tert.butyl-phenyl)-6-propargyloxy-quinazolin-2-yl]-methanone
(4-Ethoxy-phenyl)-[4-(4-cyclopropyl-phenyl)-6-propargyloxy-quinazolin-2-yl]-methanone
(3-Ethoxy-4-methoxy-phenyl)-[4-(4-isopropyl-phenyl)-6-propargyloxy-quinazolin-2-yl]-methanone
(4-tert.Butyloxy-phenyl)-[4-(4-isopropyl-phenyl)-6-propargyloxy-quinazolin-2-yl]-methanone
(4-Hydroxy)-[4-(4-isopropyl-phenyl)-6-propargyloxy-quinazolin-2-yl]-methanone
(4-Butyloxy)-[4-(4-isopropyl-phenyl)-6-propargyloxy-quinazolin-2-yl]-methanone
Furan-2-yl-[4-(4-isopropyl-phenyl)-6-propargyloxy-quinazolin-2-yl]-methanone
Furan-3-yl-[4-(4-tert.butyl-phenyl)-6-propargyloxy-quinazolin-2-yl]-methanone
Furan-3-yl-[4-(4-isopropyl-phenyl)-6-propargyloxy-quinazolin-2-yl]-methanone
Thiophen-2-yl-[4-(4-isopropyl-phenyl)-6-propargyloxy-quinazolin-2-yl]-methanone
(3-Methyl-thiophen-2-yl-[4-(4-isopropyl-phenyl)-6-propargyloxy-quinazolin-2-yl]-methanone
Benz[b]thiophen-2-yl-[4-(4-isopropyl-phenyl)-6-propargyloxy-quinazolin-2-yl]-methanone
Thiophen-3-yl-[4-(4-isopropyl-phenyl)-6-propargyloxy-quinazolin-2-yl]-methanone
(1-Methyl-1H-pyrrol)-2-yl-[4-(4-isopropyl-phenyl)-6-propargyloxy-quinazolin-2-yl]-methanone
4-(4-Isopropyl-phenyl)-6-propargyloxy-quinazoline-2-carboxylic acid ethyl ester
[4-(4-isopropyl-phenyl)-6-propargyloxy-quinazolin-2-yl]-pyridine-3-yl-methanone
[4-(4-Isopropyl-phenyl)-6-propargyloxy-quinazolin-2-yl]-naphthalen-1-yl-methanone
[4-(4-Isopropyl-phenyl)-6-propargyloxy-naphathalen-2-yl]-methanone
Benzothiazol-2-yl-[4-(4-isopropyl-phenyl)-6-propargyloxy-quinazolin-2-yl]-methanone
[4-(4-Isopropyl-phenyl)-6-propargyloxy-quinazolin-2-yl]-thiazol-5-yl-methanone
[4-(4-Isopropyl-phenyl)-6-propargyloxy-quinazolin-2-yl]-piperidin-1-yl-methanone
4-(4-Isopropyl-phenyl)-6-prop-2-ynyloxy-quinazoline-2-carboxylic acid (3-chloro-phenyl)-amide
4-(4-Isopropyl-phenyl)-6-prop-2-ynyloxy-quinazoline-2-carboxylic acid (3-methoxy-phenyl)-amide
4-(4-Isopropyl-phenyl)-6-prop-2-ynyloxy-3,4-dihydro-quinazoline-2-carboxylic acid (3-methylsulfanyl-phenyl)-amide
4-(4-Isopropyl-phenyl)-6-prop-2-ynyloxy-3,4-dihydro-quinazoline-2-carboxylic acid (3-methanesulfonyl-phenyl)-amide
4-(4-Isopropyl-phenyl)-6-prop-2-ynyloxy-quinazoline-2-carboxylic acid (3-trifluoromethylsulfanyl-phenyl)-amide
4-(4-Isopropyl-phenyl)-6-prop-2-ynyloxy-3,4-dihydro-quinazoline-2-carboxylic acid (3-sulfamoyl-phenyl)-amide
4-(4-Isopropyl-phenyl)-6-prop-2-ynyloxy-3,4-dihydro-quinazoline-2-carboxylic acid [3-(2-hydroxy-ethanesulfonyl)-phenyl]-amide
4-(4-Isopropyl-phenyl)-6-prop-2-ynyloxy-3,4-dihydro-quinazoline-2-carboxylic acid (5-ethanesulfonyl-2-hydroxy-phenyl)-amide
4-(4-Isopropyl-phenyl)-6-prop-2-ynyloxy-quinazoline-2-carboxylic acid (3-nitro-phenyl)-amide
4-(4-Isopropyl-phenyl)-6-prop-2-ynyloxy-quinazoline-2-carboxylic acid (3-cyano-phenyl)-amide
3-{[4-(4-Isopropyl-phenyl)-6-prop-2-ynyloxy-quinazoline-2-carbonyl]-amino}-benzoic acid methyl ester
3-{[4-(4-Isopropyl-phenyl)-6-prop-2-ynyloxy-quinazoline-2-carbonyl]-amino}-benzoic acid ethyl ester
3-{[4-(4-Isopropyl-phenyl)-6-prop-2-ynyloxy-quinazoline-2-carbonyl]-amino}-benzoic acid isopropyl ester
3-{[4-(4-Isopropyl-phenyl)-6-prop-2-ynyloxy-quinazoline-2-carbonyl]-amino}-benzoic acid tert-butyl ester
4-(4-Isopropyl-phenyl)-6-prop-2-ynyloxy-quinazoline-2-carboxylic acid (3-carbamoyl-phenyl)-amide
4-(4-Isopropyl-phenyl)-6-prop-2-ynyloxy-quinazoline-2-carboxylic acid (3-acetyl-phenyl)-amide
3-{[4-(4-Isopropyl-phenyl)-6-prop-2-ynyloxy-quinazoline-2-carbonyl]-amino}-5-methoxy-benzoic acid methyl ester
4-(4-Isopropyl-phenyl)-6-prop-2-ynyloxy-quinazoline-2-carboxylic acid (3-methylcarbamoyl-phenyl)-amide
4-(4-Isopropyl-phenyl)-6-prop-2-ynyloxy-quinazoline-2-carboxylic acid (3-tert-butylcarbamoyl-phenyl)-amide
4-(4-Isopropyl-phenyl)-6-prop-2-ynyloxy-quinazoline-2-carboxylic acid (3-dimethylcarbamoyl-5-trifluoromethyl-phenyl)-amide
3-{[4-(4-Isopropyl-phenyl)-6-prop-2-ynyloxy-quinazoline-2-carbonyl]-amino}-5-trifluoromethyl-benzoic acid methyl ester
3-{[4-(4-Isopropyl-phenyl)-6-prop-2-ynyloxy-quinazoline-2-carbonyl]-amino}-5-trifluoromethylbenzoic acid isopropyl ester
2-Fluoro-5-{[4-(4-isopropyl-phenyl)-6-prop-2-ynyloxy-quinazoline-2-carbonyl]-amino}-benzoic acid methyl ester
2-Fluoro-5-{[4-(4-isopropyl-phenyl)-6-prop-2-ynyloxy-quinazoline-2-carbonyl]-amino}-benzoic acid isopropyl ester
2-Chloro-5-{[4-(4-isopropyl-phenyl)-6-prop-2-ynyloxy-quinazoline-2-carbonyl]-amino}-benzoic acid methyl ester
2,5-Dichloro-3-{[4-(4-isopropyl-phenyl)-6-prop-2-ynyloxy-quinazoline-2-carbonyl]-amino}-benzoic acid methyl ester
2,5-Dichloro-3-{[4-(4-isopropyl-phenyl)-6-prop-2-ynyloxy-quinazoline-2-carbonyl]-amino}-benzoic acid isopropyl ester
4-(4-Isopropyl-phenyl)-6-prop-2-ynyloxy-quinazoline-2-carboxylic acid (3-cyano-5-fluorophenyl)-amide
4-(4-Isopropyl-phenyl)-6-prop-2-ynyloxy-quinazoline-2-carboxylic acid (3,4-dicyano-phenyl)-amide
4-(4-Isopropyl-phenyl)-6-prop-2-ynyloxy-quinazoline-2-carboxylic acid (4-cyano-3-trifluoromethyl-phenyl)-amide
4-(4-Isopropyl-phenyl)-6-prop-2-ynyloxy-quinazoline-2-carboxylic acid (3-trifluoromethylphenyl)-amide
4-(4-Isopropyl-phenyl)-6-prop-2-ynyloxy-quinazoline-2-carboxylic acid (4-acetylamino-3-trifluoromethyl-phenyl)-amide
4-(4-Isopropyl-phenyl)-6-prop-2-ynyloxy-quinazoline-2-carboxylic acid (3-methoxy-5-trifluoromethyl-phenyl)-amide
4-(4-Isopropyl-phenyl)-6-prop-2-ynyloxy-quinazoline-2-carboxylic acid (3,5-bis-trifluoromethyl-phenyl)-amide
4-(4-Isopropyl-phenyl)-6-prop-2-ynyloxy-quinazoline-2-carboxylic acid (3-fluoro-5-trifluoromethyl-phenyl)-amide
4-(4-Isopropyl-phenyl)-6-prop-2-ynyloxy-quinazoline-2-carboxylic acid (4-fluoro-3-trifluoromethyl-phenyl)-amide
3-{[4-(4-Isopropyl-phenyl)-6-prop-2-ynyloxy-quinazoline-2-carbonyl]-amino}-2-methyl-benzoic acid methyl ester
3-{[4-(4-Isopropyl-phenyl)-6-prop-2-ynyloxy-quinazoline-2-carbonyl]-amino}-4-methyl-benzoic acid methyl ester
3-{[4-(4-Isopropyl-phenyl)-6-prop-2-ynyloxy-quinazoline-2-carbonyl]-amino}-4-methoxybenzoic acid methyl ester
5-{[4-(4-Isopropyl-phenyl)-6-prop-2-ynyloxy-quinazoline-2-carbonyl]-amino}-isophthalic acid dimethyl ester
4-{[4-(4-Isopropyl-phenyl)-6-prop-2-ynyloxy-quinazoline-2-carbonyl]-amino}-phthalic acid dimethyl ester
4-(4-Isopropyl-phenyl)-6-prop-2-ynyloxy-quinazoline-2-carboxylic acid (3,5-dichloro-phenyl)-amide
4-(4-Isopropyl-phenyl)-6-prop-2-ynyloxy-quinazoline-2-carboxylic acid (3,4-dichloro-phenyl)-amide
4-(4-Isopropyl-phenyl)-6-prop-2-ynyloxy-quinazoline-2-carboxylic acid (3-chloro-4-fluorophenyl)-amide
4-(4-Isopropyl-phenyl)-6-prop-2-ynyloxy-quinazoline-2-carboxylic acid (4-chloro-3-trifluoromethyl-phenyl)-amide
5-{[4-(4-Isopropyl-phenyl)-6-prop-2-ynyloxy-quinazoline-2-carbonyl]-amino}-pyridine-2-carboxylic acid methyl ester
5-{[4-(4-Isopropyl-phenyl)-6-prop-2-ynyloxy-quinazoline-2-carbonyl]-amino}-nicotinic acid methyl ester
5-{[4-(4-Isopropyl-phenyl)-6-prop-2-ynyloxy-quinazoline-2-carbonyl]-amino}-nicotinic acid isopropyl ester
[4-(4-Isopropyl-phenyl)-6-prop-2-ynyloxy-quinazolin-2-yl]-pyrrol-1-yl-methanone
4-(4-Isopropyl-phenyl)-6-prop-2-ynyloxy-quinazoline-2-carboxylic acid (5-methyl-1H-pyrazol-3-yl)-amide
(2-{[4-(4-Isopropyl-phenyl)-6-prop-2-ynyloxy-quinazoline-2-carbonyl]-a mino}-thiazol-4-yl)-acetic acid ethyl ester
4-(4-Isopropyl-phenyl)-6-prop-2-ynyloxy-quinazoline-2-carboxylic acid naphthaien-1-ylamide
4-(4-Isopropyl-phenyl)-6-prop-2-ynyloxy-quinazoline-2-carboxylic acid isoquinolin-8-ylamide
4-(4-Isopropyl-phenyl)-6-prop-2-ynyloxy-quinazoline-2-carboxylic acid phthalazin-5-ylamide
4-(4-Isopropyl-phenyl)-6-prop-2-ynyloxy-quinazoline-2-carboxylic acid quinolin-5-ylamide
4-(4-Isopropyl-phenyl)-6-prop-2-ynyloxy-quinazoline-2-carboxylic acid quinolin-8-ylamide
4-(4-Isopropyl-phenyl)-6-prop-2-ynyloxy-quinazoline-2-carboxylic acid isoquinolin-4-ylamide
4-(4-Isopropyl-phenyl)-6-prop-2-ynyloxy-quinazoline-2-carboxylic acid (5-acetyl-quinolin-8-yl)-amide
4-(4-Isopropyl-phenyl)-6-prop-2-ynyloxy-quinazoline-2-carboxylic acid (3-bromo-6-methoxy-quinolin-8-yl)-amide
4-(4-Isopropyl-phenyl)-6-prop-2-ynyloxy-quinazoline-2-carboxylic acid quinolin-2-ylamide
4-(4-Isopropyl-phenyl)-6-prop-2-ynyloxy-quinazoline-2-carboxylic acid quinolin-6-ylamide
4-(4-Isopropyl-phenyl)-6-prop-2-ynyloxy-quinazoline-2-carboxylic acid (2-methyl-quinolin-6-yl)-amide
(6-{[4-(4-Isopropyl-phenyl)-6-prop-2-ynyloxy-quinazoline-2-carbonyl]-amino}-quinolin-8-yloxy)-acetic acid ethyl ester
4-(4-Isopropyl-phenyl)-6-prop-2-ynyloxy-quinazoline-2-carboxylic acid (1H-benzoimidazol-4-yl)-amide
4-(4-Isopropyl-phenyl)-6-prop-2-ynyloxy-quinazoline-2-carboxylic acid benzothiazol-2-ylamide
4-(4-Isopropyl-phenyl)-6-prop-2-ynyloxy-quinazoline-2-carboxylic acid (benzo[1,3]dioxol-5-ylmethyl)-amide
4-(4-Isopropyl-phenyl)-6-prop-2-ynyloxy-quinazoline-2-carboxylic acid (thiophen-2-ylmethyl)-amide
4-(4-Isopropyl-phenyl)-6-prop-2-ynyloxy-quinazoline-2-carboxylic acid 3-methoxy-phenyl ester
1-(4-Isopropyl-phenyl)-7-prop-2-ynyloxy-isoquinoline-3-carboxylic acid ethyl ester
1-(4-Isopropyl-phenyl)-7-prop-2-ynyloxy-isoquinoline-3-carboxylic acid 1,2-dimethyl-propyl ester
1-(4-Isopropyl-phenyl)-7-prop-2-ynyloxy-isoquinoline-3-carboxylic acid isobutyl ester
1-(4-Isopropyl-phenyl)-7-prop-2-ynyloxy-isoquinoline-3-carboxylic acid cyclopropylmethyl ester
1-(4-Isopropyl-phenyl)-7-prop-2-ynyloxy-isoquinoline-3-carboxylic acid benzyl ester
1-(4-Isopropyl-phenyl)-7-prop-2-ynyloxy-isoquinoline-3-carboxylic acid 2-methoxy-benzyl ester
1-(4-Isopropyl-phenyl)-7-prop-2-ynyloxy-isoquinoline-3-carboxylic acid 3-methoxy-benzyl ester
1-(4-Isopropyl-phenyl)-7-prop-2-ynyloxy-isoquinoline-3-carboxylic acid 4-methoxycarbonylbenzyl ester
1-(4-Isopropyl-phenyl)-7-prop-2-ynyloxy-isoquinoline-3-carboxylic acid phenethyl ester
1-(4-Isopropyl-phenyl)-7-prop-2-ynyloxy-isoquinoline-3-carboxylic acid 1-phenyl-ethyl ester
1-(4-Isopropyl-phenyl)-7-prop-2-ynyloxy-isoquinoline-3-carboxylic acid [3-(2-hydroxy-ethanesulfonyl)-phenyl]-amide
[1-(4-Isopropyl-phenyl)-7-prop-2-ynyloxy-isoquinolin-3-yl]-(3-methoxy-phenyl)-methanone
[1-(4-Isopropyl-phenyl)-7-prop-2-ynyloxy-isoquinolin-3-yl]-(4-methoxy-phenyl)-methanone.

In yet a further embodiment of the invention, the calcilytic is compound of formula (I) or a pharmaceutically acceptable salt or prodrug ester thereof: 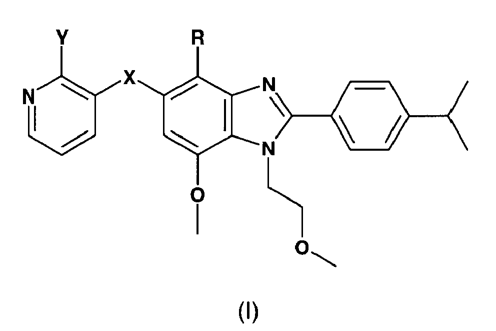 wherein
R is halo or optionally substituted C₁-C₆ alkyl;
X is selected from the group consisting of O, NH, CH₂, CO, SO, SO₂ or S;
Y represents a group selected from the following: optionally substituted C₁-C₆ alkyl, -SR₁,-S(O)R₁, -S(O)₂R₁, -OR₁, wherein R₁ is C₁-C₄alkyl;
the optional substituent or substituents on R and Y being independently selected from the group consisting of halogen, hydroxy, lower alkyl, mono or di-lower alkylamino, aminocarbonyl, sulfinyl, sulfonyl, sulfanyl, mono or di-lower alkylaminocarbonyl, amino, carboxy, lower alkoxy, C₃-C₁₂ cycloalkyl, C₃-C₁₈ heterocycloalkyl, lower alkylcarbonyl, lower alkoxycarbonyl, nitryl, aryl; all of which, except halogen, are independently optionally substituted by one or more substituents, selected from the group consisting of halogen, hydroxy, lower alkyl, mono or di-lower alkylamino, aminocarbonyl, sulfinyl, sulfonyl, sulfanyl, mono or di-lower alkylaminocarbonyl, amino, carboxy, lower alkoxy, C₃-C₁₂ cycloalkyl, C₃-C₁₈ heterocycloalkyl, lower alkylcarbonyl, lower alkoxycarbonyl, nitryl, aryl.

For example, the calcilytic maybe a compound selected from one of the following:
4-Bromo-2-(4-isopropyl-phenyl)-7-methoxy-1-(2-methoxy-ethyl)-5-(2-methylsulfanyl-pyridin-3-ylmethyl)-1H-benzoimidazole
2-(4-Isopropyl-phenyl)-7-methoxy-1-(2-methoxy-ethyl)-5-(2-methylsulfanyl-pyridin-3-ylmethyl)-4-trifluoromethyl-1H-benzoimidazole
4-Bromo-2-(4-isopropyl-phenyl)-5-(2-methanesulfinyl-pyridin-3-ylmethyl)-7-methoxy-1-(2-methoxy-ethyl)-1H-benzoimidazole
2-(4-Isopropyl-phenyl)-5-(2-methanesulfinyl-pyridin-3-ylmethyl)-7-methoxy-1-(2-methoxyethyl)-4-trifluoromethyl-1H-benzoimidazole
2-(4-Isopropyl-phenyl)-5-(2-methanesulfonyl-pyridin-3-ylmethyl)-7-methoxy-1-(2-methoxyethyl)-4-trifluoromethyl-1H-benzoimidazole
2-(4-Isopropyl-phenyl)-7-methoxy-1-(2-methoxy-ethyl)-5-(2-methoxy-pyridin-3-ylmethyl)-4-trifluoromethyl-1H-benzoimidazole.

The calcilytic may alternatively be any other calcilytic described in the literature, for example a compound described in one of the following patent applications: WO2006042007, WO2005030749, WO2002014259, WO2006041968, WO9737967.

The composition of the invention may be formulated as a spontaneously dispersible pharmaceutical composition.

"Spontaneously dispersible pharmaceutical composition" as used herein means a composition that contains an active agent herein defined and is capable of producing colloidal structures-when diluted with an aqueous medium, for example water, or in gastric juices. The colloidal structures are preferably liquid droplets in the microemulsion size range. Solid drug particles, either crystalline or amorphous may also be present. The spontaneously dispersible pharmaceutical composition is preferably a microemulsion preconcentrate.

"Microemulsion preconcentrate" as used herein means a composition which spontaneously forms a microemulsion in an aqueous medium, for example, in water, for example on dilution of 1:1 to 1:300, preferably 1:1 to 1:70, but especially 1:1 to 1:10 or in the gastric juices after oral application.

"Microemulsion" as used herein means a translucent, slightly opaque, opalescent, non-opaque or substantially non-opaque colloidal dispersion that is formed spontaneously or substantially spontaneously when its components are brought into contact with an aqueous medium. A microemulsion is thermodynamically stable and typically contains dispersed droplets.

Microemulsions offer greater ease of preparation due to spontaneous formation, thermodynamic stability, transparent and elegant appearance, increased drug loading, enhanced penetration through the biological membranes, increased bioavailability, and less inter- and intra-individual variability in drug pharmacokinetics than coarse emulsions.
Further characteristics of microemulsions can be found in United Kingdom patent specification GB 2,222,770; Rosof, Progress in Surface and Membrane Science, 12, 405 et seq. Academic Press (1975); Friberg, Dispersion Science and Technology, 6 (3), 317 et seq. (1985); and Muller et al. Pharm. Ind., 50 (3), 370 et seq. (1988)].

In another aspect, the present invention provides a pharmaceutical composition comprising an active agent administered to a subject resulting in a rapid and short release of parathyroid hormone into the plasma followed by a fast decrease of the parathyroid hormone levels,
wherein the pharmaceutical composition is in the form of a spontaneously dispersible composition.

In a further aspect, the present invention provides a spontaneously dispersible pharmaceutical composition comprising a calcilytic agent.

In a further aspect, the present invention provides a spontaneously dispersible pharmaceutical composition comprising a calcilytic active agent, and a carrier medium comprising a lipophilic component, a surfactant, a hydrophilic component and optionally a co-solvent.

The spontaneously dispersible pharmaceutical composition may be suitable for buccal, pulmonal, topical, rectal or vaginal administration, preferably for oral administration.

In another aspect, the present invention provides a pharmaceutical composition comprising an active agent administered to a subject resulting in a rapid and short last release of parathyroid hormone into the plasma followed by a fast decrease of the parathyroid hormone level to baseline levels, wherein the pharmaceutical composition is in the form of a microemulsion preconcentrate.

In a further aspect, the present invention provides a microemulsion preconcentrate comprising a calcilytic active agent and a carrier medium that comprises a lipophilic component, a surfactant, a hydrophilic component and optionally a co-solvent.

The microemulsion preconcentrate preferably forms an o/w (oil-in-water) microemulsion when diluted with water.

Preferably the relative proportions of the lipophilic component(s), the surfactant(s), the hydrophilic component(s), and optionally the co-solvent(s) lie within the "Microemulsion" region on a standard three way plot graph. These phase diagrams, can be generated in a conventional manner as described in e.g. GB 2,222,770 or WO 96/13273.

In another aspect, the present invention provides a pharmaceutical composition comprising an active agent administered to a subject resulting in a rapid and short last release of parathyroid hormone into the plasma followed by a fast decrease of the parathyroid hormone levels to baseline levels, wherein the pharmaceutical composition is in the form of a microemulsion.

In a further aspect, the present invention provides a microemulsion comprising a calcilytic active agent.

The microemulsion is preferably an o/w (oil-in-water) microemulsion.

In a further aspect, the present invention provides a microemulsion comprising a calcilytic active agent, a lipophilic component, a surfactant, water, a hydrophilic component and optionally a co-solvent

The colloidal structures of the microemulsion form spontaneously or substantially spontaneously when the components of the composition of the invention are brought into contact with an aqueous medium, e.g. by simple shaking by hand for a short period of time, for example for 10 seconds. The compositions of the invention are kinetically stable, e.g. for at least 15 minutes or up to 4 hours, even to 24 hours or longer.

In some embodiments of the compositions of the invention the carrier medium comprises a lipophilic component, a surfactant, and a hydrophilic component. In other embodiments the carrier medium comprises a lipophilic component, a surfactant, a hydrophilic component and a co-solvent.

The lipophilic component comprises one or more lipophilic substances. The hydrophilic component comprises one or more hydrophilic substances. The carrier medium can contain one or more surfactants. The carrier medium can contain one or more co-solvents.

The compositions of the invention may include a variety of additives including antioxidants, antimicrobial agents, enzyme inhibitors, stabilizers, preservatives, flavours, sweeteners and further components such as those described in Fiedler, H. P. "Lexikon der Hilfsstoffe für Pharmazie, Kosmetik und angrenzende Gebiete", Editio Cantor, D-7960 Aulendorf, 5th revised and expanded edition (2002). These additives will conveniently be dissolved in the carrier medium.

The compositions of the invention include a lipophilic component or phase. The active agent may be contained in this component of the carrier medium. The lipophilic component (when present) is preferably characterized by a low HLB value of less than 10, e.g. up to 8.

Suitable lipophilic components include:

### 1) Glyceryl mono-C₆-C₁₄-fatty acid esters

These are obtained esterifying glycerol with vegetable oil followed by molecular distillation. Monoglycerides suitable for use in the compositions of the invention include both symmetric (i.e. β-monoglycerides) as well as asymmetric monoglycerides (α-monoglycerides. They also include both uniform glycerides (in which the fatty acid constituent is composed primarily of a single fatty acid) as well as mixed glycerides (i.e. in which the fatty acid constituent is composed of various fatty acids) The fatty acid constituent may include both saturated and unsaturated fatty acids having a chain length of from e.g. C₈-C₁₄. Particularly suitable are caprylic or lauric acid monoglycerides which are commercially available, e.g. under the trade names Imwitor® 308 or Imwitor® 312, respectively, from e.g. sasol. For example Imwitor® 308 comprises at least 80 % monoglycerides and exhibits the following additional characterising data: free glycerol max 6 %, acid value max. 3, saponification value 245-265, iodine value max. 1, water content max. 1 %. Typically it comprises 1 % free glycerol, 90 % monoglycerides, 7 % diglycerides, 1 % triglycerides (H. Fiedler, *loc. cit.,* volume 1, page 906). A further example is Capmul MCM C8 from Abitec Corporation.

### 2) Mixtures of mono- and di-glycerides of C₆-C₁₈ fatty acids

These include both symmetric (i.e. β-monoglycerides and α,α¹-diglycerides) as well as asymmetric mono- and di-glycerides (i.e. α-monoglycerides and α,β-diglycerides) and acetylated derivatives thereof. They also include both uniform glycerides (in which the fatty acid constituent is composed primarily of a single fatty acid) as well as mixed glycerides (i.e. in which the fatty acid constituent is composed of various fatty acids) and any derivatives thereof with lactic or citric acid. The fatty acid constituent may include both saturated and unsaturated fatty acids having a chain length of from e.g. C₈-C₁₀. Particularly suitable are mixed caprylic and capric acid mono- and di-glycerides as commercially available, e.g. under the trade name Imwitor® 742 or lmwitor 928, from e.g. Sasol. For example Imwitor® 742 comprises at least 45% monoglycerides and exhibits the following additional characterising data: free glycerol max. 2 %, acid value max. 2, saponification value 250-280, iodine value max. 1, water max. 2 % (H. Fiedler, *loc. cit.,* vol 1, page 906). Other suitable mixtures comprise mono/diglycerides of caprylic/capric acid in glycerol as known and commercially available under e.g. the trade name Capmul® MCM from e.g. Abitec Corporation. Capmul® MCM exhibits the following additional characterising data: acid value 2.5 max., alpha-Mono (as oleate) 80% min., free glycerol 2.5% max., iodine value 1 max., chain length distribution: caproic acid (C6) 3% max., caprylic acid (C8) 75% min., capric acid (C10) 10% min., lauric acid (C12) 1.5% max., moisture (by Karl Fisher) 0.5% max. (manufacturer information). Suitable examples of mono-/di-glcyerides with additional derivatization with lactic or citric acid are those marketed under the brand names of lmwitor 375, 377 or 380 by sasol. Furthermore, the fatty acid constituent may include both saturated and unsaturated fatty acids having a chain length of from e.g. C₁₆-C₁₈. A suitable example is Tegin® O (glyceryl oleate) exhibiting the following additional characterising data: monoglyceride content 55-65%, peroxide value max. 10, water content max. 1%, acid value max. 2, iodine value 70-76, saponification value 158-175, free glycerol max. 2%, (manufacturer information).

### 3) Glyceryl di- C₆-C₁₈-fatty acid esters

These include symmetric (i.e. α,α¹-diglycerides) and asymmetric diglycerides (i.e. α,β-diglycerides) and acetylated derivatives thereof. They also include both uniform glycerides (in which the fatty acid constituent is composed primarily of a single fatty acid) as well as mixed glycerides (i.e. in which the fatty acid constituent is composed of various fatty acids) and any acetylated derivatives thereof. The fatty acid constituent can include both saturated and unsaturated fatty acids having a chain length of from C₆-C₁₈, e.g. C₆-C₁₆, e.g. C₈-C₁₀, e.g. C₈. Particularly suitable is caprylic diglycerides, which is commercially available, e.g. under the trade name Sunfat® GDC-S, e.g. from Taiyo Kagaku Co., Ltd. Sunfat® GDC-S has an acid value of about 0.3, a diglyceride content of about 78.8%, and a monoester content of about 8.9.

### 4) Medium chain fatty acid triglyceride

These include triglycerides of saturated fatty acid having 6 to 12, e.g. 8 to 10, carbon atoms. Suitable medium chain fatty acid triglycerides are those known and commercially available under the trade names Acomed®, Myritol®, Captex®, Neobee®M 5 F, Miglyol®810, Miglyol®812, Miglyol®818, Mazol®, Sefsol®860, Sefsol®870; Miglyol®812 being the most preferred. Miglyol®812 is a fractionated coconut oil comprising caprylic-capric acid triglycerides and having a molecular weight of about 520 Daltons. Fatty acid composition = C₆ max. about 3%, C₈ about 50 to 65%, C₁₀ about 30 to 45%, C₁₂ max 5%; acid value about 0.1; saponification value about 330 to 345; iodine value max 1. Miglyol® 812 is available from Condea. Neobee® M 5 F is a fractionated caprylic-capric acid triglyceride available from coconut oil; acid value max. 0.2; saponification value about 335 to 360; iodine value max 0.5, water content max. 0,15%, D.²⁰ 0,930-0,960, n_{D}²⁰ 1,448-1,451 (manufacturer information). Neobee® M 5 F is available from Stepan Europe. A further example is Miglyol 829 containing additionally esters with succinic acid.

### 5) Glyceryl mono-C₁₆-C₁₈-fatty acid esters

These are obtained esterifying glycerol with vegetable oil followed by molecular distillation. Monoglycerides suitable for use in the compositions of the invention include both symmetric (i.e. β-monoglycerides) as well as asymmetric monoglycerides (α-monoglycerides. They also include both uniform glycerides (in which the fatty acid constituent is composed primarily of a single fatty acid) as well as mixed glycerides (i.e. in which the fatty acid constituent is composed of various fatty acids). The fatty acid constituent may include both saturated and unsaturated fatty acids having a chain length of from e.g. C₁₆-C₁₈. Suitable examples include GMOrphic by Eastman, Rylo MG20 distilled monoglyceride by Danisco Ingredients, or Monomuls 90-018 by Henkel. For example GMOrphic®-80 (glyceryl monooleate) exhibits the following additional characterising data: monoglyceride content min. 94%, C18:1 content 75% min., peroxide value max. 2.5, C18:2 + C18:3 max. 15%, C16:0 + C18:0 + C20:0 max. 10%, water max. 2%, acid value max. 3, iodine value 65-75, saponification value 155-165, free glycerine max. 1%, hydroxyl number 300-330 (manufacturer information).

### 6) Mixed mono-, di-, tri-glycerides

These include mixed mono-, di-, tri-glycerides that are commercially available under the trade name Maisine® from Gattefossé. They are transesterification products of corn oil and glycerol. Such products are comprised predominantly of linoleic and oleic acid mono-, di- and tri-glycerides together with minor amounts of palmitic and stearic acid mono-, di- and tri-glycerides (corn oil itself being comprised of ca. 56% by weight linoleic acid, 30% oleic acid, ca. 10% palmitic and ca. 3% stearic acid constituents). Physical characteristics are: free glycerol max 10%, monoglycerides ca. 40%, diglycerides ca. 40%, triglycerides ca. 10%, free oleic acid content ca. 1%. Further physical characteristics are: acid value max. 2, iodine value of 85-105, saponification value of 150-175, mineral acid content = 0. The fatty acid content for Maisine® is typically: palmitic acid ca. 11 %, stearic acid ca. 2.5%, oleic acid ca. 29%, linoleic acid ca. 56%, others ca. 1.5% (H. Fiedler, *loc. cit.,* volume 2, page 1079; manufacturer information).

Mixed mono-, di-, tri-glycerides preferably comprise mixtures of C₈ to C₁₀ or C₁₂₋₂₀ fatty acid mono-, di- and tri-glycerides, especially mixed C₁₆₋₁₈ fatty acid mono-, di- and triglycerides. The fatty acid component of the mixed mono-, di- and tri-glycerides may comprise both saturated and unsaturated fatty acid residues. Preferably however they are predominantly comprised of unsaturated fatty acid residues; in particular C₁₈ unsaturated fatty acid residues. Suitably the mixed mono-, di-, tri-glycerides comprise at least 60%, preferably at least 75%, more preferably at least 85% by weight of a C₁₈ unsaturated fatty acid (for example linolenic, linoleic and oleic acid) mono-, di- and tri-glycerides. Suitably the mixed mono-, di-, tri-glycerides comprise less than 20%, for example about 15% or 10% by weight or less, saturated fatty acid (for example palmitic and stearic acid) mono-, di- and tri-glycerides. Mixed mono-, di-, tri-glycerides are preferably predominantly comprised of mono- and di-glycerides; for example mono- and di-glycerides comprise at least 50%, more preferably at least 70% based on the total weight of the lipophilic phase or component. More preferably, the mono- and di-glycerides comprise at least 75% (for example about 80% or 85% by weight of the lipophilic component. Preferably monoglycerides comprise from about 25 to about 50%, based on the total weight of the lipophilic component, of the mixed mono-, di-, tri-glycerides. More preferably from about 30 to about 40% (for example 35 to 40%) monoglycerides are present. Preferably diglycerides comprise from about 30 to about 60%, based on the total weight of the lipophilic component, of the mixed mono-, di-, tri-glycerides. More preferably from about 40 to about 55% (for example 48 to 50%) diglycerides are present. Triglycerides suitably comprise at least 5% but less than about 25 %, based on the total weight of the lipophilic component, of the mixed mono-, di-, tri-glycerides. More preferably from about 7.5 to about 15% (for example from about 9 to 12%) triglycerides are present. Mixed mono-, di-, tri-glycerides may be prepared by admixture of individual mono-, di- or tri-glycerides in appropriate relative proportion. Conveniently however they comprise trans-esterification products of vegetable oils, for example almond oil, ground nut oil, olive oil, peach oil, palm oil or, preferably, corn oil, sunflower oil or safflower oil and most preferably corn oil, with glycerol. Such transesterification products are generally obtained as described in GB 2 257 359 or WO 94/09211. Preferably some of the glycerol is first removed to give a "substantially glycerol free batch" when soft gelatine capsules are to be made. Purified transesterification products of corn oil and glycerol provide particularly suitable mixed mono-, di-, and tri-glycerides hereinafter referred to as "refined oil" and produced according to procedures described in United Kingdom patent specification GB 2,257,359 or international patent publication WO 94/09211.

### 7) Acetylated monoalvcerides (C18)

These include Myvacet 9-45.

### 8) Propylene glycol monofatty acid esters

The fatty acid constituent may include both saturated and unsaturated fatty acids having a chain length of from e.g. C₈-C₁₂. Particularly suitable are propylene glycol mono ester of caprylic and lauric acid as commercially available, e.g. under the trade names Sefsol® 218, Capryol®90 or Lauroglycol®90, from e.g. Nikko Chemicals Co., Ltd. or Gattefossé or Capmul PG-8 from Abitec Corporation. For example Lauroglycol®90 exhibits the following additional characterising data: acid value max. 8, saponification value 200-220, iodine value max. 5, free propylene glycol content max. 5%, monoester content min. 90% (H. Fiedler, *loc. cit.,* vol 2, page 1025, manufacturer information); Sefsol® 218 exhibits the following additional characterising data: acid value max. 5, hydroxy value 220-280.

### 9) Propylene glycol mono- and di- fatty acid esters

These include Laroglycol FCC and Capryol PGMC.

### 10) Propylene glycol diesters

Propylene glycol di-fatty acid esters such as propylene glycol dicaprylate (which is commercially available under the trade name Miglyol® 840 from e.g. sasol; H. Fiedler, *loc. cit.,* volume 2, page 1130) or Captex 200 from Abitec Corporation.

### 11) Propylene glycol monoacetate and propylene glycol diacetate

### 12) Transesterified ethoxylated vegetable oils

These include transesterified ethoxylated vegetable oils such as those obtained by reacting various natural vegetable oils (for example, corn oil, maize oil, castor oil, kernel oil, almond oil, ground nut oil, olive oil, soybean oil, sunflower oil, safflower oil and palm oil, or mixtures thereof) with polyethylene glycols that have an average molecular weight of from 200 to 800, in the presence of an appropriate catalyst. These procedures are described in United States patent specification US 3,288,824. Transesterified ethoxylated corn oil is particularly preferred.

Transesterified ethoxylated vegetable oils are known and are commercially available under the trade name Labrafil® (H. Fiedler, loc. cit., vol 2, page 994). Examples are Labrafil® M 2125 CS (obtained from corn oil and having an acid value of less than about 2, a saponification value of 155 to 175, an HLB value of 3 to 4, and an iodine value of 90 to 110), and Labrafil® M 1944 CS (obtained from kernel oil and having an acid value of about 2, a saponification value of 145 to 175 and an iodine value of 60 to 90). Labrafil® M 2130 CS (which is a transesterification product of a C₁₂₋₁₈ glyceride and polyethylene glycol and which has a melting point of about 35 to 40°C, an acid value of less than about 2, a saponification value of 185 to 200 and an iodine value of less than about 3) may also be used. The preferred transesterified ethoxylated vegetable oil is Labrafil® M 2125 CS which can be obtained, for example, from Gattefossé, Saint-Priest Cedex, France.

### 13) Sorbitan fatty acid esters

Such esters include e.g. sorbitan mono C₁₂₋₁₈ fatty acid esters, or sorbitan tri C₁₂₋₁₈ fatty acid esters are commercially available under the trade mark Span® from e.g. uniqema. An especially preferred product of this class is e.g. Span® 20 (sorbitan monolaurate) or Span® 80 (sorbitan monooleate) (Fiedler, *loc. cit.,* 2, p. 1571; Handbook of Pharmaceutical Excipients, , *loc. cit.,* page 511).

### 14) Esterified compounds of fatty acid and primary alcohols

These include esterified compounds of fatty acid having 8 to 20 carbon atoms and primary alcohol having 2 to 3 carbon atoms, for example, isopropyl myristate, isopropyl palmitate, ethyl linoleate, ethyl oleate, ethylmyristate etc., with an esterified compound of linoleic acid and ethanol being particularly preferable, also isopropylmyristat and isopropylpalmitat.

### 15) Glycerol triacetate or (1,2,3)-triacetin

This is obtained by esterifying glycerin with acetic anhydride. Glycerol triacetate is commercially available as, e.g. Priacetin® 1580 from Unichema International, or as Eastman™ Triacetin from Eastman, or from Courtaulds Chemicals Ltd. Glycerol triacetate exhibits the following additional characterising data: molecular weight 218,03, D.^{20,3} 1,159-1,163, n_{D}²⁰ 1,430-1,434, water content max. 0.2 %, viscosity (25°) 17.4 mPa s, acid value max. 0.1, saponification value of about 766-774, triacetin content 97% min. (H. Fiedler, loc. cit., vol 2, page 1720; Handbook of Pharmaceutical Excipients, *loc. cit.,* page 534, manufacturer information).

### 16) Acetyl triethyl citrate

This is obtained by esterification of citric acid and ethanol, followed by acetylation with acetic anhydride, respectively. Acetyl triethyl citrate is commercially available, e.g. under the trade name Citroflex® A-2, from e.g. Morflex Inc.

### 17) Tributylcitrate or acetyl tributyl citrate

### 18) Polyglycerol fatty acid esters

These have for example from 2 to 10, e.g. 6 glycerol units. The fatty acid constituent can include both saturated and unsaturated fatty acids having a chain length of from e.g. C₈-C₁₈. Particularly suitable is e.g. Plurol Oleique CC497 from Gattefossé, having a saponification value of 133-155 and a saponification value of 196-244. Further suitable polyglycerol fatty acid esters include diglyceryl monooleate (DGMO) and Hexaglyn-5-O, as known and commercially available from e.g. Nikko Chemicals Co., Ltd.

### 19) PEG-fatty alcohol ether

This includes Brij 30™ polyoxyethylene(4) lauryl ether.

### 20) Fatty alcohols and fatty acids

Fatty acids can be obtained by hydrolysing various animal and vegetable fats or oils, such as olive oil, followed by separation of the liquid acids. The fatty acid/alcohol constituent can include both saturated and mono- or di-unsaturated fatty acids/alcohols having a chain length of from e.g. C₆-C₂₀. Particularly suitable are, e.g. oleic acid, oleyl alcohol, linoleic acid, capric acid, caprylic acid, caproic acid, tetradecanol, dodecanol, or decanol. Oleyl alcohol is commercially available under the trade mark HD-Eutanol® V from e.g. Henkel KGaA. Oleyl alcohol exhibits the following additional characterising data: acid value max 0.1, hydroxy value of about 210, iodine value of about 95, saponification value max 1, D.²⁰ about 0,849, n_{D}²⁰ 1,462, molecular weight 268, viscosity (20°) about 35 mPa s (manufacturer information). Oleic acid exhibits the following additional characterising data: molecular weight 282,47, D.²⁰ 0,895, n_{D}²⁰ 1,45823, acid value 195-202, iodine value 85-95, viscosity (25°) 26 mPa s (H. Fiedler, *loc. cit.,* volume 2, page 1236; "Handbook of Pharmaceutical Excipients", 2nd Edition, Editors A. Wade and P. J. Weller (1994), Joint publication of American Pharmaceutical Assoc., Washington, USA and The Pharmaceutical Press, London, England, page 325).

### 21) Tocopherol and its derivatives (e.g. acetate)

These include Coviox T-70, Copherol 1250, Copherol F-1300, Covitol 1360 and Covitol 1100.

### 22) Pharmaceutically acceptable oils

Alternatively the lipophilic component comprises e.g. a pharmaceutically acceptable oil, preferably with an unsaturated component such as a vegetable oil.

### 23) Alkylene polyol ethers or esters

These include C₃₋₅alkylene triols, in particular glycerol, ethers or esters. Suitable C₃₋₅alkylene triol ethers or esters include mixed ethers or esters, i.e. components including other ether or ester ingredients, for example transesterification products of C₃₋₅alkylene triol esters with other mono-, di- or poly-ols. Particularly suitable alkylene polyol ethers or esters are mixed C₃₋₅alkylene triol/poly-(C₂₋₄alkylene) glycol fatty acid esters, especially mixed glycerol/polyethylene- or polypropylene-glycol fatty acid esters.

Especially suitable alkylene polyol ethers or esters include products obtainable by transesterification of glycerides, e.g. triglycerides, with poly-(C₂₋₄alkylene) glycols, e.g. poly-ethylene glycols and, optionally, glycerol. Such transesterification products are generally obtained by alcoholysis of glycerides, e.g. triglycerides, in the presence of a poly-(C-₂₋₄alkylene) glycol, e.g. polyethylene glycol and, optionally, glycerol (i.e. to effect transesterification from the glyceride to the poly-alkylene glycol/glycerol component, i.e. via poly-alkylene glycolysis/glycerolysis).

In general such reaction is effected by reacting the indicated components (glyceride, polyalkylene glycol and, optionally, glycerol) at elevated temperature under an inert atmosphere with continuous agitation.

Preferred glycerides are fatty acid triglycerides, e.g. (C₁₀₋₂₂fatty acid) triglycerides, including natural and hydrogenated oils, in particular vegetable oils. Suitable vegetable oils include, for example, olive, almond, peanut, coconut, palm, soybean and wheat germ oils and, in particular, natural or hydrogenated oils rich in (C₁₂₋₁₈fatty acid) ester residues. Preferred polyalkylene glycol materials are polyethylene glycols, in particular polyethylene glycols having a molecular weight of from ca. 500 to ca. 4,000, e.g. from ca. 1,000 to ca. 2,000.

Suitable alkylene polyol ethers or esters include mixtures of C₃₋₅alkylene triol esters, e.g. mono-, di- and tri-esters in variable relative amount, and poly (C₂₋₄alkylene) glycol mono- and di-esters, together with minor amounts of free C₃₋₅alkylene triol and free poly-(C₂₋₅alkylene) glycol. As hereinabove set forth, the preferred alkylene triol moiety is glyceryl; preferred polyalkylene glycol moieties include polyethylene glycol, in particular having a molecular weight of from ca. 500 to ca. 4,000; and preferred fatty acid moieties will be C₁₀₋₂₂fatty acid ester residues, in particular saturated C₁₀₋₂₂fatty acid ester residues.

Particularly suitable alkylene polyol ethers or esters include transesterification products of a natural or hydrogenated vegetable oil and a polyethylene glycol and, optionally, glycerol; or compositions comprising or consisting of glyceryl mono-, di- and tri-C₁₀-₂₂fatty acid esters and polyethylene glycol mono- and di- C₁₀₋₂₂ fatty esters (optionally together with, e.g. minor amounts of free glycerol and free polyethylene glycol).

Preferred vegetable oils, polyethylene glycols or polyethylene glycol moieties and fatty acid moieties in relation to the above definitions are as hereinbefore set forth.

Particularly suitable alkylene polyol ethers or esters as described above for use in the present invention include those commercially available under the trade name Gelucire® from e.g. Gattefossé, in particular the products:
a) Gelucire® 33/01, which has an m.p. = ca. 33-37°C and a saponification value of ca. 230-255;
b) Gelucire® 39/01, m.p. = ca. 37.5-41.5°C, saponification v. = ca. 225-245;
c) Gelucire® 43/01, m.p. = ca. 42-46°C, saponification v. = ca. 220-240;

Products (a) to (c) above all have an acid value of maximum of 3. The compositions of the invention may include mixtures of such ethers or esters.

### 24) Hydrocarbons

These include e.g. squalene, available from e.g. Nikko Chemicals Co., Ltd.

### 25) Ethylene glycol esters

These include Monthyle® (ethylene glycol monostearate), available from e.g. Gattefossé.

### 26) Pentaerythriol fatty acid esters and polyalkylene glycol ethers

These include, for example pentaerythrite-dioleate, -distearate, -monolaurate, - polyglycol ether, and -monostearate as well as pentaerythrite-fatty acid esters (Fiedler, *loc. cit.,* 2, p. 1288-1290, incorporated herein by reference).

Some of these, e.g. (1-3, 5-6, 8-9, 12-13, 19), display surfactant-like behaviour and may also be termed co-surfactants.

The lipophilic component preferably comprises 5 to 85 % by weight of the composition of the invention, e.g. 10 to 85%; preferably 15 to 60 % by weight, more preferably about 15 to about 40 % by weight.

Where the carrier medium comprises a hydrophilic component in addition to the lipophilic component and the surfactant the relative proportions of the lipophilic component(s), hydrophilic component(s) and the surfactant(s) lie within the "Microemulsion" region on a standard three way plot graph.

The compositions of the invention include a hydrophilic component or phase.

Suitable hydrophilic compounds include:

### 1) Polyethylene glycol glyceryl C₆-C₁₀ fatty acid esters

The fatty acid ester may include mono and/or di and/or tri fatty acid esters. It optionally includes both saturated and unsaturated fatty acids having a chain length of from e.g. C₈-C₁₀. The polyethylene glycols may have e.g. from 5 to 10 [CH₂-CH₂-O] units, e.g. 7 units. A particularly suitable fatty acid ester is polyethylene glycol (7) glyceryl monococoate, which is commercially available, e.g. under the trade name Cetiol® HE, e.g. from Henkel KGaA. Cetiol® HE has a D. (20°) of 1,05, an acid value of less than 5, a saponification value of about 95, a hydroxyl value of about 180 and an iodine value of less than 5 (H. Fiedler, *loc. cit.,* vol 1, page 410) or Lipestrol E-810.

### 2) N-alkylpyrrolidone

Particularly suitable is, e.g. N-Methyl-2-pyrrolidone, e.g. as commercially available under the trade name Pharmasolve™, from e.g. International Specialty Products (ISP). N-methylpyrrolidone exhibits the following additional characterising data: molecular weight 99,1, D.²⁵ 1,027-1,028, purity (as area % by GC) (including Methyl Isomers) 99.85% min (H. Fiedler, *loc. cit.,* vol 2, page 1303, manufacturer information).

### 3) Benzyl alcohol

This is commercially available from e.g. Merck or may be obtained by distillation of benzyl chloride with potassium or sodium carbonate. Benzyl alcohol exhibits the following additional characterising data: molecular weight 108,14, D. 1,043-1,049, n_{D} 1,538-1,541. (H. Fiedler, *loc. cit.,* vol 1, page 301; Handbook of Pharmaceutical Excipients, 3^{rd} edition *loc. cit.,* page 41).

### 4) Triethyl citrate

It is obtained esterifying citric acid and ethanol. Triethyl citrate is commercially available, e.g. under the trade names Citroflex® 2, or in a pharmaceutical grade under the name TEC-PG/N, from e.g. Morflex Inc. Particularly suitable is triethyl citrate which has molecular weight of 276,3, a specific gravity of 1,135-1,139, a refractive index of 1,439-1,441, a viscosity (25°) of 35,2 mPa s, assay (anhydrous basis) 99,0-100,5 %, water max. 0,25 % (Fiedler, H. P., *loc. cit., ,* vol 1, page 446; "Handbook of Pharmaceutical Excipients", *loc. cit.,* page 573).

### 5) Polyethylene glycols e.g. Polyethylene glycol 400 (PEG400), polyethylene glycol 300 (PEG300).

Other suitable hydrophilic compounds include transcutol (C₂H₅-[O-(CH₂)₂]₂-OH), glycofurol (also known as tetrahydrofurfuryl alcohol polyethylene glycol ether), 1,2-propylene glycol, dimethylisosorbide (Arlasolve), polyethylene glycol, triethylenglycol, ethylacetate, and ethyllactate.

The hydrophilic component may comprise 5 to 60 % by weight of the composition of the invention, e.g. 10 to 50%; preferably 10 to 40 % by weight, more preferably about 10 to about 30% by weight.

The hydrophilic component may comprise a mixture of two or more hydrophilic components. The ratio of main hydrophilic component to hydrophilic co-component is typically from about 0.5:1 to about 2:1.

The compositions of the present invention preferably contain one or more surfactants to reduce the interfacial tension thereby providing thermodynamic stability.

Surfactants may be complex mixtures containing side products or unreacted starting products involved in the preparation thereof, e.g. surfactants made by polyoxyethylation may contain another side product, e.g. polyethylene glycol. The or each surfactant preferably has a hydrophilic-lipophilic balance (HLB) value of 8 to 17, especially 10 to 17. The HLB value is preferably the mean HLB value.

Suitable surfactants include:

### 1) Reaction products of a natural or hydrogenated castor oil and ethylene oxide

The natural or hydrogenated castor oil may be reacted with ethylene oxide in a molar ratio of from about 1:35 to about 1:60, with optional removal of the polyethylene-glycol component from the products. Various such surfactants are commercially available. Particularly suitable surfactants include polyethyleneglycol-hydrogenated castor oils available under the trade name Cremophor®; Cremophor® RH 40, which has a saponification value of about 50 to 60, an acid value less than about 1, a water content (Fischer) less than about 2%, an n_{D}⁶⁰ of about 1.453-1.457 and an HLB of about 14-16; and Cremophor® RH 60, which has a saponification value of about 40-50, an acid value less than about 1, an iodine value of less than about 1, a water content (Fischer) of about 4.5-5.5%, an n_{D}⁶⁰ of about 1.453-1.457 and an HLB of about 15 to 17.

An especially preferred product of this class is Cremophor® RH40. Other useful products of this class are available under the trade names Nikkol® (e.g. Nikkol® HCO-40 and HCO-60), Mapeg® (e.g. Mapeg® CO-40h), Incrocas® (e.g. Incrocas® 40), Tagat® (for example polyoxyethylene-glycerol-fatty acid esters e.g. Tagat® RH 40) and Simulsol OL-50 (PEG-40 castor oil, which has a saponification value of about 55 to 65, an acid value of max. 2, an iodine value of 25 to 35, a water content of max. 8%, and an HLB of about 13, available from Seppic). These surfactants are further described in Fiedler *loc. cit.*

Other suitable surfactants of this class include polyethyleneglycol castor oils such as that available under the trade name Cremophor® EL, which has a molecular weight (by steam osmometry) of about 1630, a saponification value of about 65 to 70, an acid value of about 2, an iodine value of about 28 to 32 and an n_{D}²⁵ of about 1.471.

### 2) Polyoxyethylene-sorbitan-fatty acid esters

These include mono- and tri-lauryl, palmityl, stearyl and oleyl esters of the type known and commercially available under the trade name Tween® (Fiedler, *loc. cit.* p.1754 ff) from Uniqema including the products:
Tween® 20 [polyoxyethylene(20)sorbitanmonolaurate],
Tween® 21 [polyoxyethylene(4)sorbitanmonolaurate],
Tween® 40 [polyoxyethylene(20)sorbitanmonopalmitate],
Tween® 60 [polyoxyethylene(20)sorbitanmonostearate],
Tween® 65 [polyoxyethylene(20)sorbitantristearate],
Tween® 80 [polyoxyethylene(20)sorbitanmonooleate],
Tween® 81 [polyoxyethylene(5)sorbitanmonooleate], and
Tween® 85 [polyoxyethylene(20)sorbitantrioleate].
Especially preferred products of this class are Tween® 20 and Tween® 80.

### 3) Polyoxyethylene fatty acid esters

These include polyoxyethylene stearic acid esters of the type known and commercially available under the trade name Myrj® from Uniqema (Fiedler, *loc. cit.,* 2, p. 1166). An especially preferred product of this class is Myrj® 52 having a D²⁵ of about 1.1., a melting point of about 40 to 44°C, an HLB value of about 16.9., an acid value of about 0 to 1 and a saponification no. of about 25 to 35.

### 4) Polyoxyethylene-polyoxypropylene co-polymers and block co-polymers or poloxamers

These include the type known and commercially available under the trade names Pluronic® and Emkalyx® (Fiedler, *loc. cit.,* 2, p. 1329). An especially preferred product of this class is Pluronic® F68 (poloxamer 188) from BASF, having a melting point of about 52°C and a molecular weight of about 6800 to 8975. A further preferred product of this class is Synperonic® PE L44 (poloxamer 124) from Uniqema.

### 5) Polyoxyethylene mono esters of a saturated C₁₀ to C₂₂

These include C₁₈ substituted e.g. hydroxy fatty acid; e.g. 12 hydroxy stearic acid PEG ester, e.g. of PEG about e.g. 600-900 e.g. 660 Daltons MW, e.g. Solutol® HS 15 from BASF, Ludwigshafen, Germany. According to the BASF technical leaflet MEF 151 E (1986) comprises about 70% polyethoxylated 12-hydroxystearate by weight and about 30% by weight unesterified polyethylene glycol component. Solutol HS 15 has a hydrogenation value of 90 to 110, a saponification value of 53 to 63, an acid number of maximum 1, and a maximum water content of 0.5% by weight.

### 6) Polyoxyethylene alkyl ethers

These include polyoxyethylene glycol ethers of C₁₂ to C₁₈ alcohols, e.g. Polyoxyl 2-, 10- or 20-cetyl ether or Polyoxyl 23-lauryl ether, or polyoxyl 20-oleyl ether, or Polyoxyl 2-, 10-, 20- or 100-stearyl ether, as known and commercially available e.g. under the trade mark Brij® from Uniqema. An especially preferred product of this class is e.g. Brij® 35 (Polyoxyl 23 lauryl ether) or Brij® 98 (Polyoxyl 20 oleyl ether) (Fiedler, *loc. cit.,* 1, pp. 259; Handbook of Pharmaceutical Excipients, *loc. cit.,* page 367). Similarly suitable products include polyoxyethylene-polyoxypropylene-alkyl ethers, e.g. polyoxyethylene-polyoxypropylene- ethers of C₁₂ to C₁₈ alcohols, e.g. polyoxyethylen-20-polyoxypropylene-4-cetylether which is known and commercially available under the trade mark Nikkol PBC® 34, from e.g. Nikko Chemicals Co., Ltd. (Fiedler, *loc. cit.,* vol. 2, pp. 1210). Polyoxypropylene fatty acid ethers, e.g. Acconon® E are also suitable.

### 7) Sodium alkyl sulfates and sulfonates, and sodium alkyl aryl sulfonates

These include sodium lauryl sulfate, which is also known as sodium dodecyl sulfate and commercially available, e.g. under the trade name Texapon K12® from Henkel KGaA.

### 8) Water soluble tocopheryl polyethylene glycol succinic acid esters (TPGS)

These include those with a polymerisation number ca 1000, e.g. available from Eastman Fine Chemicals Kingsport, Texas, USA.

### 9) Polyglycerol fatty acid esters

These include those with e.g. from 10 to 20, e.g. 10 glycerol units. The fatty acid constituent may include both saturated and unsaturated fatty acids having a chain length of from e.g. C₈-C₁₈. Particularly suitable is e.g. decaglycerylmonolaurat or decaglycerylmonomyristat, as known and commercially available under the trade mark Decaglyn® 1-L or Decaglyn® 1-M or Decaglyn 1-O, respectively, from e.g. Nikko Chemicals C., Ltd (Fiedler, *loc. cit.,* vol. 2, pp. 1359).

### 10) Alkylene polyol ethers or esters

These include C₃₋₅alkylene triols, in particular glycerol, ethers or esters. Suitable C₃-₅alkylene triol ethers or esters include mixed ethers or esters, i.e. components including other ether or ester ingredients, for example transesterification products of C₃₋₅alkylene triol esters with other mono-, di- or poly-ols. Particularly suitable alkylene polyol ethers or esters are mixed C₃₋₅alkylene triol/poly-(C₂₋₄alkylene) glycol fatty acid esters, especially mixed glycerol/polyethylene- or polypropylene-glycol fatty acid esters.

Especially suitable alkylene polyol ethers or esters include products obtainable by transesterification of glycerides, e.g. triglycerides, with poly-(C₂₋₄alkylene) glycols, e.g. poly-ethylene glycols and, optionally, glycerol.

Such transesterification products are generally obtained by alcoholysis of glycerides, e.g. triglycerides, in the presence of a poly-(C-₂₋₄alkylene) glycol, e.g. polyethylene glycol and, optionally, glycerol (i.e. to effect transesterification from the glyceride to the poly-alkylene glycol/glycerol component, i.e. via poly-alkylene glycolysis/gly-cerolysis). In general such reaction is effected by reacting the indicated components (glyceride, polyalkylene glycol and, optionally, glycerol) at elevated temperature under an inert atmosphere with continuous agitation.

Preferred glycerides are fatty acid triglycerides, e.g. (C₁₀₋₂₂fatty acid) triglycerides, including natural and hydrogenated oils, in particular vegetable oils. Suitable vegetable oils include, for example, olive, almond, peanut, coconut, palm, soybean and wheat germ oils and, in particular, natural or hydrogenated oils rich in (C₁₂₋₁₈fatty acid) ester residues.

Preferred polyalkylene glycol materials are polyethylene glycols, in particular polyethylene glycols having a molecular weight of from ca. 500 to ca. 4,000, e.g. from ca. 1,000 to ca. 2,000.

Suitable alkylene polyol ethers or esters include mixtures of C₃₋₅alkylene triol esters, e.g. mono-, di- and tri-esters in variable relative amount, and poly (C₂₋₄alkylene) glycol mono- and di-esters, together with minor amounts of free C₃₋₅alkylene triol and free poly-(C₂₋₅alkylene) glycol. As hereinabove set forth, the preferred alkylene triol moiety is glyceryl; preferred polyalkylene glycol moieties include polyethylene glycol, in particular having a molecular weight of from ca. 500 to ca. 4,000; and preferred fatty acid moieties will be C₁₀₋₂₂fatty acid ester residues, in particular saturated C₁₀₋₂₂fatty acid ester residues.

Particularly suitable alkylene polyol ethers or esters include transesterification products of a natural or hydrogenated vegetable oil and a polyethylene glycol and, optionally, glycerol; or compositions comprising or consisting of glyceryl mono-, di- and tri-C₁₀-₂₂fatty acid esters and polyethylene glycol mono- and di-C₁₀₋₂₂fatty esters (optionally together with, e.g. minor amounts of free glycerol and free polyethylene glycol).

Preferred vegetable oils, polyethylene glycols or polyethylene glycol moieties and fatty acid moieties in relation to the above definitions are as hereinbefore set forth.

Particularly suitable alkylene polyol ethers or esters as described above for use in the present invention include those commercially available under the trade name Gelucire® from e.g. Gattefossé, in particular the products:
a) Gelucire® 44/14, m.p. = ca. 42.5-47.5°C, saponification v. = ca. 79-93;
b) Gelucire® 50/13, m.p. = ca. 46-51 °C, saponification v. = ca. 67-81;

Products (a) to (b) above all have an acid value of maximum of 2.

Alkylene polyol ethers or esters having an iodine value of maximum 2 are generally preferred. The compositions of the invention may include mixtures of such ethers or esters.

Gelucire® products are inert semi-solid waxy materials with amphiphilic character. They are identified by their melting point and their HLB value. Most Gelucire® grades are saturated polyglycolised glycerides obtainable by polyglycolysis of natural hydrogenated vegetable oils with polyethylene glycols. They are composed of a mixture of mono-, di- and tri-glycerides and mono- and di-fatty acid esters of polyethylene glycol. Particularly suitable is Gelucire® 44/14 which has a nominal melting point of 44°C and an HLB of 14. It is obtained by reacting hydrogenated palm kernels and/or hydrogenated palm oils with polyethylene glycol 1500. It consists of approximately 20% mono-, di- and triglycerides, 72 % mono- and di- fatty acid esters of polyethylene glycol 1500 and 8% of free polyethylene glycol 1500. The fatty acid distribution for Gelucire® 44/14 is as follows: 4-10 C₈, 3-9 C₁₀, 40-50 C₁₂, 14-24 C₁₄, 4-14 C₁₆, 5-15 C₁₈. Gelucire® 44/14 exhibits the following additional characterising data: acid value of max. 2, iodine value of max. 2, saponification value of 79-93, hydroxyl value of 36-56, peroxide value of max. 6, alkaline impurities max. 80, water content max. 0.50, free glycerol content max. 3, monoglycerides content 3.0-8.0. (H. Fiedler, *loc. cit.,* vol 1, page 773; manufacturer information).

### 11) Polyethylene glycol glyceryl fatty acid esters

The fatty acid ester may include mono and/or di and/or tri fatty acid ester. The fatty acid constituent may include both saturated and unsaturated fatty acids having a chain length of from e.g. C₁₂-C₁₈. The polyethylene glycols may have e.g. from 10 to 40 [CH₂-CH₂-O] units, e.g. 15 or 30 units. Particularly suitable is polyethylene glycol (15) glyceryl monostearat which is commercially available, e.g. under the trade name TGMS®-15, e.g. from Nikko Chemicals Co., Ltd. Other suitable glyceryl fatty acid esters include polyethylene glycol (30) glyceryl monooleate which is commercially available, e.g. under the trade name Tagat® O, e.g. from Goldschmidt (H. Fiedler, *loc*. *cit.,* vol. 2, p. 1502-1503), and Tagat 02 (polytheylene glycol (20) glycerol monooleate, as well as Tagat L (polytheylene glycol (30) glycerol monolaurate) and Tagat L2 (polytheylene glycol (20) glycerol monolaurate), all e.g. from Goldschmidt (H. Fiedler, *loc. cit.,* vol. 2, p. 1650). A further suitable polyethylene glycol glyceryl fatty acid ester is Tagat TO.

### 12) Sterols and derivatives thereof

These include cholesterols and derivatives thereof, in particular phytosterols, e.g. products comprising sitosterol, campesterol or stigmasterol, and ethylene oxide adducts thereof, for example soya sterols and derivatives thereof, e.g. polyethylene glycol sterols, e.g. polyethylene glycol phytosterols or polyethylene glycol soya sterols. The polyethylene glycols may have e.g. from 10 to 40 [CH₂-CH₂-O] units, e.g. 25 or 30 units. Particularly suitable is polyethylene glycol (30) phytosterol which is commercially available, e.g. under the trade name Nikkol BPS®-30, e.g. from Nikko Chemicals Co., Ltd. Further suitable is polyethylene glycol (25) soya sterol which is commercially available, e.g. under the trade name Generol® 122 E 25, e.g. from Henkel (H. Fiedler, *loc. cit.,* vol. 1, p. 779).

### 13) Transesterified, polyoxyethylated caprylic-capric acid glycerides

These include those that are commercially available under the trade name Labrasol® from e.g. Gattefossé. Labrasol® has an acid value of max. 1, a saponification value of 90-110, and an iodine value of max. 1 (H. Fiedler, *loc. cit.,* vol 2, page 995).

### 14) Sugar fatty acid esters

These include those of C₁₂-C₁₈ fatty acids, e.g. sucrose monolaurate, e.g. Ryoto L-1695®, which is commercially available from e.g. Mitsubishi-Kasei Food Corp., Tokyo, Japan.

### 15) PEG sterol ethers

These include those having, e.g. from 5 to 35 [CH₂-CH₂-O] units, e.g. 20 to 30 units., e.g. Solulan® C24, which is commercially available from e.g. Amerchol.

### 16) Dioctylsodiumsulfosuccinate

This is commercially available under the trade mark Aerosol OT® from e.g. American Cyanamid Co. (Fiedler, *loc. cit.,* 1, p. 164), or di-[2-ethylhexyl]-succinate (Fiedler, *loc*. *cit.,* volume 1, p. 574).

### 17) Phospholipids

These include in particular lecithins (Fiedler, *loc. cit.,* volume 2, p. 910, 1030). Suitable lecithins include, in particular, soya bean lecithins.

### 18) Salts of fatty acids, fatty acid sulfates and sulfonates

These include those of e.g. C₆-C₁₈, fatty acids, -fatty acid sulfates and sulfonates, as known and commercially available from e.g. Fluka.

### 19) Salts of acylated amino acids

These include those of C₆-C₁₈, acylated amino acids, e.g. sodium lauroyl sarcosinate, which is commercially available from e.g. Fluka.

### 20) Medium or lonp-chain alkyl, e.g. C₆-C₁₈, ammonium salts

These include C₆-C₁₈ acylated amino acids e.g. cetyl trimethyl ammonium bromide, which is commercially available from e.g. E. Merck AG.

The surfactant may comprise 5 to 90 % by weight of the composition of the invention; preferably 10 to 85 % by weight, more preferably 15 to 60 % by weight.

It will be appreciated that some surfactants may also act as hydrophilic component and some hydrophilic components may also act as surfactants.

The compositions of the present invention may contain co-solvents to reduce the interfacial tension thereby providing thermodynamic stability. Suitable co-solvents include lower alkanols such as ethanol and transcutol. This is because storage characteristics are improved, in particular the risk of active agent precipitation following encapsulation procedures is reduced. Thus the shelf life stability may be extended by employing ethanol or some other such co-component as an additional ingredient of the composition. The ethanol may comprise 0 to 60 % by weight of the composition; preferably 5 to about 30% by weight and more preferably about 5 to 20 % by weight.

Certain embodiments of the compositions of the invention include additives for example antioxidants, antimicrobial agents, enzyme inhibitors, stabilizers, preservatives, flavours, sweeteners and other components such as those described in Fiedler, H. P., *loc. cit.*

These additives or ingredients may comprise about 0.05 to 5% by weight of the total weight of the composition. Antimicrobial agents, enzyme inhibitors, stabilizers or preservatives typically provide up to about 0.05 to 1 % by weight based on the total weight of the composition. Sweetening or flavouring agents typically provide up to about 2.5 or 5% by weight based on the total weight of the composition.

In another aspect, the invention provides a process for preparing a spontaneously dispersible pharmaceutical composition containing a calcilytic agent as an active agent, which process comprises bringing the active agent and a carrier medium comprising (1) a lipophilic component, (2) a surfactant, (3) a hydrophilic component, and optionally (4) a co-solvent into intimate admixture.

The carrier medium can be prepared separately before bringing the active agent into intimate admixture with the carrier medium. Alternatively the two or more of the components of the carrier medium can be mixed together with the active agent.

The spontaneously dispersible pharmaceutical composition is preferably a microemulsion preconcentrate as herein defined.

The spontaneously dispersible pharmaceutical compositions are preferably spontaneously or substantially spontaneously forms an o/w (oil-in-water) emulsion, e.g. microemulsion, when diluted with an aqueous medium such as water to a dilution of 1:1 to 1:300, e.g. 1:1 to 1:70, especially 1:10 to 1:70, more especially e.g. 1:10, or in the gastric juices of a patient after oral application.

In another aspect, the invention provides a process for preparing a microemulsion containing a calcilytic as an active agent, which process comprises:
(i) bringing the active agent and a carrier comprising (1) a lipophilic component, (2) a surfactant, (3) a hydrophilic component, and optionally (4) a co-solvent into intimate admixture to form a spontaneously dispersible pharmaceutical composition; and
(ii) diluting the spontaneously dispersible pharmaceutical composition in an aqueous medium to form the microemulsion.
   As mentioned above, the active agent may be present in an amount by weight of up to about 20% by weight of the composition of the invention, e.g. from about 0.05% by weight. The active agent is preferably present in an amount of about 0.5 to about 15 % by weight of the composition, more preferably in an amount of about 1.0 to about 5% by weight of the composition.

The lipophilic component preferably comprises about 5 to about 85 % by weight of the composition of the invention, e.g. about 10 to about 85%; preferably about 15 to about 60 % by weight.

The hydrophilic component may comprise about 5 to about 60 % by weight of the composition of the invention, e.g. about 5 to about 50%; preferably about 5 to about 40 % by weight, more preferably about 5 to about 30% by weight. It may comprise a mixture of two or more hydrophilic components.

The surfactant may comprise about 5 to about 90% by weight of the composition of the invention; preferably about 15 to about 85% by weight, more preferably about 20 to about 60 % by weight.

The co-solvent may comprise about 0 to about 90% by weight of the composition of the invention, preferably about 0 to about 30% by weight, more preferably about 0 to about 25% by weight, e.g. about 20% by weight.

The relative proportion of the active agent(s), the lipophilic component(s), the surfactant(s) the hydrophilic component(s), and the co-solvents (when present) preferably lie within the "Microemulsion" region on a standard three way plot graph. The compositions will therefore be of high stability that are capable, on addition to an aqueous medium, of providing microemulsions.

When the composition of the invention is a microemulsion preconcentrate it may be combined with water or an aqueous solvent medium to obtain an emulsion, for example a microemulsion. The emulsion or microemulsion may be administered enterally, for example orally, for example in the form of a drinkable solution.

When the composition of the invention is a microemulsion preconcentrate a unit dosage of the microemulsion preconcentrate is preferably used to fill orally administrable capsule shells. The capsule shells may be soft or hard capsule shells, for example made of gelatine. Each unit dosage will suitably contain from 0.1 to 200 mg active agent, for example 0.1 mg, 0.25mg, 0.5mg, 1 mg, 2 mg, 10 mg, 15 mg, 25 mg, 50 mg, 75 mg, 100 mg, 150 mg or 200 mg of the active agent. Such unit dosage forms are suitable for administration 1 to 5 times daily depending upon the particular purpose of therapy, the phase of therapy and the like. However, if desired, the compositions may be in drink solution form and may include water or any other aqueous system, e.g. fruit juice, milk, and the like, to provide e.g. colloidal systems, suitable for drinking, e.g. with a dilution of from about 1:10 to about 1:100.

In a further aspect of the invention the composition may be formulated as a solid dispersion.

The term solid dispersion as used inhere is understood to mean a co-precipitate or co-melt of the drug substance with the carrier medium. The term solid dispersion is used for systems in which the active ingredient is more or less evenly dispersed throughout a carrier system. The active ingredient can be present in a glassy amorphous state or in fine crystalline dispersed form. The term solid dispersion also comprises systems which contain mixtures of amorphous and crystalline drug. A solid dispersion can comprise one or more than one phase. That means the drug may exist in a pure drug phase or as solid solution in which the drug is homogenously dispersed throughout the carrier as well as in any combination of these two extremes. Eutectic mixtures of the active compound are also encompassed in this definition.

The solid dispersion may comprise an active ingredient and a carrier.

The pharmaceutical compositions formulated as a solid dispersion may be prepared by a number of methods.

In a first method, the drug substance or active ingredient is dissolved in a solvent or a solvent mixture with one or more excipients, including a carrier substance. Some or all excipients may also be present in the solvent or solvent mixture dissolved or in a suspended or swollen state. The resulting feed solution or suspension may be dried by spray drying to form a solid dispersion. The term spray drying refers to processes which involve the atomization of the feed suspension or solution into small droplets and rapidly removing solvent from the mixture in a processor-chamber where there is a strong driving force for the evaporation of the solvents (such as hot dry gas or partial vacuum or combinations thereof).

In a second method, the feed solution or suspension is being atomized and dried in the processor chamber of a spray dryer or a fluidized spray drier to form primary particles which are subsequently agglomerated in a fluid bed.

In a third method, the feed solution or suspension is dried by being atomized into a the processor chamber of a fluid bed-type processor or a pan-coater which is charged with inert filler material. During drying the filler becomes agglomerated and/or coated and/or layered by the solid dispersion.

In a fourth method the solid dispersion is prepared by melting the drug substance and/or the carrier. Atomization and re-solidification is done in a fluid bed processor.

In a fifth method, the feed solution or suspension is dried and/or dried and chopped down at elevated temperature and/or partial vacuum for example in a rotavapor-like processor or paddle dryer-type processors.

In a sixth method, solid dispersions are prepared by melting the drug substance and/or the carrier using a melt extruder.

In a seventh method solid dispersions are prepared by melting drug substance and/or the carrier in presence of a filler material wherein the particles are agglomerated and/or coated using a melt extruder.

In a eighth method solid dispersions are prepared by dissolving the drug substance using a solvent or a low melting material (for example: a polymer, plasticizer, wax, surfactant) and then mixing it into a carrier using a melt extruder.

In a ninth method solid dispersions are prepared by dissolving the drug substance using a solvent and drying it under reduced pressure, for example by using sealing elements before and after a vacuum port in the melt extruder.

In a tenth method solid dispersions are prepared by precipitation e.g. by rapid mixing of the feed solution or suspension with CO₂, or an other non-solvent.

Suitable solvents for the solvent evaporation methods are alcohols such as ethanol, methanol, n-propanol, iso-proponal and butanol. Ketones such as acetone and methyl ethylketone and various other solvents like methylene chloride. Mixtures of these solvents may also be used. The solvent or solvent mixture may also contain up to 40% water in order to fine tune the swelling grade of the certain polymeric carriers in case of feed-suspensions.

The solid dispersions may be further processed into tablet, or capsule form or may be processed into multiparticular systems e.g. minitablets or pour-into mouse granules or oral powders for constitution.

Suitable polymeric carriers include water-soluble polymers, preferably a cellulose derivative such as hydroxypropylmethylcellulose (HPMC) or hydroxypropylcellulose (HPC). Good results may be obtained using HPMC with a low apparent dynamic viscosity, e.g. from about 0.01 cps to about 100 cps as measured at 20°C for a 2% by weight aqueous solution, e.g. from about 0.01 cps to about 50 cps, preferably from about 0.01 cps to about 20 cps, for example HPMC 3 cps. HPMC is well-known and described, for example, in the Handbook of Pharmaceutical Excipients, Second Edition, Pharmaceutical Society of Great Britain and American Pharmaceutical Association, 1994, pages 229 to 232, the contents of which are incorporated herein by reference. HPMC, including HPMC 3 cps, is available commercially under the trade mark Pharmacoat® 603 from the Shinetsu company. An other suitable polymer is polyvinylpyrrolidone (PVP). PVP is available, for example, under the trade mark Povidone® (Handbook of Pharmaceutical Excipients, pages 392-399), and a PVP having an average molecular weight between about 8,000 and about 50,000 Daltons is preferred, e.g. PVP K30.

Suitable polymer carriers also include: polymers which are resistant against gastric juice and soluble in intestinal juice (used e.g for enterice polymers e.g. the cellulose derivatives hydroxyl-propyl methylcellulose acetate succinate (HPMCAS), eg Aqoat MF or HF and hydroxypropylmethyl-cellulose phthalate (e.gHPMCP-.HP50 or HPMC-HP55). Suitable polymer carriers also include a copolymer formed from monomers selected from the group consisting of methacrylic acid, methacrylic acid esters, acrylic acid and acrylic acid esters, e.g. as those known and commercially available under the trade mark Eudragit® from Röhm Pharma GmbH. An especially preferred polymer is the 1:1 or 1:2 copolymer formed from monomers selected from the group consisting of methacrylic acid and methacrylic acid lower alkyl esters, such as the 1:1 or 1:2 copolymer formed from methacrylic acid and methyl methacrylate. The 1:1 copolymers are available under the trade mark Eudragit® L, the 1:2 copolymers are available under the trade mark Eudragit® S. A particularly preferred polymer is the 1:1 copolymer of methacrylic acid and the acrylic acid ethyl ester as known and commercially available under the trade mark Eudragit®L 100-55. The enteric polymers may also be used in combinations with polymers which are non resistant against gastric juice (e.g. HPMC or PVP) in order to allow to optimize locally available drug concentrations and bioavailability.

In another embodiment the polymeric carrier comprises:
(i) hydroxypropylcellulose (HPC) or a derivative thereof. Examples of HPC derivatives include those having low dynamic viscosity in aqueous media, e.g. water, e.g. from about 0.01 cps to abut 400 cps, e.g. from about 0.01 cps to about 150 cps as measured in a 2% aqueous solution at 25°C. Preferred HPC derivatives have a low degree of substitution, and an average molecular weight e.g. between about 5000 and about 200,000 Daltons, e.g. between about 50,000 and about 150,000 Daltons. Examples of HPC available commercially include Klucel® LF, Klucel® EF and Klucel® JF from the Aqualon company; and Nisso® HPC-L available from Nippon Soda Ltd;
(ii) a cyclodextrin, for example, a beta-cyclodextrin or an alpha-cyclodextrin. Examples of suitable beta-cyclodextrins include methyl-beta-cyclodextrin; dimethyl-beta-cyclodextrin; hyrdroxypropyl-beta-cyclodextrin; glycosyl-beta-cyclodexterin; maltosyl-beta-cyclo-dextrin; sulfo-beta-cyclodextrin; sulfo-alkylethers of beta-cyclodextrin, e.g. sulfo-C[1-4]-alkyl ethers. Examples of alpha-cyclodextrins include glucosyl-alpha-cyclodextrin and maltosyl-alpha-cyclodextrin;
(iii) a polyethylene glycol (PEG). Examples include PEGs having an average molecular weight between 1000 and 9000 Daltons, e.g. between about 1800 and 7000, for example PEG 2000, PEG 4000 or PEG 6000 (Handbook of Pharmaceutical Excipients, pages 355-361);
(iv) a polymethycrylate (not just gastric-resistant)
(v) a polyvinyl alcohol polymer and co-polmyers thereof with PVP or other polymers

In another embodiment non polymeric carriers may be present comprising Low molecular weight substances which from an amorphous glass such as saccharoses e.g. Mannitol, Sorbitol or substances like Urea.

The carrier may further comprise one or more surfactants or wetting agents, for example a non-ionic, ionic, anionic or amphoteric surfactant. Examples of suitable surfactants/wetting agents include:

Polyoxyethylene-polyoxypropylene co-polymers and block co-polymers known, for example, under the trade marks Pluronic® or Poloxamer®, Polyoxyethylene-sorbitan-fatty acid esters including mono- and tri-lauryl, palmityl, stearyl and oleyl esters of the type known under the trade name Tween®, Polyoxyethylene fatty acid esters including polyoxyethylene stearic acid esters of the type known under the trade name Myrj®, Polyoxyethylene alkyl ethers known under the trade mark Brij®, Sodium alkyl sulfates and sulfonates, and sodium alkyl aryl sulfonates, water soluble tocopheryl polyethylene glycol succinic acid esters (TPGS), Polyglycerol fatty acid esters, Alkylene polyol ethers or esters, Polyethylene glycol glyceryl fatty acid esters, Sterols and derivatives thereof,_transesterified, polyoxyethylated caprylic-capric acid glycerides, sugar fatty acid esters, PEG sterol ethers, Phospholipids, Salts of fatty acids, fatty acid sulfates and sulfonates, Salts of fatty acids, fatty acid sulfates and sulfonates, Medium or long-chain alkyl, e.g. C₆-C₁₈, ammonium salts, bile acid or salt thereof; for example cholic acid, glycolic acid or a salt, e.g. sodium cholate, Polyoxyethylene mono esters of a saturated C₁₀ to C₂₂.

In a further embodiment, the present invention provides a pharmaceutical composition in the form of a solid dispersion comprising an active ingredient, a polymeric carrier and a pH modifiers such as acids, bases or buffers which may retard or enhance the dissolution rate of the dispersion. In addition conventional additives such as fillers, disintegrants, antioxidants, binders or anti-sticking agents may be part of the solid dispersion itself. When these additives are included as part of the dispersion they may be dissolved or suspended or mixed into the feed from which the solid dispersion is formed or alternatively be used as starter material (filler) in e.g. pan-coaters or fluid bed processors.

Suitable pH modifiers include but are not limited to citric acid., lactic acid succinic acids and bases like sodium acetate, calcium oxide, sodium hydroxide and buffer systems.

Suitable filler (or diluent) materials include but are not limited to, water-soluble or water-insoluble compounds such as lactose, sucrose, amylose, dextrose, mannitol and inositol, xylitol, microcrystalline cellulose, but preferably lactose, mannitol or microcrystalline cellulose. Microcrystalline cellulose is available commercially under the trade mark Avicel®, Pharmacel®, Emcocell®, Vivapur®, preferably Avicel®, available e.g. from FMC Corporate (Handbook of Pharmaceutical Excipients, pages 84-87). The fillers may also be used in form of rough more or less spheric particles like Pellets ; Cellets, , Celsphere® which are preferred starting materials for coating and layering technology. Suitable filler and anti-sticking agents also include colloidal Silicon Dioxide like Aerosil®200.or Talc.

Examples of pharmaceutically acceptable disintegrants include, but are not limited to, starches; clays; celluloses; alginates; gums; cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum.

The pharmaceutical compositions of the present invention may include additional excipients that are commonly employed in the preparation of dosage forms, such as disintegrants, lubricants, glidants, binders and fillers.

Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose, glyceryl behenate, stearic acid, hydrogenated castor oil, glyceryl monostearate, and sodium stearyl fumarate.

Examples of pharmaceutically acceptable binders include, but are not limited to, starches; celluloses and derivatives thereof, e.g., microcrystalline cellulose, e.g., AVICEL PH from FMC (Philadelphia, PA), hydroxypropyl cellulose hydroxylethyl cellulose and hydroxylpropylmethyl cellulose METHOCEL from Dow Chemical Corp. (Midland, MI); sucrose; dextrose; corn syrup; polysaccharides; and gelatin.

Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc.

The pharmaceutical compositions of the present invention may further include additives or ingredients, such as antioxidants (e.g. ascorbyl palmitate, butyl hydroxy anosole (BHA), butyl hydroxy toluene (BHT), tocopherols, propyl gallate and fumaric acid), antimicrobial agents, enzyme inhibitors, stabilizers (e.g. malonic acid), and/or preserving or flavoring agents.

The active ingredient may be present in an amount by weight of the composition of about 0.01 % to about 80%; for example, in an amount by weight of about 0.01 % to about 80%, 0.1 % to about 70%, such as 0.2% to 60%, for example 2%, 5%, 10%, 20%, 30%, 40%, 50%, or 60%.

The polymeric carrier may be present in an amount from about 0.1 % to 99.99% by weight of the composition

When a plasticizer or surfactant is present, it may generally be present in an amount of from about 0.01 % to about 30%, for example from about 1% to about 20% by weight, e.g. 1 % to 15% by weight such as 5% to 15% by weight of the composition.

When a disintegrant is present in the pharmaceutical composition, it may be generally be present in an amount from about 1% to about 30% by weight of the composition, from about 10% to about 20% by weight of the composition.

When a filler is present, it may generally be present in an amount of from about 0.01 to about 80% by weight, e.g. from about 0.5 to about 70% by weight, such as about 30%, 40% or 50% to about 60%.

When a lubricant is present , it may generally be present in amounts from about 0.1% to about 5% by weight of the composition; whereas, the glidant, e.g., may be present in an amount from about 0.1 % to about 10% by weight of the composition.

When additives, for example antioxidants, are present they may generally comprise about 0.05-5%, preferably 0.05-1 % by weight of the composition.

When pH modifying agents is present they may generally comprise about 0.05-20% by weight of the composition

When required, the solid dispersions of the invention are preferably compounded in unit dosage form, e.g. as a tablet, capsule and multi-particulate systems like granules or powder, for administration. Where the composition is in unit dosage form, each unit dosage will suitably contain from 0.1 and 150 mg active agent, for example 0.1 mg, 1 mg, 5 mg, 10 mg, 15 mg, 25 mg, 50 mg, or 100 mg, e.g. between 20 and 100 mg of the active agent. Such unit dosage forms are suitable for administration 1 to 5 times daily depending upon the particular purpose of therapy, the phase of therapy and the like.

The dosage form used, e.g. a tablet, granules or powder may be coated, for example using an enteric coating. Suitable coatings may comprise but are not limited to cellulose acetate phthalate; hydroxypropylmethylcellulose phthalate; a polymethyacrylic acid copolymer, e.g. Eudragit L or Eudragit S; or hydroxyl-propyl methylcellulose acetate succinate, eg Aqoat MF or HF.

The pharmaceutical compositions of the invention exhibit especially advantageous properties when administered orally; for example in terms of consistency and high level of bioavailability obtained in standard bioavailability trials. Such trials are performed in animals, e.g. rats or dogs or healthy volunteers using chromatographic methods, e.g. HPLC.

The compositions of the invention, e.g. those in the examples hereinafter, may show good stability characteristics as indicated by standard stability trials, for example having a shelf life stability of up to one, two or three years, and even longer. One group of compositions of the invention may be of high stability that are capable, on addition to water, of providing aqueous microemulsions having an average particle size of < 200 nm (2,000 Å), e.g. <150 nm (1,500 Å), e.g. < 100 nm (1,000 Å).

The compositions of the invention exhibit especially advantageous properties when administered orally; for example in terms of consistency and high level of bioavailability obtained in standard bioavailability trials.

Pharmacokinetic parameters, for example drug substance absorption and measured for example as blood levels, also become surprisingly more predictable and problems in administration with erratic absorption may be eliminated or reduced. Additionally the pharmaceutical compositions are effective with biosurfactants or tenside materials, for example bile salts, being present in the gastro-intestinal tract. That is, the pharmaceutical compositions of the present invention are fully dispersible in aqueous systems comprising such natural tensides and thus capable of providing emulsion or microemulsion systems and/or particulate systems in situ which are stable. The function of the pharmaceutical compositions upon oral administration remain substantially independent of and/or unimpaired by the relative presence or absence of bile salts at any particular time or for any given individual. The compositions of this invention may also reduce variability in inter- and intra-patient dose response.

The optimal dosage of active agent to be administered to a particular patient must be considered carefully. It may be advisable to monitor the blood serum levels of the active agent by radioimmunoassay, monoclonal antibody assay, or other appropriate conventional means. Dosages of a calcilytic agent will generally range from 1 to 1000 mg per day, e.g. 2.5 mg to 1000 mg per day for a 75 kilogram adult, preferably 25 mg to 500 mg, with the optimal dosage being approximately 50 to 300 mg per day.

The pharmaceutical compositions are preferably compounded in unit dosage form, for example by filling them into orally administrable capsule shells. The capsule shells may be soft or hard gelatine capsule shells. Where the pharmaceutical composition is in unit dosage form, each unit dosage will suitably contain between 10 and 400 mg of the active agent; for example 20mg, 50mg, 100mg, 200mg, 300 mg, 400mg. Such unit dosage forms are suitable for administration once or more times daily depending upon the particular purpose of therapy, the phase of therapy and the like.

Thus in another aspect, the present invention provides a method of treatment of a subject suffering from a disorder treatable with a calcilytic agent as an active agent comprising administering a therapeutically effective amount of a pharmaceutical composition of the invention to a subject in need of such treatment.

In a further aspect, the present invention provides the use of a calcilytic agent for the manufacture of a pharmaceutical composition for the treatment of a subject suffering from a disorder treatable with a calcilytic active agent, wherein the medicament, when administered, induces a rapid and short-lasting release of endogenous parathyroid hormone.

The utility of all the pharmaceutical compositions of the present invention may be observed in standard clinical tests in, for example, known indications of active agent dosages giving equivalent blood levels of active agent; for example using dosages in the range of 2.5 mg to 1000 mg of active agent per day for a 75 kilogram mammal, e.g. adult and in standard animal models. The increased bioavailability of the active agent provided by the compositions may be observed in standard animal tests and in clinical trials, e.g. as described above.

The pharmaceutical compositions of the present invention are particularly useful:
a) For treatment and prevention of skeletal disorder characterized by low bone mass and micro-architectural deterioration of bone tissue, with a consequent increase in bone fragility and susceptibility to fracture.
b) For treatment and prevention of rapid bone loss caused by estrogen deficiency, e.g. in postmenopausal women, e.g. caused by genetic factors
c) as calcium receptor antagonists which are useful in the treatment of a variety of diseases with abnormal bone or mineral homeostasis, including hypoparathyroidism, osteosarcoma, periodontal disease, fracture healing, osteoarthritis, rheumatoid arthritis, Paget's disease, humoral hypercalcemia associated with malignancy and fracture healing, and osteoporosis.

### Brief description of the drawings:

Fig 1 shows the plasma levels of compound A and the parathyroid hormone levels (hPTH expressed as hPTH equivalents) after p.o. administration of the composition illustrated in Example 1.
Fig 2 shows the plasma levels of compound B and the parathyroid hormone levels (hPTH expressed as hPTH equivalents) after p.o. administration of the composition illustrated in Example 2.
Fig 3 shows the plasma levels of compound C and the parathyroid hormone levels (hPTH expressed as hPTH equivalents) after p.o. administration of the composition illustrated in Example 3.
Fig 4 shows the plasma levels of compound D and the parathyroid hormone levels (hPTH expressed as hPTH equivalents) after p.o. administration of the composition illustrated in Example 4.
Fig 5 shows the plasma levels of compound E and the parathyroid hormone levels (hPTH expressed as hPTH equivalents) after p.o. administration of the composition illustrated in Example 5.
Fig 6 shows the plasma levels of compound F and the parathyroid hormone levels (hPTH expressed as hPTH equivalents) after p.o. administration of the composition illustrated in Example 6.
Fig 7 shows the plasma levels of compound G and the parathyroid hormone levels (hPTH expressed as hPTH equivalents) after p.o. administration of the composition illustrated in Example 7.
Fig 8 shows the plasma levels of compound H and the parathyroid hormone levels (hPTH expressed as hPTH equivalents) after p.o. administration of the composition illustrated in Example 8.
Fig 9 shows the plasma levels of compound I and the parathyroid hormone levels (hPTH expressed as hPTH equivalents) after p.o. administration of the composition illustrated in Example 9.
Fig 10 shows the plasma levels of compound J and the parathyroid hormone levels (hPTH expressed as hPTH equivalents) after p.o. administration of the composition illustrated in Example 10.
Fig 11 shows the plasma levels of compound K and the parathyroid hormone levels (hPTH expressed as hPTH equivalents) after p.o. administration of the composition illustrated in Example 11.
Fig 12 shows the plasma levels of compound L and the parathyroid hormone levels (hPTH expressed as hPTH equivalents) after p.o. administration of the composition illustrated in Example 12.
Fig 13 shows the plasma levels of compound M and the parathyroid hormone levels (hPTH expressed as hPTH equivalents) after p.o. administration of the composition illustrated in Example 13.

The following non-limiting examples illustrate further aspects of the invention.

In the examples below the following compounds are used:
- Compound A:: 1-Isopropyl-4-(4-isopropyl-phenyl)-6-propargyloxy-1H-quinazolin-2-one
- Compound B:: 1-(3-Ethoxy-4-methoxy-benzyl)-4-(4-isopropyl-phenyl)-6- propargyloxy -1H-quinazolin-2-one
- Compound C:: 4-(4-Isopropyl-phenyl)-6-propargyloxy -1-(3,4,5-trimethoxy-benzyl)-1H-quinazolin-2-one
- Compound D:: 1-[3-(2-Hydroxy-ethoxy)-benzyl]-4-(4-isopropyl-phenyl)-6- propargyloxy-1H-quinazolin-2-one
- Compound E:: 1-[3-(2-Hydroxy-ethoxy)-benzyl]-4-(4-isopropyl-phenyl)-6- propargyloxy-1H-quinazolin-2-thione
- Compound F:: [4-(4-Isopropyl-phenyl)-6-propargyloxy-quinazolin-2-yl]-(3-methoxy-phenyl)-methanone
- Compound G:: [4-(4-Isopropyl-phenyl)-6- propargyloxy -quinazolin-2-yl]-p-tolyl-methanone
- Compound H:: (4-Ethoxy-phenyl)-[4-(4-isopropyl-phenyl)-6- propargyloxy -quinazolin-2-yl]-methanone
- Compound I:: 1-[4-Bromo-2-(4-isopropyl-phenyl)-7-methoxy-1-(2-methoxy-ethyl)-1H-benzoimidazol-5-ylmethyl]-1H-imidazole-2-carboxylic acid methylamide
- Compound J:: 4-Bromo-2-(4-isopropyl-phenyl)-7-methoxy-1-(2-methoxy-ethyl)-5-(2-methylsulfanyl-pyridin-3-ylmethyl)-1H-benzoimidazole
- Compound K:: 2-(4-Isopropyl-phenyl)-7-methoxy-1-(2-methoxy-ethyl)-5-(2-methylsulfanyl-pyridin-3-ylmethyl)-4-trifluoromethyl-1H-benzoimidazole
- Compound L:: 2-(4-Isopropyl-phenyl)-5-(2-methanesulfonyl-pyridin-3-ylmethyl)-7-methoxy-1-(2-methoxy-ethyl)-4-trifluoromethyl-1H-benzoimidazole
- Compound M:: 1-[6-(2-Hydroxy-ethoxy)-pyridin-2-ylmethyl]-4-(4-isopropyl-phenyl)-6-propargyloxy -1H-quinazolin-2-one

### EXAMPLE 1

This Example (and Examples 2 through 12) illustrates making a microemulsion preconcentrate according to the invention.

Compound A is formulated with the composition indicated in Table 1. The microemulsion preconcentrate is prepared as follows. Cremophor® RH40 is heated to 65°C with stirring. Surfactant, co-solvent, anti-oxidant, lipophilic and hydrophilic components are then combined at the weight ratios indicated in Table 1 and stirred for one hour. The clear solution thereby obtained is mixed with compound A and the resulting mixture is stirred at ambient temperature for 8 to 12 hours. Complete dissolution of compound A in the microemulsion preconcentrate is assessed by crossed polarized light microscopy.

**Table 1 - Composition of microemulsion preconcentrate with 1 % of compound A**

| Component | Function | wt% |
|---|---|---|
| Compound A | API | 1.0 |
| Cremophor® RH40 | Surfactant | 42.5 |
| Tocopherol, DL-alpha | Anti-oxidant | 0.1 |
| Corn oil mono-, di-, triglycerides | Lipophilic component | 35.3 |
| Propylene glycol (1,2-propanediol) | Hydrophilic component | 10.5 |
| Ethanol absolute | Co-solvent | 10.5 |

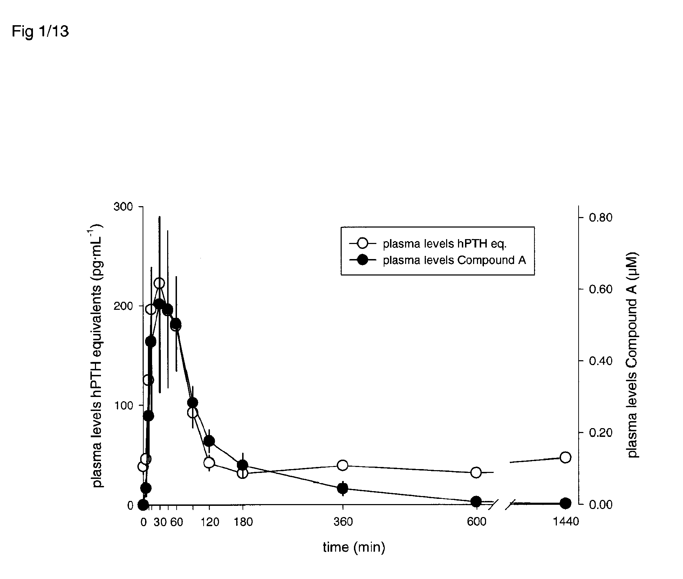

Fasted male beagle dogs (n=3 per group) between the ages of 2-9 years and weighing about 10 kg are dosed with composition A as follows. The microemulsion preconcentrate is diluted ten-fold with deionized water, mixed well, and administered by gavage with a subsequent rinse with 15 mL of tap water. The dogs are dosed with 30 mg/dog. Blood samples from conscious dogs are taken into EDTA-coated tubes at 0, 5, 10, 15, 30, 45, 60, 90, 120, 180, 360, 600, and 1440 minutes post administration. Plasma concentrations of parent compound are determined using a specific HPLC/MS-MS method, and levels of bioactive parathyroid hormone (hPTH) are determined with an intact PTH radioimmunoassay. The results are shown in Fig. 1.

### EXAMPLE 2

Compound B is formulated with the composition indicated in Table 2. The microemulsion preconcentrate is prepared as described in Example 1.

**Table 2 - Composition of microemulsion preconcentrate with 2% of compound B**

| Component | Function | wt% |
|---|---|---|
| Compound B | API | 2.0 |
| Cremophor® RH40 | Surfactant | 44.1 |
| Corn oil mono-, di-, triglycerides | Lipophilic component | 35.3 |
| Propylene glycol (1,2-propanediol) | Hydrophilic component | 8.8 |
| Ethanol absolute | Co-solvent | 9.8 |

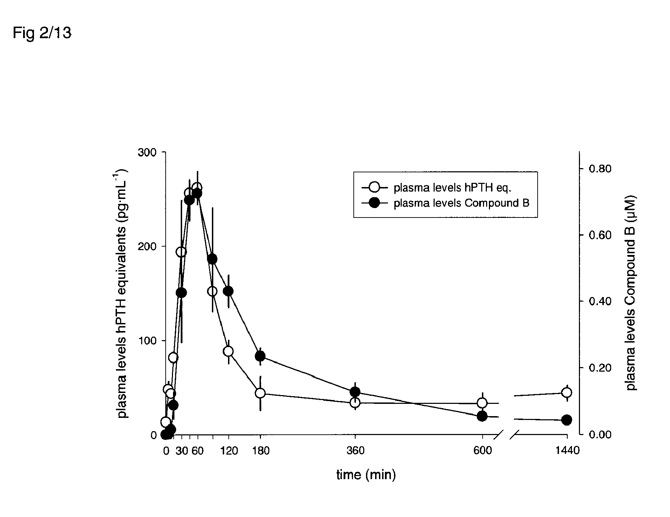

The composition is administered to dogs as described in Example 1 with a dose of 10 mg/dog. The results are shown in Fig. 2.

### EXAMPLE 3

Compound C is formulated with the composition indicated in Table 3. The microemulsion preconcentrate is prepared following the instructions described for Example 1.

**Table 3 - Composition of microemulsion preconcentrate with 1.7% of compound C**

| Component | Function | wt% |
|---|---|---|
| Compound C | API | 1.7 |
| Cremophor® RH40 | Surfactant | 44.2 |
| Corn oil mono-, di-, triglycerides | Lipophilic component | 17.7 |
| PEG400 | Hydrophilic component | 26.5 |
| Ethanol absolute | Co-solvent | 9.8 |

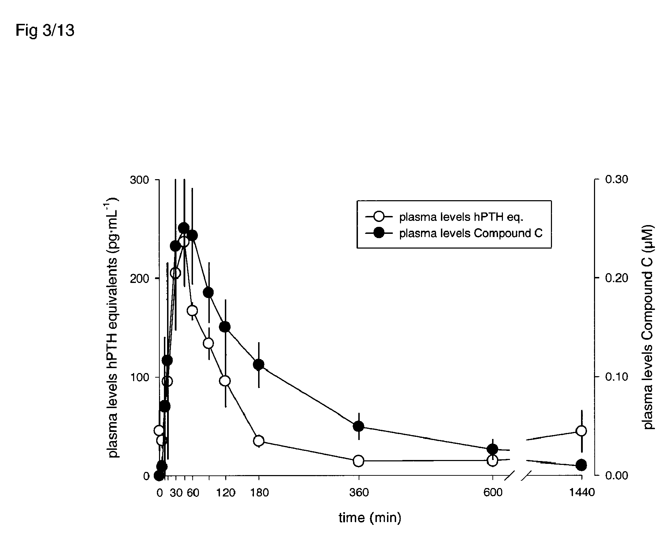

The composition is administered to dogs as described in Example 1 with a dose of 10 mg/dog. The results are shown in Fig. 3.

### EXAMPLE 4

Compound D is formulated with the composition indicated in Table 4. The microemulsion preconcentrate is prepared following the instructions described for Example 1.

**Table 4 - Composition of microemulsion preconcentrate with 2% of compound D**

| Component | Function | Wt% |
|---|---|---|
| Compound D | API | 2.0 |
| Cremophor® RH40 | Surfactant | 42.1 |
| Tocopherol, DL-alpha | Anti-oxidant | 0.1 |
| Corn oil mono-, di-, triglycerides | Lipophilic component | 35.0 |
| Propylene glycol (1,2-propanediol) | Hydrophilic component | 10.4 |
| Ethanol absolute | Co-solvent | 10.4 |

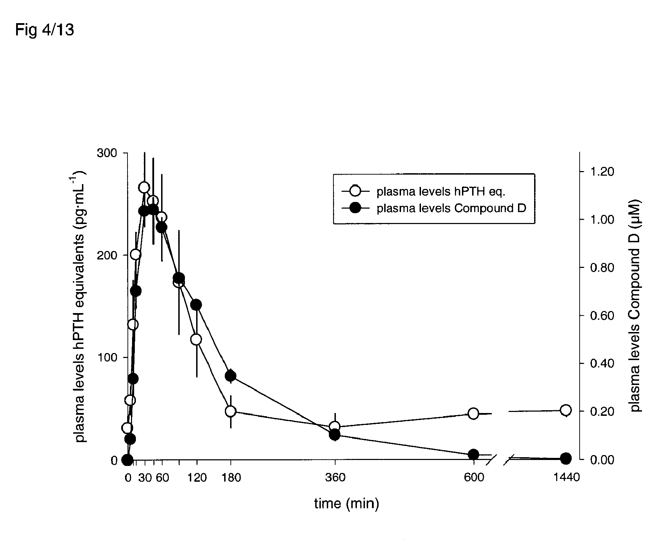

The composition is administered to dogs as described in Example 1 with a dose of 28 mg/dog. The results are shown in Fig. 4.

### EXAMPLE5

Compound E is formulated with the composition indicated in Table 5. The microemulsion preconcentrate is prepared following the instructions described for Example 1.

**Table 5 - Composition of microemulsion preconcentrate with 0.6% of compound E**

| Component | Function | wt% |
|---|---|---|
| Compound E | API | 0.6 |
| Cremophor® RH40 | Surfactant | 57.8 |
| Labrafil® M2125 CS | Lipophilic component | 16.8 |
| Propylene glycol (1,2-propanediol) | Hydrophilic component | 8.3 |
| Ethanol absolute | Co-solvent | 16.6 |

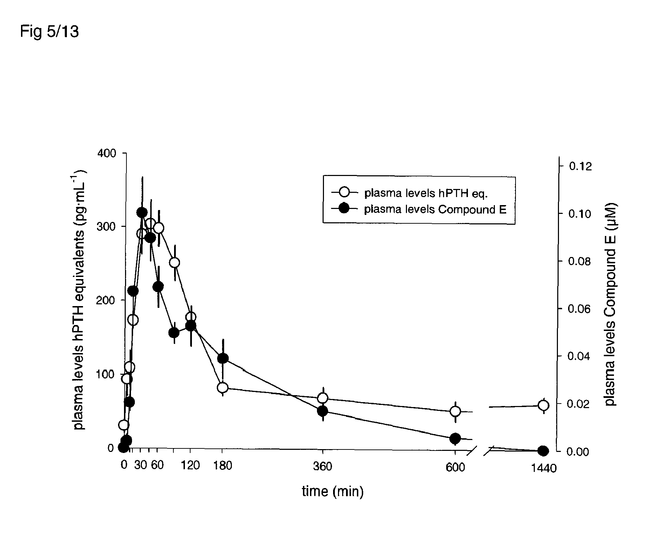

The composition is administered to dogs as described in Example 1 with a dose of 28 mg/dog. The results are shown in Fig. 5.

### EXAMPLE 6

Compound F is formulated with the same composition as compound B (Table 2). The microemulsion preconcentrate is prepared following the instructions described for Example 1.

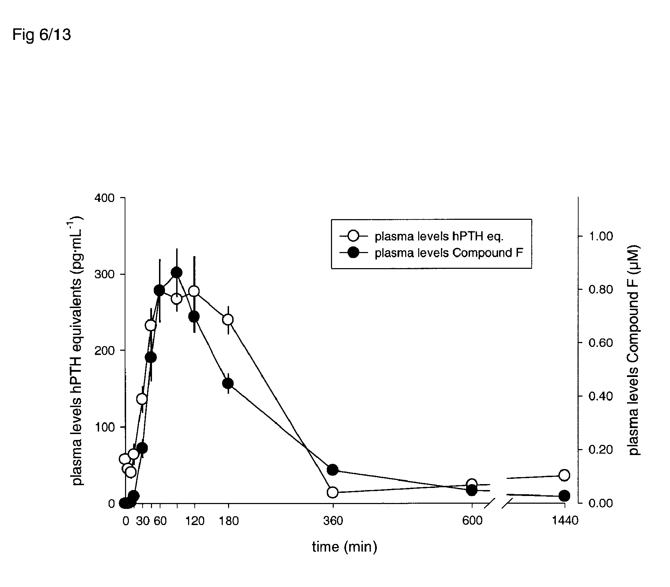

The composition is administered to dogs as described in Example 1 with a dose of 30 mg/dog. The results are shown in Fig. 6.

### EXAMPLE 7

Compound G is formulated with the same composition as compound B (Table 2). The microemulsion preconcentrate is prepared following the instructions described for Example 1.

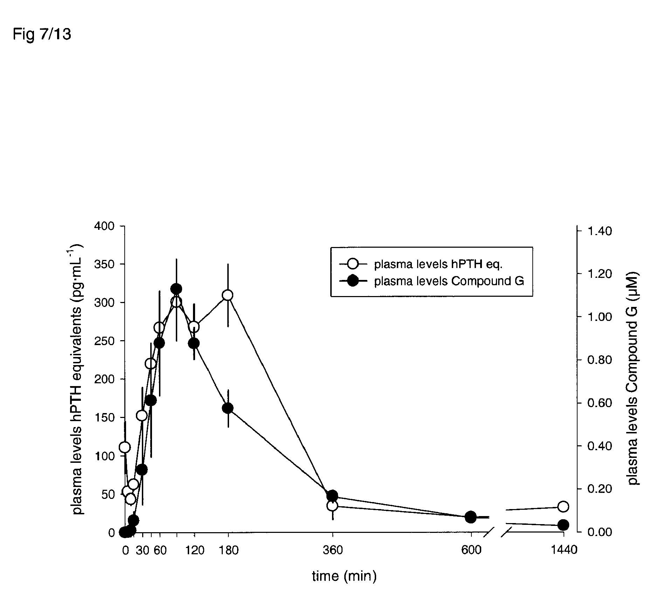

The composition is administered to dogs as described in Example 1 with a dose of 30 mg/dog. The results are shown in Fig. 7.

### EXAMPLE 8

Compound H is formulated with the same composition as compound B (Table 2). The microemulsion preconcentrate is prepared following the instructions described for Example 1.

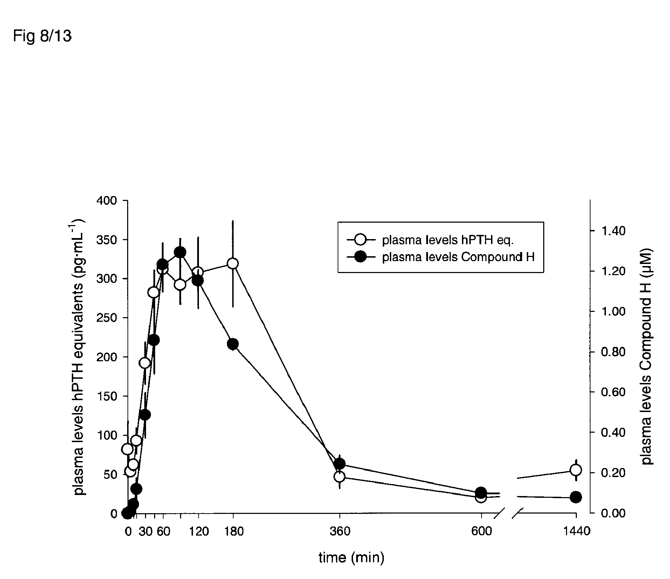

The composition is administered to dogs as described in Example 1 with a dose of 30 mg/dog. The results are shown in Fig. 8.

### EXAMPLE 9

Compound I is formulated with the same composition as compound B (Table 2). The microemulsion preconcentrate is prepared following the instructions described for Example 1.

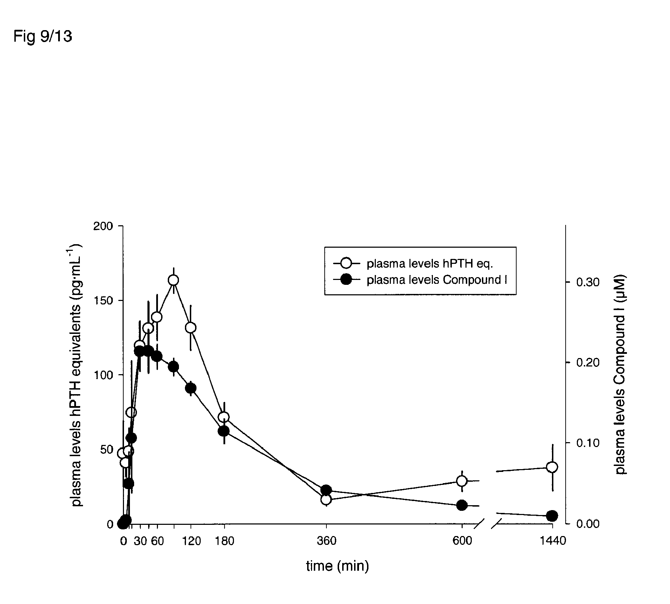

The composition is administered to dogs as described in Example 1 with a dose of 30 mg/dog. The results are shown in Fig. 9.

### EXAMPLE 10

Compound J is formulated with the same composition as compound B (Table 2). The microemulsion preconcentrate is prepared following the instructions described for Example 1.

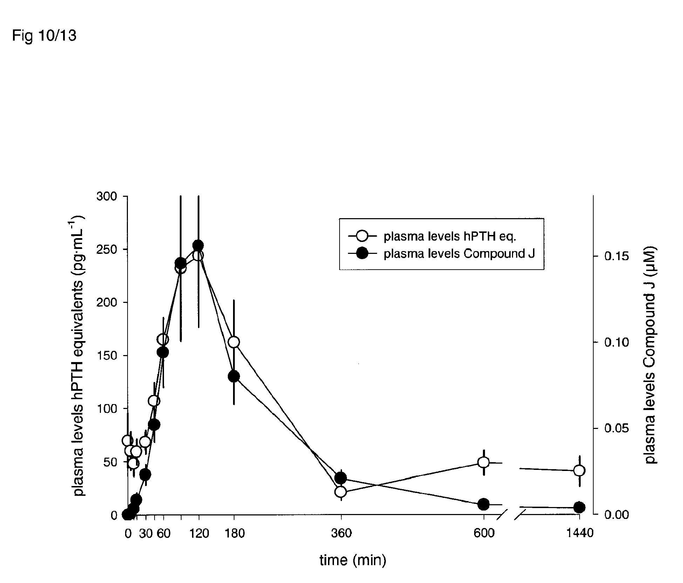

The composition is administered to dogs as described in Example 1 with a dose of 30 mg/dog. The results are shown in Fig. 10.

### EXAMPLE 11

Compound K is formulated with the composition indicated in Table 6. The microemulsion preconcentrate is prepared following the instructions described for Example 1.

**Table 6 - Composition of microemulsion preconcentrate with 0.7% of compound K**

| Component | Function | Wt% |
|---|---|---|
| Compound K | API | 0.7 |
| Cremophor® RH40 | Surfactant | 44.7 |
| Corn oil mono-, di-, triglycerides | Lipophilic component | 35.8 |
| Propylene glycol (1,2-propanediol) | Hydrophilic component | 8.9 |
| Ethanol absolute | Co-solvent | 9.9 |

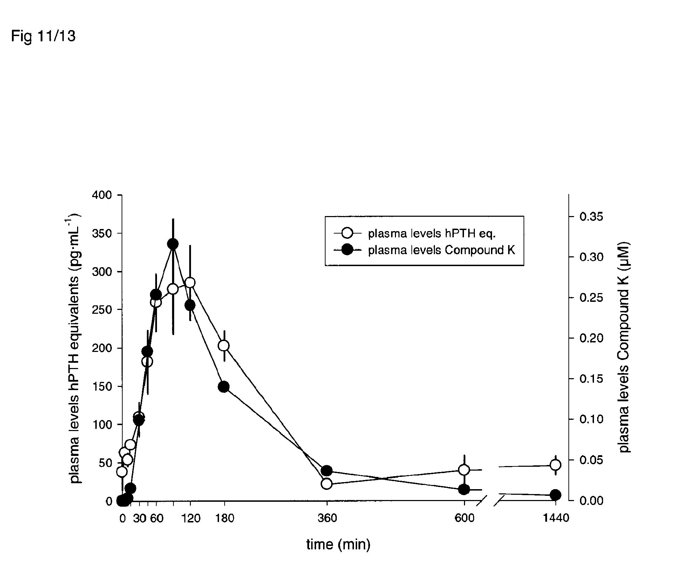

The composition is administered to dogs as described in Example 1 with a dose of 10 mg/dog. The results are shown in Fig. 11.

### EXAMPLE 12

Compound L is formulated with the composition indicated in Table 7. The microemulsion preconcentrate is prepared following the instructions described for Example 1.

**Table 7 - Composition of microemulsion preconcentrate with 0.7% of compound L**

| Component | Function | wt% |
|---|---|---|
| Compound L | API | 0.7 |
| Cremophor® RH40 | Surfactant | 57.7 |
| Labrafil® M2125 CS | Lipophilic component | 16.8 |
| Propylene glycol (1,2-propanediol) | Hydrophilic component | 8.2 |
| Ethanol absolute | Co-solvent | 16.6 |

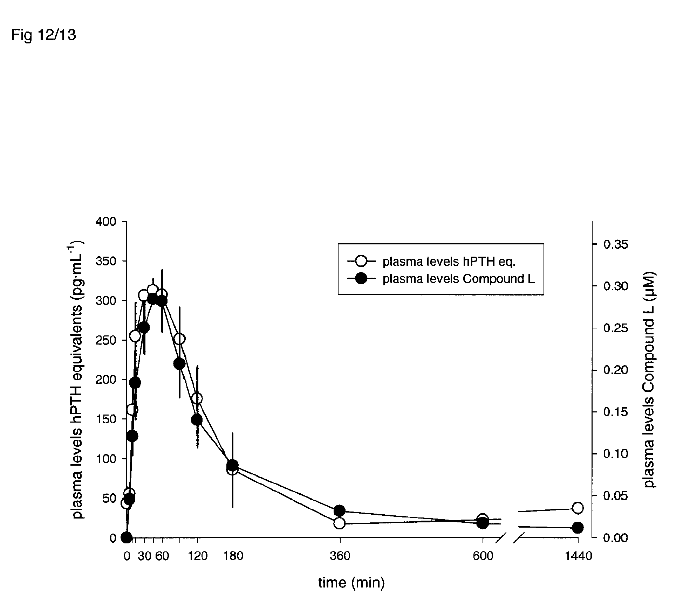

The composition is administered to dogs as described in Example 1 with a dose of 10 mg/dog. The results are shown in Fig. 12.

### EXAMPLE 13

This Example illustrates making a solid dispersion according to the invention.

Compound M is formulated as a solid dispersion containing 10% (wt%) compound M and 90% (wt%) hydroxypropyl methylcellulose (3 cps viscosity grade). The solid dispersion is prepared as follows. To a mixture of 10 mL of ethanol absolute and 10 mL of acetone 0.4g of compound M are added. The resulting mixture is stirred at ambient temperature until complete dissolution of compound M is achieved. To the mixture kept under constant stirring 3.6 g of hydroxypropyl methylcellulose are added. The final mixture is stirred for one hour at ambient temperature until full dispersion of the polymer is achieved. Then, the solvent is evaporated under vacuum at 28 mbar, using a rotary evaporator equipped with a water bath set to 40°C. The solid dispersion is further dried in a vacuum oven overnight. It is then removed from the glass wall using a spatula and transferred to a mortar where it is milled using a pestle. Finally, it is sieved through a 0.8 mm sieve.

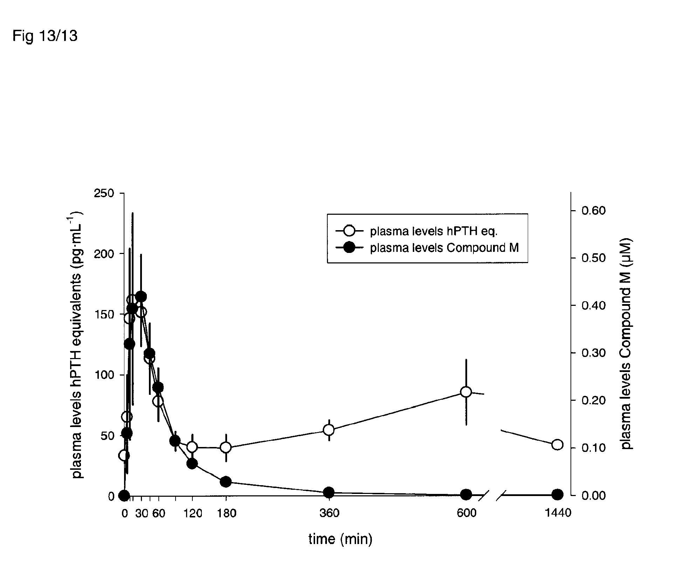

Fasted male beagle dogs (n=3 per group) between the ages of 2-9 years and weighing about 10 kg are dosed with composition M. 0.6 g of solid dispersion are weighted into a glass recipient followed by addition of 59.41 g of deionized water. After 5 minutes stirring, 10 mL of the resultant mixture are applied by gavage under constant stirring to dogs, followed by rinse with 30 mL of tap water. Blood samples from conscious dogs are taken into EDTA-coated tubes at 0, 5, 10, 15, 30, 45, 60, 90, 120, 180, 360, 600, and 1440 minutes post administration. Plasma concentrations of parent compound are determined using a specific HPLC/MS-MS method, and levels of bioactive parathyroid hormone (hPTH) are determined with an intact PTH radioimmunoassay. The results are shown in Fig. 13.

## Claims

1. A pharmaceutical composition comprising a calcilytic agent which, when administered orally to a subject induces a rapid and short-lasting release of parathyroid hormone into the plasma.

2. A pharmaceutical composition comprising a calcilytic agent which, when administered orally to a subject results in a rapid release and gastro-intestinal absorption of the calcilytic agent with a subsequent, rapid and short-lasting increase of its plasma levels.

3. A pharmaceutical composition comprising a calcilytic agent which, when administered orally to a subject induces a rapid and short-lasting release and gastro-intestinal absorption of the calcilytic agent and a rapid and short-lasting release of the parathyroid hormone.

4. A pharmaceutical composition according to claim 3 wherein the parathyroid hormone and the calcilytic agent show super-imposable plasma concentration profiles.

5. A pharmaceutical composition according to any one of the precding claims wherein the calcilytic agent and the parathyroid hormone show a release profile having a Tmax not later than 90 minutes after Tstart and a Cmax which is a five to seven-fold increase compared to baseline levels.

6. A pharmaceutical composition according to any one of the preceding claims wherein the pharmaceutical composition is in the form of a spontaneously dispersible composition.

7. A pharmaceutical composition according to any one of the preceding claims further comprising a carrier medium comprising a lipophilic component, a surfactant, and a hydrophilic component.

8. A composition according to claim 7 wherein the carrier medium further comprises a co-solvent.

9. A composition according to claim 7 or 8 wherein the lipophilic component comprises C₈-C₁₀ fatty acid monoglycerides, diglycerides or propylene glycol mono fatty esters.

10. The composition according to any one of claims 7-9 wherein the hydrophilic component comprises propylene glycol, PEG 400. demethylisosorbide or transcutol.

11. The composition according to any one of claims 7-10 wherein the surfactant comprises polyethylenegylcol-hydrogenated castor oil.

12. The composition according to any one of claims 8-11 wherein the co-solvent comprises ethanol.

13. A composition according to any one of the preceding claims which is a gel capsule.

14. A composition according to any one of the preceding claims wherein the composition is in the form of a solid dispersion.

15. A composition according to claim 14 comprising a calcylitic agent and a carrier.

16. A composition according to claim 15 wherein the carrier is hydroxypropyl methylcellulose.

17. A method of treatment of a subject suffering from a disorder treatable with a calcilytic agent comprising administering a therapeutically effective amount of a pharmaceutical composition as claimed in any preceding claim to a subject in need of such treatment.

18. Use of a calcilytic agent for the manufacture of a pharmaceutical composition as claimed in any preceding claim for the treatment of a subject suffering from a disorder treatable with a calcilytic active agent.

19. A process for preparing a spontaneously dispersible pharmaceutical composition according to claim 6, which process comprises bringing the calcilytic agent and a carrier medium comprising a lipophilic component, a surfactant, and a hydrophilic component into intimate admixture.

20. A process according to claim 19 wherein the carrier medium further comprises a co-solvent.
